# EUROPÄISCHE PATENTANMELDUNG

(11) **EP 1 710 315 A1**
(43) Veröffentlichungstag der Anmeldung: **11.10.2006**
(21) Anmeldenummer: 05090095.0
(22) Anmeldetag: 08.04.2005
(51) Int. Cl.: C12N 15/82, C08B 30/04

(54) **Hoch Phosphat Stärke**

(71) Anmelder: Bayer CropScience GmbH, 65929 Frankfurt/Main (DE)
(72) Erfinder: Soyka, Stephan, 9051 Sint-Denijs-Western (BE); Frohberg, Claus, 14532 Kleinmachnow (DE)
(74) Vertreter: Kossmann, Jochen

(57) **Zusammenfassung**

Die Erfindung betrifft modifizierte Stärken mit einem erhöhten Gehalt an Phosphat und einem erhöhten Gehalt an Amylose.

SEQUENCE LISTING

<110>  Bayer CropScience GmbH

<120>  Hoch Phosphat Stärke

<130>  BCS 05-5002-EP

<150>  EP 02028530.0
<151>  2002-12-19

<150>  EP 03090275.3
<151>  2003-08-29

<160>  14

<170>  PatentIn version 3.1

<210>  1
<211>  4167
<212>  DNA
<213>  Solanum tuberosum

<220>
<221>  CDS
<222>  (207)..(3899)
<223>

<300>
<301>  Abel,G.J., Springer,F., Willmitzer,L. and Kossmann,J.
<302>  Cloning and functional analysis of a cDNA encoding a novel 139 kDa
<303>  Plant J.
<304>  10
<305>  6

<306>  981-991

<307>  1996

<308>  X94400

<309>  1995-12-22

<313>  (1)..(4167)


<300>

<308>  EMBL / X94400

<309>  1997-04-16

<313>  (1)..(4167)


<400>  1
   ttttttaata gatttttaaa accccattaa agcaaatacg tatataattg cagcacagat      60
   acagagaggg agagagaaag atagtgtgtt gatgaaggag aagagagata tttcacatgg     120
   gatgttctat ttgattctgt ggtgaacaag agttttacaa agaacattcc tttttctttt     180
   tttcttggtt cttgtgtggg tcagcc atg gat gtt cca ttt cca ctg cat aga      233
                                Met Asp Val Pro Phe Pro Leu His Arg
                                1               5
   cca ttg agt tgc aca agt gtc tcc aat gca ata acc cac ctc aag atc      281
   Pro Leu Ser Cys Thr Ser Val Ser Asn Ala Ile Thr His Leu Lys Ile
   10                  15                  20                  25
   aaa cct ttt ctt ggg ttt gtc tct cat gga acc aca agt cta tca gta      329
   Lys Pro Phe Leu Gly Phe Val Ser His Gly Thr Thr Ser Leu Ser Val
                   30                  35                  40
   caa tct tct tca tgg agg aag gat gga atg gtt act ggg gtt tca ttt      377
   Gln Ser Ser Ser Trp Arg Lys Asp Gly Met Val Thr Gly Val Ser Phe
               45                  50                  55
   cca ttt tgt gca aat ctc tcg gga aga aga cgg aga aaa gtt tca act      425
   Pro Phe Cys Ala Asn Leu Ser Gly Arg Arg Arg Arg Lys Val Ser Thr
           60                  65                  70
   act agg agt caa gga tct tca cct aag ggg ttt gtg cca agg aag ccc      473
   Thr Arg Ser Gln Gly Ser Ser Pro Lys Gly Phe Val Pro Arg Lys Pro
       75                  80                  85
   tca ggg atg agc acg caa aga aag gtt cag aag agc aat ggt gat aaa      521
   Ser Gly Met Ser Thr Gln Arg Lys Val Gln Lys Ser Asn Gly Asp Lys
   90                  95                  100                 105
   gaa agt caa agt act tca aca tct aaa gaa tct gaa att tcc aac cag      569
   Glu Ser Gln Ser Thr Ser Thr Ser Lys Glu Ser Glu Ile Ser Asn Gln
                   110                 115                 120
   aag acg gtt gaa gca aga gtt gaa act agt gac gat gac act aaa gta      617
   Lys Thr Val Glu Ala Arg Val Glu Thr Ser Asp Asp Asp Thr Lys Val
               125                 130                 135
   gtg gtg agg gac cac aag ttt ctg gag gat gag gat gaa atc aat ggt      665
   Val Val Arg Asp His Lys Phe Leu Glu Asp Glu Asp Glu Ile Asn Gly
           140                 145                 150
   tct act aaa tca ata agt atg tca cct gtt cgt gta tca tct caa ttt 713
   Ser Thr Lys Ser Ile Ser Met Ser Pro Val Arg Val Ser Ser Gln Phe
   155 160 165
   gtt gaa agt gaa gaa act ggt ggt gat gac aag gat gct gta aag tta 761
   Val Glu Ser Glu Glu Thr Gly Gly Asp Asp Lys Asp Ala Val Lys Leu
   170 175 180 185
   aac aaa tca aag aga tcg gaa gag agt gat ttt cta att gat tct gta 809
   Asn Lys Ser Lys Arg Ser Glu Glu Ser Asp Phe Leu Ile Asp Ser Val
   190 195 200
   ata aga gaa caa agt gga tct cag ggg gaa act aat gcc agt agc aag 857
   Ile Arg Glu Gln Ser Gly Ser Gln Gly Glu Thr Asn Ala Ser Ser Lys
   205 210 215
   gga agc cat gct gtg ggt aca aaa ctt tat gag ata ttg cag gtg gat 905
   Gly Ser His Ala Val Gly Thr Lys Leu Tyr Glu Ile Leu Gln Val Asp
   220 225 230
   gtt gag cca caa caa ttg aaa gaa aat aat gct ggg aat gtt gaa tac 953
   Val Glu Pro Gln Gln Leu Lys Glu Asn Asn Ala Gly Asn Val Glu Tyr
   235 240 245
   aaa gga cct gta gca agt aag cta ttg gaa att act aag gct agt gat 1001
   Lys Gly Pro Val Ala Ser Lys Leu Leu Glu Ile Thr Lys Ala Ser Asp
   250 255 260 265
   gtg gaa cac act gaa agc aat gag att gat gac tta gac act aat agt 1049
   Val Glu His Thr Glu Ser Asn Glu Ile Asp Asp Leu Asp Thr Asn Ser
   270 275 280
   ttc ttt aaa tca gat tta att gaa gag gat gag cca tta gct gca gga 1097
   Phe Phe Lys Ser Asp Leu Ile Glu Glu Asp Glu Pro Leu Ala Ala Gly
   285 290 295
   aca gtg gag act gga gat tct tct cta aac tta aga ttg gag atg gaa 1145
   Thr Val Glu Thr Gly Asp Ser Ser Leu Asn Leu Arg Leu Glu Met Glu
   300 305 310
   gca aat cta cgt agg cag gct ata gaa agg ctt gcc gag gaa aat tta 1193
   Ala Asn Leu Arg Arg Gln Ala Ile Glu Arg Leu Ala Glu Glu Asn Leu
   315 320 325
   ttg caa ggg atc aga tta ttt tgt ttt cca gag gtt gta aaa cct gat 1241
   Leu Gln Gly Ile Arg Leu Phe Cys Phe Pro Glu Val Val Lys Pro Asp
   330 335 340 345
   gaa gat gtc gag ata ttt ctt aac aga ggt ctt tcc act ttg aag aat 1289
   Glu Asp Val Glu Ile Phe Leu Asn Arg Gly Leu Ser Thr Leu Lys Asn
   350 355 360
   gag tct gat gtc ttg att atg gga gct ttt aat gag tgg cgc tat agg 1337
   Glu Ser Asp Val Leu Ile Met Gly Ala Phe Asn Glu Trp Arg Tyr Arg
   365 370 375
   tct ttt act aca agg cta act gag act cat ctc aat gga gat tgg tgg 1385
   Ser Phe Thr Thr Arg Leu Thr Glu Thr His Leu Asn Gly Asp Trp Trp
   380 385 390
   tct tgc aag atc cat gtt ccc aag gaa gca tac agg gct gat ttt gtg 1433
   Ser Cys Lys Ile His Val Pro Lys Glu Ala Tyr Arg Ala Asp Phe Val
   395 400 405
   ttt ttt aat gga caa gat gtc tat gac aac aat gat gga aat gac ttc 1481
   Phe Phe Asn Gly Gln Asp Val Tyr Asp Asn Asn Asp Gly Asn Asp Phe
   410 415 420 425
   agt ata act gtg aaa ggt ggt atg caa atc att gac ttt gaa aat ttc 1529
   Ser Ile Thr Val Lys Gly Gly Met Gln Ile Ile Asp Phe Glu Asn Phe
   430 435 440
   ttg ctt gag gag aaa tgg aga gaa cag gag aaa ctt gct aaa gaa caa 1577
   Leu Leu Glu Glu Lys Trp Arg Glu Gln Glu Lys Leu Ala Lys Glu Gln
   445 450 455
   gct gaa aga gaa aga cta gcg gaa gaa caa aga cga ata gaa gca gag 1625
   Ala Glu Arg Glu Arg Leu Ala Glu Glu Gln Arg Arg Ile Glu Ala Glu
   460 465 470
   aaa gct gaa att gaa gct gac aga gca caa gca aag gaa gag gct gca 1673
   Lys Ala Glu Ile Glu Ala Asp Arg Ala Gln Ala Lys Glu Glu Ala Ala
   475 480 485
   aag aaa aag aaa gta ttg cga gaa ttg atg gta aaa gcc acg aag act 1721
   Lys Lys Lys Lys Val Leu Arg Glu Leu Met Val Lys Ala Thr Lys Thr
   490 495 500 505
   cgt gat atc acg tgg tac ata gag cca agt gaa ttt aaa tgc gag gac 1769
   Arg Asp Ile Thr Trp Tyr Ile Glu Pro Ser Glu Phe Lys Cys Glu Asp
   510 515 520
   aag gtc agg tta tac tat aac aaa agt tca ggt cct ctc tcc cat gct 1817
   Lys Val Arg Leu Tyr Tyr Asn Lys Ser Ser Gly Pro Leu Ser His Ala
   525 530 535
   aag gac ttg tgg atc cac gga gga tat aat aat tgg aag gat ggt ttg 1865
   Lys Asp Leu Trp Ile His Gly Gly Tyr Asn Asn Trp Lys Asp Gly Leu
   540 545 550
   tct att gtc aaa aag ctt gtt aaa tct gag aga ata gat ggt gat tgg 1913
   Ser Ile Val Lys Lys Leu Val Lys Ser Glu Arg Ile Asp Gly Asp Trp
   555 560 565
   tgg tat aca gag gtt gtt att cct gat cag gca ctt ttc ttg gat tgg 1961
   Trp Tyr Thr Glu Val Val Ile Pro Asp Gln Ala Leu Phe Leu Asp Trp
   570 575 580 585
   gtt ttt gct gat ggt cca ccc aag cat gcc att gct tat gat aac aat 2009
   Val Phe Ala Asp Gly Pro Pro Lys His Ala Ile Ala Tyr Asp Asn Asn
   590 595 600
   cac cgc caa gac ttc cat gcc att gtc ccc aac cac att ccg gag gaa 2057
   His Arg Gln Asp Phe His Ala Ile Val Pro Asn His Ile Pro Glu Glu
   605 610 615
   tta tat tgg gtt gag gaa gaa cat cag atc ttt aag aca ctt cag gag 2105
   Leu Tyr Trp Val Glu Glu Glu His Gln Ile Phe Lys Thr Leu Gln Glu
   620 625 630
   gag aga agg ctt aga gaa gcg gct atg cgt gct aag gtt gaa aaa aca 2153
   Glu Arg Arg Leu Arg Glu Ala Ala Met Arg Ala Lys Val Glu Lys Thr
   635 640 645
   gca ctt ctg aaa act gaa aca aag gaa aga act atg aaa tca ttt tta 2201
   Ala Leu Leu Lys Thr Glu Thr Lys Glu Arg Thr Met Lys Ser Phe Leu
   650 655 660 665
   ctg tct cag aag cat gta gta tat act gag cct ctt gat atc caa gct 2249
   Leu Ser Gln Lys His Val Val Tyr Thr Glu Pro Leu Asp Ile Gln Ala
   670 675 680
   gga agc agc gtc aca gtt tac tat aat ccc gcc aat aca gta ctt aat 2297
   Gly Ser Ser Val Thr Val Tyr Tyr Asn Pro Ala Asn Thr Val Leu Asn
   685 690 695
   ggt aaa cct gaa att tgg ttc aga tgt tca ttt aat cgc tgg act cac 2345
   Gly Lys Pro Glu Ile Trp Phe Arg Cys Ser Phe Asn Arg Trp Thr His
   700 705 710
   cgc ctg ggt cca ttg cca cct cag aaa atg tcg cct gct gaa aat ggc 2393
   Arg Leu Gly Pro Leu Pro Pro Gln Lys Met Ser Pro Ala Glu Asn Gly
   715 720 725
   acc cat gtc aga gca act gtg aag gtt cca ttg gat gca tat atg atg 2441
   Thr His Val Arg Ala Thr Val Lys Val Pro Leu Asp Ala Tyr Met Met
   730 735 740 745
   gat ttt gta ttt tcc gag aga gaa gat ggt ggg att ttt gac aat aag 2489
   Asp Phe Val Phe Ser Glu Arg Glu Asp Gly Gly Ile Phe Asp Asn Lys
   750 755 760
   agc gga atg gac tat cac ata cct gtg ttt gga gga gtc gct aaa gaa 2537
   Ser Gly Met Asp Tyr His Ile Pro Val Phe Gly Gly Val Ala Lys Glu
   765 770 775
   cct cca atg cat att gtc cat att gct gtc gaa atg gca cca att gca 2585
   Pro Pro Met His Ile Val His Ile Ala Val Glu Met Ala Pro Ile Ala
   780 785 790
   aag gtg gga ggc ctt ggt gat gtt gtt act agt ctt tcc cgt gct gtt 2633
   Lys Val Gly Gly Leu Gly Asp Val Val Thr Ser Leu Ser Arg Ala Val
   795 800 805
   caa gat tta aac cat aat gtg gat att atc tta cct aag tat gac tgt 2681
   Gln Asp Leu Asn His Asn Val Asp Ile Ile Leu Pro Lys Tyr Asp Cys
   810 815 820 825
   ttg aag atg aat aat gtg aag gac ttt cgg ttt cac aaa aac tac ttt 2729
   Leu Lys Met Asn Asn Val Lys Asp Phe Arg Phe His Lys Asn Tyr Phe
   830 835 840
   tgg ggt ggg act gaa ata aaa gta tgg ttt gga aag gtg gaa ggt ctc 2777
   Trp Gly Gly Thr Glu Ile Lys Val Trp Phe Gly Lys Val Glu Gly Leu
   845 850 855
   tcg gtc tat ttt ttg gag cct caa aac ggg tta ttt tcg aaa ggg tgc 2825
   Ser Val Tyr Phe Leu Glu Pro Gln Asn Gly Leu Phe Ser Lys Gly Cys
   860 865 870
   gtc tat ggt tgt agc aat gat ggt gaa cga ttt ggt ttc ttc tgt cac 2873
   Val Tyr Gly Cys Ser Asn Asp Gly Glu Arg Phe Gly Phe Phe Cys His
   875 880 885
   gcg gct ttg gag ttt ctt ctg caa ggt gga ttt agt ccg gat atc att 2921
   Ala Ala Leu Glu Phe Leu Leu Gln Gly Gly Phe Ser Pro Asp Ile Ile
   890 895 900 905
   cat tgc cat gat tgg tct agt gct cct gtt gct tgg ctc ttt aag gaa 2969
   His Cys His Asp Trp Ser Ser Ala Pro Val Ala Trp Leu Phe Lys Glu
   910 915 920
   caa tat aca cac tat ggt cta agc aaa tct cgt ata gtc ttc acg ata 3017
   Gln Tyr Thr His Tyr Gly Leu Ser Lys Ser Arg Ile Val Phe Thr Ile
   925 930 935
   cat aat ctt gaa ttt ggg gca gat ctc att ggg aga gca atg act aac 3065
   His Asn Leu Glu Phe Gly Ala Asp Leu Ile Gly Arg Ala Met Thr Asn
   940 945 950
   gca gac aaa gct aca aca gtt tca cca act tac tca cag gag gtg tct 3113
   Ala Asp Lys Ala Thr Thr Val Ser Pro Thr Tyr Ser Gln Glu Val Ser
   955 960 965
   gga aac cct gta att gcg cct cac ctt cac aag ttc cat ggt ata gtg     3161
   Gly Asn Pro Val Ile Ala Pro His Leu His Lys Phe His Gly Ile Val
   970                 975                 980                 985
   aat ggg att gac cca gat att tgg gat cct tta aac gat aag ttc att     3209
   Asn Gly Ile Asp Pro Asp Ile Trp Asp Pro Leu Asn Asp Lys Phe Ile
                   990                 995                 1000
   ccg att ccg tac acc tca gaa aac gtt gtt gaa ggc aaa aca gca     3254
   Pro Ile Pro Tyr Thr Ser Glu Asn Val Val Glu Gly Lys Thr Ala
               1005                1010                1015
   gcc aag gaa gct ttg cag cga aaa ctt gga ctg aaa cag gct gac     3299
   Ala Lys Glu Ala Leu Gln Arg Lys Leu Gly Leu Lys Gln Ala Asp
               1020                1025                1030
   ctt cct ttg gta gga att atc acc cgc tta act cac cag aaa gga     3344
   Leu Pro Leu Val Gly Ile Ile Thr Arg Leu Thr His Gln Lys Gly
               1035                1040                1045
   atc cac ctc att aaa cat gct att tgg cgc acc ttg gaa cgg aac     3389
   Ile His Leu Ile Lys His Ala Ile Trp Arg Thr Leu Glu Arg Asn
               1050                1055                1060
   gga cag gta gtc ttg ctt ggt tct gct cct gat cct agg gta caa     3434
   Gly Gln Val Val Leu Leu Gly Ser Ala Pro Asp Pro Arg Val Gln
               1065                1070                1075
   aac gat ttt gtt aat ttg gca aat caa ttg cac tcc aaa tat aat     3479
   Asn Asp Phe Val Asn Leu Ala Asn Gln Leu His Ser Lys Tyr Asn
               1080                1085                1090
   gac cgc gca cga ctc tgt cta aca tat gac gag cca ctt tct cac     3524
   Asp Arg Ala Arg Leu Cys Leu Thr Tyr Asp Glu Pro Leu Ser His
               1095                1100                1105
   ctg ata tat gct ggt gct gat ttt att cta gtt cct tca ata ttt     3569
   Leu Ile Tyr Ala Gly Ala Asp Phe Ile Leu Val Pro Ser Ile Phe
               1110                1115                1120
   gag cca tgt gga cta aca caa ctt acc gct atg aga tat ggt tca     3614
   Glu Pro Cys Gly Leu Thr Gln Leu Thr Ala Met Arg Tyr Gly Ser
               1125                1130                1135
   att cca gtc gtg cgt aaa act gga gga ctt tat gat act gta ttt     3659
   Ile Pro Val Val Arg Lys Thr Gly Gly Leu Tyr Asp Thr Val Phe
               1140                1145                1150
   gat gtt gac cat gac aaa gag aga gca caa cag tgt ggt ctt gaa     3704
   Asp Val Asp His Asp Lys Glu Arg Ala Gln Gln Cys Gly Leu Glu
               1155                1160                1165
   cca aat gga ttc agc ttt gat gga gca gat gct ggc gga gtt gat     3749
   Pro Asn Gly Phe Ser Phe Asp Gly Ala Asp Ala Gly Gly Val Asp
               1170                1175                1180
   tat gct ctg aat aga gct ctc tct gct tgg tac gat ggt cgg gat     3794
   Tyr Ala Leu Asn Arg Ala Leu Ser Ala Trp Tyr Asp Gly Arg Asp
               1185                1190                1195
   tgg ttc aac tct tta tgc aag cag gtc atg gaa caa gat tgg tct     3839
   Trp Phe Asn Ser Leu Cys Lys Gln Val Met Glu Gln Asp Trp Ser
               1200                1205                1210
   tgg aac cga cct gct ctt gat tat ttg gag ctt tac cat gct gct     3884
   Trp Asn Arg Pro Ala Leu Asp Tyr Leu Glu Leu Tyr His Ala Ala
               1215                1220                1225
   aga aag tta gaa  tag ttagtttgtg agatgctagc agaaaaattc acgagatctg    3939
   Arg Lys Leu Glu
               1230
   caatctgtac aggttcagtg tttgcgtctg gacagctttt ttatttccta tatcaaagta    3999
   taaatcaagt ctacactgag atcaatagca gacagtcctc agttcatttc attttttgtg    4059
   caacatatga aagagcttag cctctaataa tgtagtcatt gatgattatt tgttttggga    4119
   agaaatgaga aatcaaagga tgcaaaatac tctgaaaaaa aaaaaaaa                4167

<210>  2

<211>  1230

<212>  PRT

<213>  Solanum tuberosum

<400>  2
   Met Asp Val Pro Phe Pro Leu His Arg Pro Leu Ser Cys Thr Ser Val
   1               5                   10                  15
   Ser Asn Ala Ile Thr His Leu Lys Ile Lys Pro Phe Leu Gly Phe Val
               20                  25                  30
   Ser His Gly Thr Thr Ser Leu Ser Val Gln Ser Ser Ser Trp Arg Lys
           35                  40                  45
   Asp Gly Met Val Thr Gly Val Ser Phe Pro Phe Cys Ala Asn Leu Ser
       50                  55                  60
   Gly Arg Arg Arg Arg Lys Val Ser Thr Thr Arg Ser Gln Gly Ser Ser
   65                  70                  75                  80
   Pro Lys Gly Phe Val Pro Arg Lys Pro Ser Gly Met Ser Thr Gln Arg
                   85                  90                  95
   Lys Val Gln Lys Ser Asn Gly Asp Lys Glu Ser Gln Ser Thr Ser Thr
               100                 105                 110
   Ser Lys Glu Ser Glu Ile Ser Asn Gln Lys Thr Val Glu Ala Arg Val
           115                 120                 125
   Glu Thr Ser Asp Asp Asp Thr Lys Val Val Val Arg Asp His Lys Phe
       130                 135                 140
   Leu Glu Asp Glu Asp Glu Ile Asn Gly Ser Thr Lys Ser Ile Ser Met
   145                 150                 155                 160
   Ser Pro Val Arg Val Ser Ser Gln Phe Val Glu Ser Glu Glu Thr Gly
                   165                 170                 175
   Gly Asp Asp Lys Asp Ala Val Lys Leu Asn Lys Ser Lys Arg Ser Glu
               180                 185                 190
   Glu Ser Asp Phe Leu Ile Asp Ser Val Ile Arg Glu Gln Ser Gly Ser
           195                 200                 205
   Gln Gly Glu Thr Asn Ala Ser Ser Lys Gly Ser His Ala Val Gly Thr
       210                 215                 220
   Lys Leu Tyr Glu Ile Leu Gln Val Asp Val Glu Pro Gln Gln Leu Lys
   225                 230                 235                 240
   Glu Asn Asn Ala Gly Asn Val Glu Tyr Lys Gly Pro Val Ala Ser Lys
                   245                 250                 255
   Leu Leu Glu Ile Thr Lys Ala Ser Asp Val Glu His Thr Glu Ser Asn
               260                 265                 270
   Glu Ile Asp Asp Leu Asp Thr Asn Ser Phe Phe Lys Ser Asp Leu Ile
           275                 280                 285
   Glu Glu Asp Glu Pro Leu Ala Ala Gly Thr Val Glu Thr Gly Asp Ser
       290                 295                 300
   Ser Leu Asn Leu Arg Leu Glu Met Glu Ala Asn Leu Arg Arg Gln Ala
   305                 310                 315                 320
   Ile Glu Arg Leu Ala Glu Glu Asn Leu Leu Gln Gly Ile Arg Leu Phe
                   325                 330                 335
   Cys Phe Pro Glu Val Val Lys Pro Asp Glu Asp Val Glu Ile Phe Leu
               340                 345                 350
   Asn Arg Gly Leu Ser Thr Leu Lys Asn Glu Ser Asp Val Leu Ile Met
           355                 360                 365
   Gly Ala Phe Asn Glu Trp Arg Tyr Arg Ser Phe Thr Thr Arg Leu Thr
       370                 375                 380
   Glu Thr His Leu Asn Gly Asp Trp Trp Ser Cys Lys Ile His Val Pro
   385                 390                 395                 400
   Lys Glu Ala Tyr Arg Ala Asp Phe Val Phe Phe Asn Gly Gln Asp Val
                   405                 410                 415
   Tyr Asp Asn Asn Asp Gly Asn Asp Phe Ser Ile Thr Val Lys Gly Gly
               420                 425                 430
   Met Gln Ile Ile Asp Phe Glu Asn Phe Leu Leu Glu Glu Lys Trp Arg
           435                 440                 445
   Glu Gln Glu Lys Leu Ala Lys Glu Gln Ala Glu Arg Glu Arg Leu Ala
       450                 455                 460
   Glu Glu Gln Arg Arg Ile Glu Ala Glu Lys Ala Glu Ile Glu Ala Asp
   465                 470                 475                 480
   Arg Ala Gln Ala Lys Glu Glu Ala Ala Lys Lys Lys Lys Val Leu Arg
                   485                 490                 495
   Glu Leu Met Val Lys Ala Thr Lys Thr Arg Asp Ile Thr Trp Tyr Ile
               500                 505                 510
   Glu Pro Ser Glu Phe Lys Cys Glu Asp Lys Val Arg Leu Tyr Tyr Asn
           515                 520                 525
   Lys Ser Ser Gly Pro Leu Ser His Ala Lys Asp Leu Trp Ile His Gly
       530                 535                 540
   Gly Tyr Asn Asn Trp Lys Asp Gly Leu Ser Ile Val Lys Lys Leu Val
   545                 550                 555                 560
   Lys Ser Glu Arg Ile Asp Gly Asp Trp Trp Tyr Thr Glu Val Val Ile
                   565                 570                 575
   Pro Asp Gln Ala Leu Phe Leu Asp Trp Val Phe Ala Asp Gly Pro Pro
               580                 585                 590
   Lys His Ala Ile Ala Tyr Asp Asn Asn His Arg Gln Asp Phe His Ala
           595                 600                 605
   Ile Val Pro Asn His Ile Pro Glu Glu Leu Tyr Trp Val Glu Glu Glu
       610                 615                 620
   His Gln Ile Phe Lys Thr Leu Gln Glu Glu Arg Arg Leu Arg Glu Ala
   625                 630                 635                 640
   Ala Met Arg Ala Lys Val Glu Lys Thr Ala Leu Leu Lys Thr Glu Thr
                   645                 650                 655
   Lys Glu Arg Thr Met Lys Ser Phe Leu Leu Ser Gln Lys His Val Val
               660                 665                 670
   Tyr Thr Glu Pro Leu Asp Ile Gln Ala Gly Ser Ser Val Thr Val Tyr
           675                 680                 685
   Tyr Asn Pro Ala Asn Thr Val Leu Asn Gly Lys Pro Glu Ile Trp Phe
       690                 695                 700
   Arg Cys Ser Phe Asn Arg Trp Thr His Arg Leu Gly Pro Leu Pro Pro
   705                 710                 715                 720
   Gln Lys Met Ser Pro Ala Glu Asn Gly Thr His Val Arg Ala Thr Val
                   725                 730                 735
   Lys Val Pro Leu Asp Ala Tyr Met Met Asp Phe Val Phe Ser Glu Arg
               740                 745                 750
   Glu Asp Gly Gly Ile Phe Asp Asn Lys Ser Gly Met Asp Tyr His Ile
           755                 760                 765
   Pro Val Phe Gly Gly Val Ala Lys Glu Pro Pro Met His Ile Val His
       770                 775                 780
   Ile Ala Val Glu Met Ala Pro Ile Ala Lys Val Gly Gly Leu Gly Asp
   785                 790                 795                 800
   Val Val Thr Ser Leu Ser Arg Ala Val Gln Asp Leu Asn His Asn Val
                   805                 810                 815
   Asp Ile Ile Leu Pro Lys Tyr Asp Cys Leu Lys Met Asn Asn Val Lys
               820                 825                 830
   Asp Phe Arg Phe His Lys Asn Tyr Phe Trp Gly Gly Thr Glu Ile Lys
           835                 840                 845
   Val Trp Phe Gly Lys Val Glu Gly Leu Ser Val Tyr Phe Leu Glu Pro
       850                 855                 860
   Gln Asn Gly Leu Phe Ser Lys Gly Cys Val Tyr Gly Cys Ser Asn Asp
   865                 870                 875                 880
   Gly Glu Arg Phe Gly Phe Phe Cys His Ala Ala Leu Glu Phe Leu Leu
                   885                 890                 895
   Gln Gly Gly Phe Ser Pro Asp Ile Ile His Cys His Asp Trp Ser Ser
               900                 905                 910
   Ala Pro Val Ala Trp Leu Phe Lys Glu Gln Tyr Thr His Tyr Gly Leu
           915                 920                 925
   Ser Lys Ser Arg Ile Val Phe Thr Ile His Asn Leu Glu Phe Gly Ala
       930                 935                 940
   Asp Leu Ile Gly Arg Ala Met Thr Asn Ala Asp Lys Ala Thr Thr Val
   945                 950                 955                 960
   Ser Pro Thr Tyr Ser Gln Glu Val Ser Gly Asn Pro Val Ile Ala Pro
                   965                 970                 975
   His Leu His Lys Phe His Gly Ile Val Asn Gly Ile Asp Pro Asp Ile
               980                 985                 990
   Trp Asp Pro Leu Asn Asp Lys Phe Ile Pro Ile Pro Tyr Thr Ser Glu
           995                 1000                1005
   Asn Val Val Glu Gly Lys Thr Ala Ala Lys Glu Ala Leu Gln Arg
       1010                1015                1020
   Lys Leu Gly Leu Lys Gln Ala Asp Leu Pro Leu Val Gly Ile Ile
       1025                1030                1035
   Thr Arg Leu Thr His Gln Lys Gly Ile His Leu Ile Lys His Ala
       1040                1045                1050
   Ile Trp Arg Thr Leu Glu Arg Asn Gly Gln Val Val Leu Leu Gly
       1055                1060                1065
   Ser Ala Pro Asp Pro Arg Val Gln Asn Asp Phe Val Asn Leu Ala
       1070                1075                1080
   Asn Gln Leu His Ser Lys Tyr Asn Asp Arg Ala Arg Leu Cys Leu
       1085                1090                1095
   Thr Tyr Asp Glu Pro Leu Ser His Leu Ile Tyr Ala Gly Ala Asp
       1100                1105                1110
   Phe Ile Leu Val Pro Ser Ile Phe Glu Pro Cys Gly Leu Thr Gln
       1115                1120                1125
   Leu Thr Ala Met Arg Tyr Gly Ser Ile Pro Val Val Arg Lys Thr
       1130                1135                1140
   Gly Gly Leu Tyr Asp Thr Val Phe Asp Val Asp His Asp Lys Glu
       1145                1150                1155
   Arg Ala Gln Gln Cys Gly Leu Glu Pro Asn Gly Phe Ser Phe Asp
       1160                1165                1170
   Gly Ala Asp Ala Gly Gly Val Asp Tyr Ala Leu Asn Arg Ala Leu
       1175                1180                1185
   Ser Ala Trp Tyr Asp Gly Arg Asp Trp Phe Asn Ser Leu Cys Lys
       1190                1195                1200
   Gln Val Met Glu Gln Asp Trp Ser Trp Asn Arg Pro Ala Leu Asp
       1205                1210                1215
   Tyr Leu Glu Leu Tyr His Ala Ala Arg Lys Leu Glu
       1220                1225                1230

<210>  3

<211>  61

<212>  PRT

<213>  Solanum tuberosum

<400>  3

Arg Ser Phe Thr Thr Arg Leu Thr Glu Thr His Leu Asn Gly Asp Trp
1               5                   10                  15

Trp Ser Cys Lys Ile His Val Pro Lys Glu Ala Tyr Arg Ala Asp Phe
            20                  25                  30

Val Phe Phe Asn Gly Gln Asp Val Tyr Asp Asn Asn Asp Gly Asn Asp
        35                  40                  45

Phe Ser Ile Thr Val Lys Gly Gly Met Gln Ile Ile Asp
    50                  55                  60

<210>  4

<211>  1641

<212>  DNA

<213>  Solanum tuberosum

<400>  4
atgaagcaca gttcagctat ttccgctgtt ttgaccgatg acaattcgac aatggcaccc      60

ctagaggaag atgtcaacac tgaaaatatt ggcctcctaa atttggatcc aactttggaa     120

ccttatctag atcacttcag acacagaatg aagagatatg tggatcagaa aatgctcatt     180

gaaaaatatg agggacccct tgaggaattt gctcaaggtt atttaaaatt tggattcaac     240

agggaagatg gttgcatagt ctatcgtgaa tgggctcctg ctgctcagga agcagaagtt     300

attggcgatt tcaatggtag gaacggttct aaccacatga tggagaagga ccagtttggt     360

gtttggagta ttagaattcc tgatgttgac agtaagccag tcattccaca caactccaga     420

gttaagtttc gtttcaaaca tggtaatgga gtgtgggtag atcgtatccc tgcttggata     480

aagtatgcca ctgcagacgc cacaaagttt gcagcaccat atgatggtgt ctactgggac     540

ccaccacctt cagaaaggta ccacttcaaa taccctcgcc ctcccaaacc ccgagcccca     600

cgaatctacg aagcacatgt cggcatgagc agctctgagc cacgtgtaaa ttcgtatcgt     660

gagtttgcag atgatgtttt acctcggatt aaggcaaata actataatac tgtccagttg     720

atggccataa tggaacattc ttactatgga tcatttggat atcatgttac aaactttttt     780

gctgtgagca atagatatgg aaacccggag gacctaaagt atctgataga taaagcacat     840

agcttgggtt tacaggttct ggtggatgta gttcacagtc atgcaagcaa taatgtcact     900

gatggcctca atggctttga tattggccaa ggttctcaag aatcctactt tcatgctgga     960

gagcgagggt accataagtt gtgggatagc aggctgttca actatgccaa ttgggaggtt 1020
cttcgtttcc ttctttccaa cttgaggtgg tggctagaag agtataactt tgacggattt 1080
cgatttgatg gaataacttc tatgctgtat gttcatcatg gaatcaatat gggatttaca 1140
ggaaactata atgagtattt cagcgaggct acagatgttg atgctgtggt ctatttaatg 1200
ttggccaata atctgattca caagattttc ccagacgcaa ctgttattgc cgaagatgtt 1260
tctggtatgc cgggccttag ccggcctgtt tctgagggag gaattggttt tgattaccgc 1320
ctggcaatgg caatcccaga taagtggata gattatttaa agaataagaa tgatgaagat 1380
tggtccatga aggaagtaac atcgagtttg acaaatagga gatatacaga gaagtgtata 1440
gcatatgcgg agagccatga tcagtctatt gtcggtgaca agaccattgc atttctccta 1500
atgaacaaag agatgtattc tggcatgtct tgcttgacag atgcttctcc tgttgttgat 1560
gcaggaattg cgcttgacaa gatgatccat ttttttcaca atggccttgg gaggagaggg 1620
gtacctcaat ttcatgggta a 1641

<210> 5

<211> 546

<212> PRT

<213> Solanum tuberosum

<300>

<308> Swiss Prot / P30924

<309> 1993-07-26

<400> 5

Met Lys His Ser Ser Ala Ile Ser Ala Val Leu Thr Asp Asp Asn Ser
1               5                   10                  15

Thr Met Ala Pro Leu Glu Glu Asp Val Asn Thr Glu Asn Ile Gly Leu
            20                  25                  30

Leu Asn Leu Asp Pro Thr Leu Glu Pro Tyr Leu Asp His Phe Arg His
        35                  40                  45

Arg Met Lys Arg Tyr Val Asp Gln Lys Met Leu Ile Glu Lys Tyr Glu
    50                  55                  60

Gly Pro Leu Glu Glu Phe Ala Gln Gly Tyr Leu Lys Phe Gly Phe Asn
65                  70                  75                  80

Arg Glu Asp Gly Cys Ile Val Tyr Arg Glu Trp Ala Pro Ala Ala Gln
                85                  90                  95

Glu Ala Glu Val Ile Gly Asp Phe Asn Gly Arg Asn Gly Ser Asn His
            100                 105                 110

Met Met Glu Lys Asp Gln Phe Gly Val Trp Ser Ile Arg Ile Pro Asp
        115                 120                 125

Val Asp Ser Lys Pro Val Ile Pro His Asn Ser Arg Val Lys Phe Arg
    130                 135                 140

Phe Lys His Gly Asn Gly Val Trp Val Asp Arg Ile Pro Ala Trp Ile
145                 150                 155                 160

Lys Tyr Ala Thr Ala Asp Ala Thr Lys Phe Ala Ala Pro Tyr Asp Gly
                165                 170                 175

Val Tyr Trp Asp Pro Pro Pro Ser Glu Arg Tyr His Phe Lys Tyr Pro
            180                 185                 190

Arg Pro Pro Lys Pro Arg Ala Pro Arg Ile Tyr Glu Ala His Val Gly
        195                 200                 205

Met Ser Ser Ser Glu Pro Arg Val Asn Ser Tyr Arg Glu Phe Ala Asp
    210                 215                 220

Asp Val Leu Pro Arg Ile Lys Ala Asn Asn Tyr Asn Thr Val Gln Leu
225                 230                 235                 240

Met Ala Ile Met Glu His Ser Tyr Tyr Gly Ser Phe Gly Tyr His Val
                245                 250                 255

Thr Asn Phe Phe Ala Val Ser Asn Arg Tyr Gly Asn Pro Glu Asp Leu
            260                 265                 270

Lys Tyr Leu Ile Asp Lys Ala His Ser Leu Gly Leu Gln Val Leu Val
        275                 280                 285

Asp Val Val His Ser His Ala Ser Asn Asn Val Thr Asp Gly Leu Asn
    290                 295                 300

Gly Phe Asp Ile Gly Gln Gly Ser Gln Glu Ser Tyr Phe His Ala Gly
305                 310                 315                 320

Glu Arg Gly Tyr His Lys Leu Trp Asp Ser Arg Leu Phe Asn Tyr Ala
                325                 330                 335

Asn Trp Glu Val Leu Arg Phe Leu Leu Ser Asn Leu Arg Trp Trp Leu
            340                 345                 350

Glu Glu Tyr Asn Phe Asp Gly Phe Arg Phe Asp Gly Ile Thr Ser Met
        355                 360                 365

Leu Tyr Val His His Gly Ile Asn Met Gly Phe Thr Gly Asn Tyr Asn
    370                 375                 380

Glu Tyr Phe Ser Glu Ala Thr Asp Val Asp Ala Val Val Tyr Leu Met
385                 390                 395                 400

Leu Ala Asn Asn Leu Ile His Lys Ile Phe Pro Asp Ala Thr Val Ile
                405                 410                 415

Ala Glu Asp Val Ser Gly Met Pro Gly Leu Ser Arg Pro Val Ser Glu
            420                 425                 430

Gly Gly Ile Gly Phe Asp Tyr Arg Leu Ala Met Ala Ile Pro Asp Lys
        435                 440                 445

Trp Ile Asp Tyr Leu Lys Asn Lys Asn Asp Glu Asp Trp Ser Met Lys
    450                 455                 460

Glu Val Thr Ser Ser Leu Thr Asn Arg Arg Tyr Thr Glu Lys Cys Ile
465                 470                 475                 480

Ala Tyr Ala Glu Ser His Asp Gln Ser Ile Val Gly Asp Lys Thr Ile
                485                 490                 495

Ala Phe Leu Leu Met Asn Lys Glu Met Tyr Ser Gly Met Ser Cys Leu
            500                 505                 510

Thr Asp Ala Ser Pro Val Val Asp Ala Gly Ile Ala Leu Asp Lys Met
        515                 520                 525

Ile His Phe Phe His Asn Gly Leu Gly Arg Arg Gly Val Pro Gln Phe
    530                 535                 540

His Gly
545

<210>  6

<211>  2649

<212>  DNA

<213>  Solanum tuberosum


<300>

<308>  EMBL / AJ011890

<309>  1999-04-07

<300>

<302> Improvments in or relating to plant starch composition

<308> EMBL / A58164

<309> 1998-03-05

<310> WO 96 34968

<311> 1996-05-03

<312> 1996-11-07

<400> 6
atggtgtata cactctctgg agttcgtttt cctactgttc catcagtgta caaatctaat 60
ggattcagca gtaatggtga tcggaggaat gctaatgttt ctgtattctt gaaaaagcac 120
tctctttcac ggaagatctt ggctgaaaag tcttcttaca attccgaatt ccgaccttct 180
acagttgcag catcggggaa agtccttgtg cctggaaccc agagtgatag ctcctcatcc 240
tcaacagacc aatttgagtt cactgagaca tctccagaaa attccccagc atcaactgat 300
gtagatagtt caacaatgga acacgctagc cagattaaaa ctgagaacga tgacgttgag 360
ccgtcaagtg atcttacagg aagtgttgaa gagctggatt ttgcttcatc actacaacta 420
caagaaggtg gtaaactgga ggagtctaaa acattaaata cttctgaaga gacaattatt 480
gatgaatctg ataggatcag agagaggggc atccctccac ctggacttgg tcagaagatt 540
tatgaaatag accccctttt gacaaactat cgtcaacacc ttgattacag gtattcacag 600
tacaagaaac tgagggaggc aattgacaag tatgagggtg gtttggaagc cttttctcgt 660
ggttatgaaa aaatgggttt cactcgtagt gctacaggta tcacttaccg tgagtgggct 720
cttggtgccc agtcagctgc cctcattgga gatttcaaca attgggacgc aaatgctgac 780
attatgactc ggaatgaatt tggtgtctgg gagatttttc tgccaaataa tgtggatggt 840
tctcctgcaa ttcctcatgg gtccagagtg aagatacgta tggacactcc atcaggtgtt 900
aaggattcca ttcctgcttg gatcaactac tctttacagc ttcctgatga aattccatat 960
aatggaatac attatgatcc acccgaagag gagaggtata tcttccaaca cccacggcca 1020
aagaaaccaa agtcgctgag aatatatgaa tctcatattg gaatgagtag tccggagcct 1080
aaaattaact catacgtgaa ttttagagat gaagttcttc ctcgcataaa aaagcttggg 1140
tacaatgcgc tgcaaattat ggctattcaa gagcattctt attacgctag ttttggttat 1200
catgtcacaa atttttttgc accaagcagc cgttttggaa cgcccgacga ccttaagtct 1260
ttgattgata aagctcatga gctaggaatt gttgttctca tggacattgt tcacagccat 1320
gcatcaaata atactttaga tggactgaac atgtttgact gcaccgatag ttgttacttt 1380
cactctggag ctcgtggtta tcattggatg tgggattccc gcctctttaa ctatggaaac 1440
tgggaggtac ttaggtatct tctctcaaat gcgagatggt ggttggatgc gttcaaattt 1500
gatggattta gatttgatgg tgtgacatca atgatgtata ttcaccacgg attatcggtg 1560

ggattcactg ggaactacga ggaatacttt ggactcgcaa ctgatgtgga tgctgttgtg 1620
tatctgatgc tggtcaacga tcttattcat gggcttttcc cagatgcaat taccattggt 1680
gaagatgtta gcggaatgcc gacattttgt attcccgtcc aagagggggg tgttggcttt 1740
gactatcggc tgcatatggc aattgctgat aaacggattg agttgctcaa gaaacgggat 1800
gaggattgga gagtgggtga tattgttcat acactgacaa atagaagatg gtcggaaaag 1860
tgtgtttcat acgctgaaag tcatgatcaa gctctagtcg gtgataaaac tatagcattc 1920
tggctgatgg acaaggatat gtatgatttt atggctctgg atagaccgtc aacatcatta 1980
atagatcgtg ggatagcatt gcacaagatg attaggcttg taactatggg attaggagga 2040
gaagggtacc taaatttcat gggaaatgaa ttcggccacc ctgagtggat tgatttccct 2100
agggctgaac aacacctctc tgatggctca gtaatccccg gaaaccaatt ccgttatgat 2160
aaatgcagac ggagatttga cctgggagat gcagaatatt taagataccg tgggttgcaa 2220
gaatttgacc ggcctatgca gtatcttgaa gataaatatg agtttatgac ttcagaacac 2280
cagttcatat cacgaaagga tgaaggagat aggatgattg tatttgaaaa aggaaaccta 2340
gtttttgtct ttaattttca ctggacaaaa agctattcag actatcgcat agcctgcctg 2400
aagcctggaa aatacccggt tgccttggac tcagatgatc cactttttgg tggcttcggg 2460
agaattgatc ataatgccga atatttcacc tttgaaggat ggtatgatga tcgtcctcgt 2520
tcaattatgg tgtatgcacc ttgtaaaaca gcagtggtct atgcactagt agacaaagaa 2580
gaagaagaag aagaagaaga agaagaagaa gtagcagcag tagaagaagt agtagtagaa 2640
gaagaatga 2649

<210> 7
<211> 882
<212> PRT
<213> Solanum tuberosum

<400> 7

Met Val Tyr Thr Leu Ser Gly Val Arg Phe Pro Thr Val Pro Ser Val
1               5                   10                  15

Tyr Lys Ser Asn Gly Phe Ser Ser Asn Gly Asp Arg Arg Asn Ala Asn
            20                  25                  30

Val Ser Val Phe Leu Lys Lys His Ser Leu Ser Arg Lys Ile Leu Ala
        35                  40                  45

Glu Lys Ser Ser Tyr Asn Ser Glu Phe Arg Pro Ser Thr Val Ala Ala
    50                  55                  60

Ser Gly Lys Val Leu Val Pro Gly Thr Gln Ser Asp Ser Ser Ser Ser
65                  70                  75                  80

Ser Thr Asp Gln Phe Glu Phe Thr Glu Thr Ser Pro Glu Asn Ser Pro
                85                  90                  95

Ala Ser Thr Asp Val Asp Ser Ser Thr Met Glu His Ala Ser Gln Ile
            100                 105                 110

Lys Thr Glu Asn Asp Asp Val Glu Pro Ser Ser Asp Leu Thr Gly Ser
        115                 120                 125

Val Glu Glu Leu Asp Phe Ala Ser Ser Leu Gln Leu Gln Glu Gly Gly
    130                 135                 140

Lys Leu Glu Glu Ser Lys Thr Leu Asn Thr Ser Glu Glu Thr Ile Ile
145                 150                 155                 160

Asp Glu Ser Asp Arg Ile Arg Glu Arg Gly Ile Pro Pro Pro Gly Leu
                165                 170                 175

Gly Gln Lys Ile Tyr Glu Ile Asp Pro Leu Leu Thr Asn Tyr Arg Gln
            180                 185                 190

His Leu Asp Tyr Arg Tyr Ser Gln Tyr Lys Lys Leu Arg Glu Ala Ile
        195                 200                 205

Asp Lys Tyr Glu Gly Gly Leu Glu Ala Phe Ser Arg Gly Tyr Glu Lys
    210                 215                 220

Met Gly Phe Thr Arg Ser Ala Thr Gly Ile Thr Tyr Arg Glu Trp Ala
225                 230                 235                 240

Leu Gly Ala Gln Ser Ala Ala Leu Ile Gly Asp Phe Asn Asn Trp Asp
                245                 250                 255

Ala Asn Ala Asp Ile Met Thr Arg Asn Glu Phe Gly Val Trp Glu Ile
            260                 265                 270

Phe Leu Pro Asn Asn Val Asp Gly Ser Pro Ala Ile Pro His Gly Ser
        275                 280                 285

Arg Val Lys Ile Arg Met Asp Thr Pro Ser Gly Val Lys Asp Ser Ile
    290                 295                 300

Pro Ala Trp Ile Asn Tyr Ser Leu Gln Leu Pro Asp Glu Ile Pro Tyr
305                 310                 315                 320

Asn Gly Ile His Tyr Asp Pro Pro Glu Glu Glu Arg Tyr Ile Phe Gln
                325                 330                 335

His Pro Arg Pro Lys Lys Pro Lys Ser Leu Arg Ile Tyr Glu Ser His
            340                 345                 350

Ile Gly Met Ser Ser Pro Glu Pro Lys Ile Asn Ser Tyr Val Asn Phe
        355                 360                 365

Arg Asp Glu Val Leu Pro Arg Ile Lys Lys Leu Gly Tyr Asn Ala Leu
    370                 375                 380

Gln Ile Met Ala Ile Gln Glu His Ser Tyr Tyr Ala Ser Phe Gly Tyr
385                 390                 395                 400

His Val Thr Asn Phe Phe Ala Pro Ser Ser Arg Phe Gly Thr Pro Asp
                405                 410                 415

Asp Leu Lys Ser Leu Ile Asp Lys Ala His Glu Leu Gly Ile Val Val
            420                 425                 430

Leu Met Asp Ile Val His Ser His Ala Ser Asn Asn Thr Leu Asp Gly
        435                 440                 445

Leu Asn Met Phe Asp Cys Thr Asp Ser Cys Tyr Phe His Ser Gly Ala
    450                 455                 460

Arg Gly Tyr His Trp Met Trp Asp Ser Arg Leu Phe Asn Tyr Gly Asn
465                 470                 475                 480

Trp Glu Val Leu Arg Tyr Leu Leu Ser Asn Ala Arg Trp Trp Leu Asp
                485                 490                 495

Ala Phe Lys Phe Asp Gly Phe Arg Phe Asp Gly Val Thr Ser Met Met
            500                 505                 510

Tyr Ile His His Gly Leu Ser Val Gly Phe Thr Gly Asn Tyr Glu Glu
        515                 520                 525

Tyr Phe Gly Leu Ala Thr Asp Val Asp Ala Val Val Tyr Leu Met Leu
    530                 535                 540

Val Asn Asp Leu Ile His Gly Leu Phe Pro Asp Ala Ile Thr Ile Gly
545                 550                 555                 560

Glu Asp Val Ser Gly Met Pro Thr Phe Cys Ile Pro Val Gln Glu Gly
                565                 570                 575

Gly Val Gly Phe Asp Tyr Arg Leu His Met Ala Ile Ala Asp Lys Arg
            580                 585                 590

Ile Glu Leu Leu Lys Lys Arg Asp Glu Asp Trp Arg Val Gly Asp Ile
        595                 600                 605

Val His Thr Leu Thr Asn Arg Arg Trp Ser Glu Lys Cys Val Ser Tyr
    610                 615                 620

Ala Glu Ser His Asp Gln Ala Leu Val Gly Asp Lys Thr Ile Ala Phe
625                 630                 635                 640

Trp Leu Met Asp Lys Asp Met Tyr Asp Phe Met Ala Leu Asp Arg Pro
                645                 650                 655

Ser Thr Ser Leu Ile Asp Arg Gly Ile Ala Leu His Lys Met Ile Arg
            660                 665                 670

Leu Val Thr Met Gly Leu Gly Gly Glu Gly Tyr Leu Asn Phe Met Gly
        675                 680                 685

Asn Glu Phe Gly His Pro Glu Trp Ile Asp Phe Pro Arg Ala Glu Gln
    690                 695                 700

His Leu Ser Asp Gly Ser Val Ile Pro Gly Asn Gln Phe Arg Tyr Asp
705                 710                 715                 720

Lys Cys Arg Arg Arg Phe Asp Leu Gly Asp Ala Glu Tyr Leu Arg Tyr
                725                 730                 735

Arg Gly Leu Gln Glu Phe Asp Arg Pro Met Gln Tyr Leu Glu Asp Lys
            740                 745                 750

Tyr Glu Phe Met Thr Ser Glu His Gln Phe Ile Ser Arg Lys Asp Glu
        755                 760                 765

Gly Asp Arg Met Ile Val Phe Glu Lys Gly Asn Leu Val Phe Val Phe
    770                 775                 780

Asn Phe His Trp Thr Lys Ser Tyr Ser Asp Tyr Arg Ile Ala Cys Leu
785                 790                 795                 800

Lys Pro Gly Lys Tyr Pro Val Ala Leu Asp Ser Asp Asp Pro Leu Phe
                805                 810                 815

Gly Gly Phe Gly Arg Ile Asp His Asn Ala Glu Tyr Phe Thr Phe Glu
            820                 825                 830

Gly Trp Tyr Asp Asp Arg Pro Arg Ser Ile Met Val Tyr Ala Pro Cys
        835                 840                 845

Lys Thr Ala Val Val Tyr Ala Leu Val Asp Lys Glu Glu Glu Glu Glu
    850                 855                 860

Glu Glu Glu Glu Glu Glu Val Ala Ala Val Glu Glu Val Val Val Glu
865                 870                 875                 880

Glu Glu

<210> 8
<211> 1255
<212> DNA
<213> Solanum tuberosum

<400> 8
attttgtatt cccgttcaag atgggggtgt tggctttgac tatcggctgc atatggcaat 60
tgctgataaa tggattgagt tgctcaagaa acgggatgag gattggagag tgggtgatat 120
tgttcataca ctgacaaata gaagatggtc ggaaaagtgt gtttcatacg ctgaaagtca 180
tgatcaagct ctagtcggtg ataaaactat agcattctgg ctgatggaca aggatatgta 240
tgattttatg gctttggata gaccgtcaac atcattaata gatcgtggga tagcattgca 300
caagatgatt aggcttgtaa ctatgggatt aggaggagaa gggtacctaa atttcatggg 360
aaatgaattc ggccaccctg agtggattga tttccctagg gctgaacaac acctctctga 420
tggctcagta attcccggaa accaattcag ttatgataaa tgcagacgga gatttgacct 480
gggagatgca gaatatttaa gataccgtgg gttgcaagaa tttgaccggg ctatgcagta 540
tcttgaagat aaatatgagt ttatgacttc agaacaccag ttcatatcac gaaaggatga 600
aggagatagg atgattgtat ttgaaaaagg aaacctagtt tttgtcttta attttcactg 660
gacaaaaagc tattcagact atcgcatagg ctgcctgaag cctggaaaat acaaggttgc 720
cttggactca gatgatccac tttttggtgg cttcgggaga attgatcata atgccgaatg 780
tttcaccttt gaaggatggt atgatgatcg tcctcgttca attatggtgt atgcacctag 840
tagaacagca gtggtctatg cactagtaga caaagaagaa gaagaagaag aagtagcagt 900
agtagaagaa gtagtagtag aagaagaatg aacgaacttg tgatcgcgtt gaaagatttg 960
aacgctacat agagcttctt gacgtatctg gcaatattgc atcagtcttg gcggaatttc 1020
atgtgacaaa aggtttgcaa ttctttccac tattagtagt gcaacgatat acgcagagat 1080
gaagtgctga acaaacatat gtaaaatcga tgaatttatg tcgaatgctg ggacgggctt 1140
cagcaggttt tgcttagtga gttctgtaaa ttgtcatctc tttatatgta cagccaacta 1200
gaaatcaatt atgtgagacc taaaatacaa taaccataaa atggaaatag tgctg 1255

<210> 9
<211> 1004
<212> DNA
<213> Solanum tuberosum

<400> 9
tcaaactagt cacaaccagt ccatttctgg aggtcgttcc ttcgcagaaa tactgattgg 60
taactccttg gggaaatcct ccatatcaca agagtcatta cttagaggct gctcgttaca 120
caagatgatc agattaatta catctacaat tggtggtcat gcatacctca acttcatggg 180
caatgaattt ggtcacccaa agagagtaga gtttccaatg tcaagcaaca atttctcctt 240
ttcactggct aaccgtcgct gggatctatt ggaagatgtt gtacattatc aattgttctc 300
atttgataag ggtatgatgg acttggataa aaatgggaga attttgtcca gaggtcttgc 360
caacattcac catgtcaatg atactaccat ggtgatttct tacttgagag gtcccaatct 420
ctttgtgttc aactttcatc ctgtcaattc atatgaaaga tacattatag gtgtggaaga 480
agctggagag tatcaagtca cattaaatac agatgaaaac aagtatggtg gtagaggact 540
acttggccat gatcagaata ttcaaagaac cattagtaga agagctgatg gaatgagatt 600
ttgcttggaa gtgcctctgc caagtagaag tgctcaggtc tacaagttga cccgaattct 660
aagagcatga tcactctagt aatcaaagtg cctcatatga tgacacaaaa ggaaaggttc 720
tacattgccc ttacactgat caatattgac acctttccga ggtgagtttc tgtgattctt 780
gagcagactg ttggctagtc aattatcatg aacttttgcc ttcagcatcc ggatagtcgc 840
ttctcctgtg caatgagggc atggacgaat ttttttttgg cttgtcatgg gggtcataag 900
catccgccag attaagattt cacaggcctc gagtaaaacc atcacttact ttaaggatac 960
acaaacacac caacggggtg caggctctga taccttctaa agtg 1004

<210> 10
<211> 2096
<212> DNA
<213> Solanum tuberosum

<400> 10
aacaatgctc tctctgtcgg attcaattcg aatttcttca ccattgagcg attctcgtct 60
tagttttcta tctcaaaccg gaagcagaac cagtcgccag cttaaatttg ttcgcagccg 120
ccgggctcga gtttcgaggt gtagatgctc agcaacggag caaccgccac cgcaacgacg 180
gaagcaacga ccggagaagt acaaacagtc ggaggaaggg aaaggaatcg atcctgttgg 240
atttctcagc aaatacggca ttactcataa agcgtttgct caatttcttc gtgaaagata 300
taaatcattg aaggacttga aggatgaaat attgactcgt catttcagtc tcaaggagat 360
gtctactggg tatgaattaa tgggtatgca tcgcaacata caacatcgag tggatttctt 420
ggaatgggct ccaggtgctc gctactgtgc tctgattggt gacttcaatg ggtggtcaac 480
aactggtaac tgtgccagag agggtcattt tggtcatgac gattatgggt attggtttat 540
tattcttgaa gataaattac gtgaaggaga agaacctgat aaattgtatt ttcaacagta 600
caattatgcg gaggactatg gtaaaggtga cacgggtatt accgtcgagg aaatctttaa 660

aaaagcaaat gatgagtatt gggaacctgg agaagatcgc ttcattaaat cacgttatga 720
ggtggcagca aagttatatg aggaaatgtt cggaccaaat ggacctcaaa cagaagagga 780
actagaagca atgcctgatg cagctacacg atacaaaact tggaaagagc aacaaaaaga 840
ggatccggca agcaatttgc catcgtatga tgtggtagat agtggaaaag aatatgatat 900
ttacaatatt ataggtgatc ctgaatcgtt taagaaattt cgtatgaaac agcctcctat 960
tgcttactgg ttagaaacta aaaagggaag gaaaggctgg ttacagaaat atatgcctgc 1020
tttacctcat ggaagcaaat acagggtgta ttttaacaca ccaaatgggc ctcttgaacg 1080
agttcctgcg tgggccaatt ttgtcattcc agatgcaggc gggatggcat tagcagtcca 1140
ttgggaacca cctcctgaat atgcttataa atggaaacac aagctaccag tcaagcctaa 1200
gtccttgcgc atatatgaat gtcatgttgg catctctggc caggaaccaa aagtttcatc 1260
tttcaatgat tttattagca aggtccttcc gcatgtaaaa gaagctggat acaatgcaat 1320
acaaattatt ggagttgttg agcacaagga ttatttcact gttggatata gagtgaccaa 1380
tttttatgct gttagtagcc gttatggcac accggatgac ttcaagcgct tggttgatga 1440
agcacatggg cttggactgc ttgtcttttt ggagattgtg cactcttatg cagcagcaga 1500
tgaaatggtt gggttatctc tttttgatgg agcaaatgat tgctatttcc acactggtaa 1560
acgtggacac cacaaattct ggggcacacg gatgttcaaa tatggagatc ttgatgttct 1620
gcactttctt ctttcaaatc tgaactggtg ggtggaggag tatcatgtcg atggcttcca 1680
ttttcattcg ctctcgtcca tgttgtatac gcataatgga tttgcttcat ttactggtga 1740
catggatgaa tactgtaacc aatatgttga caaggaggcc ttattgtacc tcatattagc 1800
aaatgaagta ttacatgctc ttcatcctaa tgtgatcacg attgctgagg atgcaactct 1860
gtatcctgga ctctgcgatc caacatctca aggtggactg ggctttgatt attttgccaa 1920
tctttctgcc tcagagatgt ggcttgcatt acttgaaaat actcctgatc atgaatggtg 1980
catgagtaag attgttagca cattagtggg cgatagacaa aatactgata aaatgctttt 2040
gtatgcagaa aatcacaacc agtccatttc tggaggtcgt tccttcgcag aaatac 2096

<210> 11
<211> 3204
<212> DNA
<213> Solanum tuberosum

<220>
<221> CDS
<222> (99)..(2804)
<223>

<400> 11
gaattgtaat acgactcact atagggcgaa ttgggccctc tagatgcatg ctcgagcggc 60

cgccagtgtg atggatatct gcagaattcg gcttaaca atg ctc tct ctg tcg gat 116
Met Leu Ser Leu Ser Asp
1               5

tca att cga att tct tca cca ttg agc gat tct cgt ctt agt ttt cta 164
Ser Ile Arg Ile Ser Ser Pro Leu Ser Asp Ser Arg Leu Ser Phe Leu
            10                  15                  20

tct caa acc gga agc aga acc agt cgc cag ctt aaa ttt gtt cgc agc 212
Ser Gln Thr Gly Ser Arg Thr Ser Arg Gln Leu Lys Phe Val Arg Ser
        25                  30                  35

cgc cgg gct cga gtt tcg agg tgt aga tgc tca gca acg gag caa ccg 260
Arg Arg Ala Arg Val Ser Arg Cys Arg Cys Ser Ala Thr Glu Gln Pro
    40                  45                  50

cca ccg caa cga cgg aag caa cga ccg gag aag tac aaa cag tcg gag 308
Pro Pro Gln Arg Arg Lys Gln Arg Pro Glu Lys Tyr Lys Gln Ser Glu
55                  60                  65                  70

gaa gag aaa gga atc gat cct gtt gga ttt ctc agc aaa tac ggc att 356
Glu Glu Lys Gly Ile Asp Pro Val Gly Phe Leu Ser Lys Tyr Gly Ile
                75                  80                  85

act cat aaa gcg ttt gct caa ttt ctt cgt gaa aga tat aaa tca ttg 404
Thr His Lys Ala Phe Ala Gln Phe Leu Arg Glu Arg Tyr Lys Ser Leu
            90                  95                  100

aag gac ttg aag gat gaa ata ttg act cgt cat ttc agt ctc aag gag 452
Lys Asp Leu Lys Asp Glu Ile Leu Thr Arg His Phe Ser Leu Lys Glu
        105                 110                 115

atg tct act ggg tat gaa tta atg ggt atg cat cgc aac ata caa cat 500
Met Ser Thr Gly Tyr Glu Leu Met Gly Met His Arg Asn Ile Gln His
    120                 125                 130

cga gtg gat ttc ttg gaa tgg gct cca ggt gct cgc tac tgt gct ctg 548
Arg Val Asp Phe Leu Glu Trp Ala Pro Gly Ala Arg Tyr Cys Ala Leu
135                 140                 145                 150

att ggt gac ttc aat ggg tgg tca aca act ggt aac tgt gcc aga gag 596
Ile Gly Asp Phe Asn Gly Trp Ser Thr Thr Gly Asn Cys Ala Arg Glu
                155                 160                 165

ggt cat ttt ggt cat gac gat tat ggg tat tgg ttt att att ctt gaa 644
Gly His Phe Gly His Asp Asp Tyr Gly Tyr Trp Phe Ile Ile Leu Glu
            170                 175                 180

gat aaa tta cgt gaa gga gaa gaa cct gat aaa ttg tat ttt caa cag 692
Asp Lys Leu Arg Glu Gly Glu Glu Pro Asp Lys Leu Tyr Phe Gln Gln
        185                 190                 195

tac aat tat gcg gag gac tat gat aaa ggt gac acg ggt att acc gtc 740
Tyr Asn Tyr Ala Glu Asp Tyr Asp Lys Gly Asp Thr Gly Ile Thr Val
    200                 205                 210

gag gaa atc ttt aaa aaa gca aat gat gag tat tgg gaa cct gga gaa 788
Glu Glu Ile Phe Lys Lys Ala Asn Asp Glu Tyr Trp Glu Pro Gly Glu
215                 220                 225                 230

gat cgc ttc att aaa tca cgt tat gag gtg gca gca aag tta tat gag 836
Asp Arg Phe Ile Lys Ser Arg Tyr Glu Val Ala Ala Lys Leu Tyr Glu
                235                 240                 245

gaa atg ttc gga cca aat gga cct caa aca gaa gag gaa cta gaa gca 884
Glu Met Phe Gly Pro Asn Gly Pro Gln Thr Glu Glu Glu Leu Glu Ala
            250                 255                 260

atg cct gat gca gct aca cga tac aaa act tgg aaa gag caa caa aaa 932
Met Pro Asp Ala Ala Thr Arg Tyr Lys Thr Trp Lys Glu Gln Gln Lys
        265                 270                 275

aag gat ccg gca agc aat ttg cca tcg tat gat gtg gta gat agt gga 980
Lys Asp Pro Ala Ser Asn Leu Pro Ser Tyr Asp Val Val Asp Ser Gly
    280                 285                 290

aaa gaa tat gat att tac aat att ata ggt gat cct gaa tcg ttt aag 1028
Lys Glu Tyr Asp Ile Tyr Asn Ile Ile Gly Asp Pro Glu Ser Phe Lys
295                 300                 305                 310

aaa ttt cgt atg aaa cag cct cct att gct tac tgg tta gaa act aaa 1076
Lys Phe Arg Met Lys Gln Pro Pro Ile Ala Tyr Trp Leu Glu Thr Lys
                315                 320                 325

aag gga agg aaa ggc tgg tta cag aaa tat atg cct gct tta cct cat 1124
Lys Gly Arg Lys Gly Trp Leu Gln Lys Tyr Met Pro Ala Leu Pro His
            330                 335                 340

gga agc aaa cac agg gtg tat ttt aac aca cca aat ggg cct ctt gaa 1172
Gly Ser Lys His Arg Val Tyr Phe Asn Thr Pro Asn Gly Pro Leu Glu
        345                 350                 355

cga gtt cct gcg tgg gcc aat ttt gtc att cca gat gca gac ggg atg 1220
Arg Val Pro Ala Trp Ala Asn Phe Val Ile Pro Asp Ala Asp Gly Met
    360                 365                 370

gca tta gca gtc cat tgg gaa cca cct cct gaa tat gct tat aaa tgg 1268
Ala Leu Ala Val His Trp Glu Pro Pro Pro Glu Tyr Ala Tyr Lys Trp
375                 380                 385                 390

aaa cac aag cta cca gtc aag cct aag tcc ttg cgc ata tat gaa tgt 1316
Lys His Lys Leu Pro Val Lys Pro Lys Ser Leu Arg Ile Tyr Glu Cys
                395                 400                 405

cat gtt ggc atc tct ggc cag gaa cca aaa gtt tca tct ttc aat gat 1364
His Val Gly Ile Ser Gly Gln Glu Pro Lys Val Ser Ser Phe Asn Asp
            410                 415                 420

ttt att agc aag gtc ctt ccg cat gta aaa gaa gct gga tac aat gca 1412
Phe Ile Ser Lys Val Leu Pro His Val Lys Glu Ala Gly Tyr Asn Ala
        425                 430                 435

acg caa att att gga gtt gtt gag cac aag gat tat ttc act gtt gga 1460
Thr Gln Ile Ile Gly Val Val Glu His Lys Asp Tyr Phe Thr Val Gly
    440                 445                 450

tat aga gtg acc aat ttt tat gct gtt agt agc cgt tat ggc aca ccg 1508
Tyr Arg Val Thr Asn Phe Tyr Ala Val Ser Ser Arg Tyr Gly Thr Pro
455                 460                 465                 470

gat gac ttc aag cgc ttg gtt gat gaa gca cat ggg ctt gga ctg ctt 1556
Asp Asp Phe Lys Arg Leu Val Asp Glu Ala His Gly Leu Gly Leu Leu
                475                 480                 485

gtc ttt ttg gag att gtg cac tcc tat gca gca gca gat gaa atg gtt 1604
Val Phe Leu Glu Ile Val His Ser Tyr Ala Ala Ala Asp Glu Met Val
            490                 495                 500

ggg tta tct ctt ttt gat gga gca aat gat tgc tat ttc cac act ggt 1652
Gly Leu Ser Leu Phe Asp Gly Ala Asn Asp Cys Tyr Phe His Thr Gly
        505                 510                 515

aaa cgt gga cac cac aaa ttc tgg ggc aca cgg atg ttc aaa tat gga     1700
Lys Arg Gly His His Lys Phe Trp Gly Thr Arg Met Phe Lys Tyr Gly
    520                 525                 530

gat cct gat gtt ctg cac ttt ctt ctt tca aat ctg aac tgg tgg gtg     1748
Asp Pro Asp Val Leu His Phe Leu Leu Ser Asn Leu Asn Trp Trp Val
535                 540                 545                 550

gag gag tat cat gtc gat ggc ttc cat ttt cat tcg ctc tcg tcc atg     1796
Glu Glu Tyr His Val Asp Gly Phe His Phe His Ser Leu Ser Ser Met
                555                 560                 565

ttg tat acg cat aat gga ttt gct tca ttt act ggt gac atg gat gaa     1844
Leu Tyr Thr His Asn Gly Phe Ala Ser Phe Thr Gly Asp Met Asp Glu
            570                 575                 580

tac tgt aac caa tat gtt gac aag gag gcc tta ttg tac ctc ata tta     1892
Tyr Cys Asn Gln Tyr Val Asp Lys Glu Ala Leu Leu Tyr Leu Ile Leu
        585                 590                 595

gca aat gaa gta tta cat gct ctt cat cct aat gtg atc acg att gct     1940
Ala Asn Glu Val Leu His Ala Leu His Pro Asn Val Ile Thr Ile Ala
    600                 605                 610

gtg gat gca act ctg tat cct gga ctc tgc gat cca aca tct caa ggt     1988
Val Asp Ala Thr Leu Tyr Pro Gly Leu Cys Asp Pro Thr Ser Gln Gly
615                 620                 625                 630

gga ctg ggc ttt gat tat ttt gcc aat ctt tct gcc tca gag atg tgg     2036
Gly Leu Gly Phe Asp Tyr Phe Ala Asn Leu Ser Ala Ser Glu Met Trp
                635                 640                 645

ctt gca tta ctt gaa aat act cct gat cat gaa tgg tgc atg agt aag     2084
Leu Ala Leu Leu Glu Asn Thr Pro Asp His Glu Trp Cys Met Ser Lys
            650                 655                 660

att gtt agc aca tta gtg ggc gat aga caa aat act gat aaa atg ctt     2132
Ile Val Ser Thr Leu Val Gly Asp Arg Gln Asn Thr Asp Lys Met Leu
        665                 670                 675

ttg tat gca gaa aat cac aac cag tcc att tct gga ggt cgt tcc ttc     2180
Leu Tyr Ala Glu Asn His Asn Gln Ser Ile Ser Gly Gly Arg Ser Phe
    680                 685                 690

gca gaa ata ctg att ggt aac tcc ttg ggg aaa tct tcc ata tca caa     2228
Ala Glu Ile Leu Ile Gly Asn Ser Leu Gly Lys Ser Ser Ile Ser Gln
695                 700                 705                 710

gag tca tta ctt aga ggc tgc tcg tta cac aag atg atc aga tta att     2276
Glu Ser Leu Leu Arg Gly Cys Ser Leu His Lys Met Ile Arg Leu Ile
                715                 720                 725

aca tct aca att ggt ggt cat gca tac ctc aac ttc atg ggc aat gaa     2324
Thr Ser Thr Ile Gly Gly His Ala Tyr Leu Asn Phe Met Gly Asn Glu
            730                 735                 740

ttt ggt cac cca aag aga gta gag ttt cca atg tca agc aac aat ttc     2372
Phe Gly His Pro Lys Arg Val Glu Phe Pro Met Ser Ser Asn Asn Phe
        745                 750                 755

tcc ttt tca ctg gct aac cgt cgc tgg gat cta ttg gaa gat gtt gta     2420
Ser Phe Ser Leu Ala Asn Arg Arg Trp Asp Leu Leu Glu Asp Val Val
    760                 765                 770

cat tat caa tta ttc tca ttt gat aag gat atg atg gac ttg gat aaa     2468
His Tyr Gln Leu Phe Ser Phe Asp Lys Asp Met Met Asp Leu Asp Lys
775                 780                 785                 790

aat ggg aga att ttg tcc aga ggt ctt gcc aac att cac cat gtc aat     2516
Asn Gly Arg Ile Leu Ser Arg Gly Leu Ala Asn Ile His His Val Asn
                795                 800                 805

gat act acc atg gtg att tct tac ttg aga ggt ccc aat ctc ttt gtg     2564
Asp Thr Thr Met Val Ile Ser Tyr Leu Arg Gly Pro Asn Leu Phe Val
            810                 815                 820

ttc aac ttt cat cct gtc aat tca tat gaa aga tac att ata ggt gtg     2612
Phe Asn Phe His Pro Val Asn Ser Tyr Glu Arg Tyr Ile Ile Gly Val
        825                 830                 835

gaa gaa gct gga gag tat caa gtc aca tta aat aca gat gaa aac aag     2660
Glu Glu Ala Gly Glu Tyr Gln Val Thr Leu Asn Thr Asp Glu Asn Lys
    840                 845                 850

tat ggt ggt aga gga cta ctt ggc cat gat cag aat act caa aga acc     2708
Tyr Gly Gly Arg Gly Leu Leu Gly His Asp Gln Asn Thr Gln Arg Thr
855                 860                 865                 870

att agt aga aga gct gat gga atg aga ttt tgc ttg gaa gta cct ctg     2756
Ile Ser Arg Arg Ala Asp Gly Met Arg Phe Cys Leu Glu Val Pro Leu
                875                 880                 885

cca agt aga agt gct cag gtc tac aag ttg acc cga att cta aga gca     2804
Pro Ser Arg Ser Ala Gln Val Tyr Lys Leu Thr Arg Ile Leu Arg Ala
            890                 895                 900

tgatcactct agcaatcaaa gtgcctcata tgatcacaca aaagggaagg ttctacattg     2864

cccttatact gaccaatatt gtggcctttc cgaggtgagt ttctgtgatt cttgagcaca     2924

ggctgttggc tagtcagtta tcatgaactt ttgccttcag catctggata agcgcttctc     2984

ctgtgcaatg agggcatgga cgaaattttt ttggttcgtc atgggagtca aaagcatctg     3044

ccagattaag atttcacagg cctcgagtaa aaccatcact tacttaggat acacaaacac     3104

atcaacgggg tgcaggctct gataccttct aaagtgaagc cgaattccag cacactggcg     3164

gccgttacta gtggatccga gctcggtacc aagcttggcg                          3204

<210>  12

<211>  902

<212>  PRT

<213>  Solanum tuberosum

<400>  12
   Met Leu Ser Leu Ser Asp Ser Ile Arg Ile Ser Ser Pro Leu Ser Asp
   1               5                   10                  15
   Ser Arg Leu Ser Phe Leu Ser Gln Thr Gly Ser Arg Thr Ser Arg Gln
               20                  25                  30
   Leu Lys Phe Val Arg Ser Arg Arg Ala Arg Val Ser Arg Cys Arg Cys
           35                  40                  45
   Ser Ala Thr Glu Gln Pro Pro Pro Gln Arg Arg Lys Gln Arg Pro Glu
       50                  55                  60
   Lys Tyr Lys Gln Ser Glu Glu Glu Lys Gly Ile Asp Pro Val Gly Phe
   65                  70                  75                  80
   Leu Ser Lys Tyr Gly Ile Thr His Lys Ala Phe Ala Gln Phe Leu Arg
                   85                  90                  95
   Glu Arg Tyr Lys Ser Leu Lys Asp Leu Lys Asp Glu Ile Leu Thr Arg
               100                 105                 110
   His Phe Ser Leu Lys Glu Met Ser Thr Gly Tyr Glu Leu Met Gly Met
           115                 120                 125
   His Arg Asn Ile Gln His Arg Val Asp Phe Leu Glu Trp Ala Pro Gly
       130                 135                 140
   Ala Arg Tyr Cys Ala Leu Ile Gly Asp Phe Asn Gly Trp Ser Thr Thr
   145                 150                 155                 160
   Gly Asn Cys Ala Arg Glu Gly His Phe Gly His Asp Asp Tyr Gly Tyr
                   165                 170                 175
   Trp Phe Ile Ile Leu Glu Asp Lys Leu Arg Glu Gly Glu Glu Pro Asp
               180                 185                 190
   Lys Leu Tyr Phe Gln Gln Tyr Asn Tyr Ala Glu Asp Tyr Asp Lys Gly
           195                 200                 205
   Asp Thr Gly Ile Thr Val Glu Glu Ile Phe Lys Lys Ala Asn Asp Glu
       210                 215                 220
   Tyr Trp Glu Pro Gly Glu Asp Arg Phe Ile Lys Ser Arg Tyr Glu Val
   225                 230                 235                 240
   Ala Ala Lys Leu Tyr Glu Glu Met Phe Gly Pro Asn Gly Pro Gln Thr
                   245                 250                 255
   Glu Glu Glu Leu Glu Ala Met Pro Asp Ala Ala Thr Arg Tyr Lys Thr
               260                 265                 270
   Trp Lys Glu Gln Gln Lys Lys Asp Pro Ala Ser Asn Leu Pro Ser Tyr
           275                 280                 285
   Asp Val Val Asp Ser Gly Lys Glu Tyr Asp Ile Tyr Asn Ile Ile Gly
       290                 295                 300
   Asp Pro Glu Ser Phe Lys Lys Phe Arg Met Lys Gln Pro Pro Ile Ala
   305                 310                 315                 320
   Tyr Trp Leu Glu Thr Lys Lys Gly Arg Lys Gly Trp Leu Gln Lys Tyr
                   325                 330                 335
   Met Pro Ala Leu Pro His Gly Ser Lys His Arg Val Tyr Phe Asn Thr
               340                 345                 350
   Pro Asn Gly Pro Leu Glu Arg Val Pro Ala Trp Ala Asn Phe Val Ile
           355                 360                 365
   Pro Asp Ala Asp Gly Met Ala Leu Ala Val His Trp Glu Pro Pro Pro
       370                 375                 380
   Glu Tyr Ala Tyr Lys Trp Lys His Lys Leu Pro Val Lys Pro Lys Ser
   385                 390                 395                 400
   Leu Arg Ile Tyr Glu Cys His Val Gly Ile Ser Gly Gln Glu Pro Lys
                   405                 410                 415
   Val Ser Ser Phe Asn Asp Phe Ile Ser Lys Val Leu Pro His Val Lys
               420                 425                 430
   Glu Ala Gly Tyr Asn Ala Thr Gln Ile Ile Gly Val Val Glu His Lys
           435                 440                 445
   Asp Tyr Phe Thr Val Gly Tyr Arg Val Thr Asn Phe Tyr Ala Val Ser
       450                 455                 460
   Ser Arg Tyr Gly Thr Pro Asp Asp Phe Lys Arg Leu Val Asp Glu Ala
   465                 470                 475                 480
   His Gly Leu Gly Leu Leu Val Phe Leu Glu Ile Val His Ser Tyr Ala
                   485                 490                 495
   Ala Ala Asp Glu Met Val Gly Leu Ser Leu Phe Asp Gly Ala Asn Asp
               500                 505                 510
   Cys Tyr Phe His Thr Gly Lys Arg Gly His His Lys Phe Trp Gly Thr
           515                 520                 525
   Arg Met Phe Lys Tyr Gly Asp Pro Asp Val Leu His Phe Leu Leu Ser
       530                 535                 540
   Asn Leu Asn Trp Trp Val Glu Glu Tyr His Val Asp Gly Phe His Phe
   545                 550                 555                 560
   His Ser Leu Ser Ser Met Leu Tyr Thr His Asn Gly Phe Ala Ser Phe
                   565                 570                 575
   Thr Gly Asp Met Asp Glu Tyr Cys Asn Gln Tyr Val Asp Lys Glu Ala
               580                 585                 590
   Leu Leu Tyr Leu Ile Leu Ala Asn Glu Val Leu His Ala Leu His Pro
           595                 600                 605
   Asn Val Ile Thr Ile Ala Val Asp Ala Thr Leu Tyr Pro Gly Leu Cys
       610                 615                 620
   Asp Pro Thr Ser Gln Gly Gly Leu Gly Phe Asp Tyr Phe Ala Asn Leu
   625                 630                 635                 640
   Ser Ala Ser Glu Met Trp Leu Ala Leu Leu Glu Asn Thr Pro Asp His
                   645                 650                 655
   Glu Trp Cys Met Ser Lys Ile Val Ser Thr Leu Val Gly Asp Arg Gln
               660                 665                 670
   Asn Thr Asp Lys Met Leu Leu Tyr Ala Glu Asn His Asn Gln Ser Ile
           675                 680                 685
   Ser Gly Gly Arg Ser Phe Ala Glu Ile Leu Ile Gly Asn Ser Leu Gly
       690                 695                 700
   Lys Ser Ser Ile Ser Gln Glu Ser Leu Leu Arg Gly Cys Ser Leu His
   705                 710                 715                 720
   Lys Met Ile Arg Leu Ile Thr Ser Thr Ile Gly Gly His Ala Tyr Leu
                   725                 730                 735
   Asn Phe Met Gly Asn Glu Phe Gly His Pro Lys Arg Val Glu Phe Pro
               740                 745                 750
   Met Ser Ser Asn Asn Phe Ser Phe Ser Leu Ala Asn Arg Arg Trp Asp
           755                 760                 765
   Leu Leu Glu Asp Val Val His Tyr Gln Leu Phe Ser Phe Asp Lys Asp
       770                 775                 780
   Met Met Asp Leu Asp Lys Asn Gly Arg Ile Leu Ser Arg Gly Leu Ala
   785                 790                 795                 800
   Asn Ile His His Val Asn Asp Thr Thr Met Val Ile Ser Tyr Leu Arg
                   805                 810                 815
   Gly Pro Asn Leu Phe Val Phe Asn Phe His Pro Val Asn Ser Tyr Glu
               820                 825                 830
   Arg Tyr Ile Ile Gly Val Glu Glu Ala Gly Glu Tyr Gln Val Thr Leu
           835                 840                 845
   Asn Thr Asp Glu Asn Lys Tyr Gly Gly Arg Gly Leu Leu Gly His Asp
       850                 855                 860
   Gln Asn Thr Gln Arg Thr Ile Ser Arg Arg Ala Asp Gly Met Arg Phe
   865                 870                 875                 880
   Cys Leu Glu Val Pro Leu Pro Ser Arg Ser Ala Gln Val Tyr Lys Leu
                   885                 890                 895
   Thr Arg Ile Leu Arg Ala
               900

<210>  13

<211>  3047

<212>  DNA

<213>  Solanum tuberosum

<220>

<221>  CDS

<222>  (5)..(2710)

<223>

<400>  13
aaca atg ctc tct ctg tcg gat tca att cga att tct tca cca ttg agc      49
     Met Leu Ser Leu Ser Asp Ser Ile Arg Ile Ser Ser Pro Leu Ser
     1               5                   10                  15

gat tct cgt ctt agt ttt cta tct caa acc gga agc aga acc agt cgc      97
Asp Ser Arg Leu Ser Phe Leu Ser Gln Thr Gly Ser Arg Thr Ser Arg
                20                  25                  30

cag ctt aaa ttt gtt cgc agc cgc cgg gct cga gtt tcg agg tgt aga      145
Gln Leu Lys Phe Val Arg Ser Arg Arg Ala Arg Val Ser Arg Cys Arg
            35                  40                  45

tgc tca gca acg gag caa ccg cca ccg caa cga cgg aag caa cga ccg      193
Cys Ser Ala Thr Glu Gln Pro Pro Pro Gln Arg Arg Lys Gln Arg Pro
        50                  55                  60

gag aag tac aaa cag tcg gag gaa ggg aaa gga atc gat cct gtt gga      241
Glu Lys Tyr Lys Gln Ser Glu Glu Gly Lys Gly Ile Asp Pro Val Gly
    65                  70                  75

ttt ctc agc aaa tac ggc att act cat aaa gcg ttt gct caa ttt ctt      289
Phe Leu Ser Lys Tyr Gly Ile Thr His Lys Ala Phe Ala Gln Phe Leu
80                  85                  90                  95

cgt gaa aga tat aaa tca ttg aag gac ttg aag gat gaa ata ttg act      337
Arg Glu Arg Tyr Lys Ser Leu Lys Asp Leu Lys Asp Glu Ile Leu Thr
                100                 105                 110

cgt cat ttc agt ctc aag gag atg tct act ggg tat gaa tta atg ggt      385
Arg His Phe Ser Leu Lys Glu Met Ser Thr Gly Tyr Glu Leu Met Gly
            115                 120                 125

atg cat cgc aac ata caa cat cga gtg gat ttc ttg gaa tgg gct cca      433
Met His Arg Asn Ile Gln His Arg Val Asp Phe Leu Glu Trp Ala Pro
        130                 135                 140

ggt gct cgc tac tgt gct ctg att ggt gac ttc aat ggg tgg tca aca 481
Gly Ala Arg Tyr Cys Ala Leu Ile Gly Asp Phe Asn Gly Trp Ser Thr
    145                 150                 155

act ggt aac tgt gcc aga gag ggt cat ttt ggt cat gac gat tat ggg 529
Thr Gly Asn Cys Ala Arg Glu Gly His Phe Gly His Asp Asp Tyr Gly
160                 165                 170                 175

tat tgg ttt att att ctt gaa gat aaa tta cgt gaa gga gaa gaa cct 577
Tyr Trp Phe Ile Ile Leu Glu Asp Lys Leu Arg Glu Gly Glu Glu Pro
                180                 185                 190

gat aaa ttg tat ttt caa cag tac aat tat gcg gag gac tat ggt aaa 625
Asp Lys Leu Tyr Phe Gln Gln Tyr Asn Tyr Ala Glu Asp Tyr Gly Lys
            195                 200                 205

ggt gac acg ggt att acc gtc gag gaa atc ttt aaa aaa gca aat gat 673
Gly Asp Thr Gly Ile Thr Val Glu Glu Ile Phe Lys Lys Ala Asn Asp
        210                 215                 220

gag tat tgg gaa cct gga gaa gat cgc ttc att aaa tca cgt tat gag 721
Glu Tyr Trp Glu Pro Gly Glu Asp Arg Phe Ile Lys Ser Arg Tyr Glu
    225                 230                 235

gtg gca gca aag tta tat gag gaa atg ttc gga cca aat gga cct caa 769
Val Ala Ala Lys Leu Tyr Glu Glu Met Phe Gly Pro Asn Gly Pro Gln
240                 245                 250                 255

aca gaa gag gaa cta gaa gca atg cct gat gca gct aca cga tac aaa 817
Thr Glu Glu Glu Leu Glu Ala Met Pro Asp Ala Ala Thr Arg Tyr Lys
                260                 265                 270

act tgg aaa gag caa caa aaa gag gat ccg gca agc aat ttg cca tcg 865
Thr Trp Lys Glu Gln Gln Lys Glu Asp Pro Ala Ser Asn Leu Pro Ser
            275                 280                 285

tat gat gtg gta gat agt gga aaa gaa tat gat att tac aat att ata 913
Tyr Asp Val Val Asp Ser Gly Lys Glu Tyr Asp Ile Tyr Asn Ile Ile
        290                 295                 300

ggt gat cct gaa tcg ttt aag aaa ttt cgt atg aaa cag cct cct att 961
Gly Asp Pro Glu Ser Phe Lys Lys Phe Arg Met Lys Gln Pro Pro Ile
    305                 310                 315

gct tac tgg tta gaa act aaa aag gga agg aaa ggc tgg tta cag aaa 1009
Ala Tyr Trp Leu Glu Thr Lys Lys Gly Arg Lys Gly Trp Leu Gln Lys
320                 325                 330                 335

tat atg cct gct tta cct cat gga agc aaa tac agg gtg tat ttt aac 1057
Tyr Met Pro Ala Leu Pro His Gly Ser Lys Tyr Arg Val Tyr Phe Asn
                340                 345                 350

aca cca aat ggg cct ctt gaa cga gtt cct gcg tgg gcc aat ttt gtc 1105
Thr Pro Asn Gly Pro Leu Glu Arg Val Pro Ala Trp Ala Asn Phe Val
            355                 360                 365

att cca gat gca ggc ggg atg gca tta gca gtc cat tgg gaa cca cct 1153
Ile Pro Asp Ala Gly Gly Met Ala Leu Ala Val His Trp Glu Pro Pro
        370                 375                 380

cct gaa tat gct tat aaa tgg aaa cac aag cta cca gtc aag cct aag 1201
Pro Glu Tyr Ala Tyr Lys Trp Lys His Lys Leu Pro Val Lys Pro Lys
    385                 390                 395

tcc ttg cgc ata tat gaa tgt cat gtt ggc atc tct ggc cag gaa cca 1249
Ser Leu Arg Ile Tyr Glu Cys His Val Gly Ile Ser Gly Gln Glu Pro
400                 405                 410                 415

aaa gtt tca tct ttc aat gat ttt att agc aag gtc ctt ccg cat gta 1297
Lys Val Ser Ser Phe Asn Asp Phe Ile Ser Lys Val Leu Pro His Val
                420                 425                 430

aaa gaa gct gga tac aat gca ata caa att att gga gtt gtt gag cac 1345
Lys Glu Ala Gly Tyr Asn Ala Ile Gln Ile Ile Gly Val Val Glu His
            435                 440                 445

aag gat tat ttc act gtt gga tat aga gtg acc aat ttt tat gct gtt 1393
Lys Asp Tyr Phe Thr Val Gly Tyr Arg Val Thr Asn Phe Tyr Ala Val
        450                 455                 460

agt agc cgt tat ggc aca ccg gat gac ttc aag cgc ttg gtt gat gaa 1441
Ser Ser Arg Tyr Gly Thr Pro Asp Asp Phe Lys Arg Leu Val Asp Glu
    465                 470                 475

gca cat ggg ctt gga ctg ctt gtc ttt ttg gag att gtg cac tct tat 1489
Ala His Gly Leu Gly Leu Leu Val Phe Leu Glu Ile Val His Ser Tyr
480                 485                 490                 495

gca gca gca gat gaa atg gtt ggg tta tct ctt ttt gat gga gca aat 1537
Ala Ala Ala Asp Glu Met Val Gly Leu Ser Leu Phe Asp Gly Ala Asn
                500                 505                 510

gat tgc tat ttc cac act ggt aaa cgt gga cac cac aaa ttc tgg ggc 1585
Asp Cys Tyr Phe His Thr Gly Lys Arg Gly His His Lys Phe Trp Gly
            515                 520                 525

aca cgg atg ttc aaa tat gga gat ctt gat gtt ctg cac ttt ctt ctt 1633
Thr Arg Met Phe Lys Tyr Gly Asp Leu Asp Val Leu His Phe Leu Leu
        530                 535                 540

tca aat ctg aac tgg tgg gtg gag gag tat cat gtc gat ggc ttc cat 1681
Ser Asn Leu Asn Trp Trp Val Glu Glu Tyr His Val Asp Gly Phe His
    545                 550                 555

ttt cat tcg ctc tcg tcc atg ttg tat acg cat aat gga ttt gct tca 1729
Phe His Ser Leu Ser Ser Met Leu Tyr Thr His Asn Gly Phe Ala Ser
560                 565                 570                 575

ttt act ggt gac atg gat gaa tac tgt aac caa tat gtt gac aag gag 1777
Phe Thr Gly Asp Met Asp Glu Tyr Cys Asn Gln Tyr Val Asp Lys Glu
                580                 585                 590

gcc tta ttg tac ctc ata tta gca aat gaa gta tta cat gct ctt cat 1825
Ala Leu Leu Tyr Leu Ile Leu Ala Asn Glu Val Leu His Ala Leu His
            595                 600                 605

cct aat gtg atc acg att gct gag gat gca act ctg tat cct gga ctc 1873
Pro Asn Val Ile Thr Ile Ala Glu Asp Ala Thr Leu Tyr Pro Gly Leu
        610                 615                 620

tgc gat cca aca tct caa ggt gga ctg ggc ttt gat tat ttt gcc aat 1921
Cys Asp Pro Thr Ser Gln Gly Gly Leu Gly Phe Asp Tyr Phe Ala Asn
    625                 630                 635

ctt tct gcc tca gag atg tgg ctt gca tta ctt gaa aat act cct gat 1969
Leu Ser Ala Ser Glu Met Trp Leu Ala Leu Leu Glu Asn Thr Pro Asp
640                 645                 650                 655

cat gaa tgg tgc atg agt aag att gtt agc aca tta gtg ggc gat aga 2017
His Glu Trp Cys Met Ser Lys Ile Val Ser Thr Leu Val Gly Asp Arg
                660                 665                 670

caa aat act gat aaa atg ctt ttg tat gca gaa aat cac aac cag tcc 2065
Gln Asn Thr Asp Lys Met Leu Leu Tyr Ala Glu Asn His Asn Gln Ser
            675                 680                 685

att tct gga ggt cgt tcc ttc gca gaa ata ctg att ggt aac tcc ttg 2113
Ile Ser Gly Gly Arg Ser Phe Ala Glu Ile Leu Ile Gly Asn Ser Leu
        690                 695                 700

ggg aaa tcc tcc ata tca caa gag tca tta ctt aga ggc tgc tcg tta 2161
Gly Lys Ser Ser Ile Ser Gln Glu Ser Leu Leu Arg Gly Cys Ser Leu
    705                 710                 715

cac aag atg atc aga tta att aca tct aca att ggt ggt cat gca tac 2209
His Lys Met Ile Arg Leu Ile Thr Ser Thr Ile Gly Gly His Ala Tyr
720                 725                 730                 735

ctc aac ttc atg ggc aat gaa ttt ggt cac cca aag aga gta gag ttt 2257
Leu Asn Phe Met Gly Asn Glu Phe Gly His Pro Lys Arg Val Glu Phe
                740                 745                 750

cca atg tca agc aac aat ttc tcc ttt tca ctg gct aac cgt cgc tgg 2305
Pro Met Ser Ser Asn Asn Phe Ser Phe Ser Leu Ala Asn Arg Arg Trp
            755                 760                 765

gat cta ttg gaa gat gtt gta cat tat caa ttg ttc tca ttt gat aag 2353
Asp Leu Leu Glu Asp Val Val His Tyr Gln Leu Phe Ser Phe Asp Lys
        770                 775                 780

ggt atg atg gac ttg gat aaa aat ggg aga att ttg tcc aga ggt ctt 2401
Gly Met Met Asp Leu Asp Lys Asn Gly Arg Ile Leu Ser Arg Gly Leu
    785                 790                 795

gcc aac att cac cat gtc aat gat act acc atg gtg att tct tac ttg 2449
Ala Asn Ile His His Val Asn Asp Thr Thr Met Val Ile Ser Tyr Leu
800                 805                 810                 815

aga ggt ccc aat ctc ttt gtg ttc aac ttt cat cct gtc aat tca tat 2497
Arg Gly Pro Asn Leu Phe Val Phe Asn Phe His Pro Val Asn Ser Tyr
                820                 825                 830

gaa aga tac att ata ggt gtg gaa gaa gct gga gag tat caa gtc aca 2545
Glu Arg Tyr Ile Ile Gly Val Glu Glu Ala Gly Glu Tyr Gln Val Thr
            835                 840                 845

tta aat aca gat gaa aac aag tat ggt ggt aga gga cta ctt ggc cat 2593
Leu Asn Thr Asp Glu Asn Lys Tyr Gly Gly Arg Gly Leu Leu Gly His
        850                 855                 860

gat cag aat att caa aga acc att agt aga aga gct gat gga atg aga 2641
Asp Gln Asn Ile Gln Arg Thr Ile Ser Arg Arg Ala Asp Gly Met Arg
    865                 870                 875

ttt tgc ttg gaa gtg cct ctg cca agt aga agt gct cag gtc tac aag 2689
Phe Cys Leu Glu Val Pro Leu Pro Ser Arg Ser Ala Gln Val Tyr Lys
880                 885                 890                 895

ttg acc cga att cta aga gca tgatcactct agtaatcaaa gtgcctcata 2740
Leu Thr Arg Ile Leu Arg Ala
                900

tgatgacaca aaaggaaagg ttctacattg cccttacact gatcaatatt gacacctttc 2800

cgaggtgagt ttctgtgatt cttgagcaga ctgttggcta gtcaattatc atgaactttt 2860

gccttcagca tccggatagt cgcttctcct gtgcaatgag ggcatggacg aatttttttt 2920

tggcttgtca tgggggtcat aagcatccgc cagattaaga tttcacaggc ctcgagtaaa 2980

accatcactt actttaagga tacacaaaca caccaacggg gtgcaggctc tgataccttc 3040

taaagtg 3047

<210>  14

<211>  902

<212>  PRT

<213>  Solanum tuberosum


<400>  14

Met Leu Ser Leu Ser Asp Ser Ile Arg Ile Ser Ser Pro Leu Ser Asp
1               5                   10                  15

Ser Arg Leu Ser Phe Leu Ser Gln Thr Gly Ser Arg Thr Ser Arg Gln
            20                  25                  30

Leu Lys Phe Val Arg Ser Arg Arg Ala Arg Val Ser Arg Cys Arg Cys
        35                  40                  45

Ser Ala Thr Glu Gln Pro Pro Pro Gln Arg Arg Lys Gln Arg Pro Glu
    50                  55                  60

Lys Tyr Lys Gln Ser Glu Glu Gly Lys Gly Ile Asp Pro Val Gly Phe
65                  70                  75                  80

Leu Ser Lys Tyr Gly Ile Thr His Lys Ala Phe Ala Gln Phe Leu Arg
                85                  90                  95

Glu Arg Tyr Lys Ser Leu Lys Asp Leu Lys Asp Glu Ile Leu Thr Arg
            100                 105                 110

His Phe Ser Leu Lys Glu Met Ser Thr Gly Tyr Glu Leu Met Gly Met
        115                 120                 125

His Arg Asn Ile Gln His Arg Val Asp Phe Leu Glu Trp Ala Pro Gly
    130                 135                 140

Ala Arg Tyr Cys Ala Leu Ile Gly Asp Phe Asn Gly Trp Ser Thr Thr
145                 150                 155                 160

Gly Asn Cys Ala Arg Glu Gly His Phe Gly His Asp Asp Tyr Gly Tyr
                165                 170                 175

Trp Phe Ile Ile Leu Glu Asp Lys Leu Arg Glu Gly Glu Glu Pro Asp
            180                 185                 190

Lys Leu Tyr Phe Gln Gln Tyr Asn Tyr Ala Glu Asp Tyr Gly Lys Gly
        195                 200                 205

Asp Thr Gly Ile Thr Val Glu Glu Ile Phe Lys Lys Ala Asn Asp Glu
    210                 215                 220

Tyr Trp Glu Pro Gly Glu Asp Arg Phe Ile Lys Ser Arg Tyr Glu Val
225                 230                 235                 240

Ala Ala Lys Leu Tyr Glu Glu Met Phe Gly Pro Asn Gly Pro Gln Thr
                245                 250                 255

Glu Glu Glu Leu Glu Ala Met Pro Asp Ala Ala Thr Arg Tyr Lys Thr
            260                 265                 270

Trp Lys Glu Gln Gln Lys Glu Asp Pro Ala Ser Asn Leu Pro Ser Tyr
        275                 280                 285

Asp Val Val Asp Ser Gly Lys Glu Tyr Asp Ile Tyr Asn Ile Ile Gly
    290                 295                 300

Asp Pro Glu Ser Phe Lys Lys Phe Arg Met Lys Gln Pro Pro Ile Ala
305                 310                 315                 320

Tyr Trp Leu Glu Thr Lys Lys Gly Arg Lys Gly Trp Leu Gln Lys Tyr
                325                 330                 335

Met Pro Ala Leu Pro His Gly Ser Lys Tyr Arg Val Tyr Phe Asn Thr
            340                 345                 350

Pro Asn Gly Pro Leu Glu Arg Val Pro Ala Trp Ala Asn Phe Val Ile
        355                 360                 365

Pro Asp Ala Gly Gly Met Ala Leu Ala Val His Trp Glu Pro Pro Pro
    370                 375                 380

Glu Tyr Ala Tyr Lys Trp Lys His Lys Leu Pro Val Lys Pro Lys Ser
385                 390                 395                 400

Leu Arg Ile Tyr Glu Cys His Val Gly Ile Ser Gly Gln Glu Pro Lys
                405                 410                 415

Val Ser Ser Phe Asn Asp Phe Ile Ser Lys Val Leu Pro His Val Lys
            420                 425                 430

Glu Ala Gly Tyr Asn Ala Ile Gln Ile Ile Gly Val Val Glu His Lys
        435                 440                 445

Asp Tyr Phe Thr Val Gly Tyr Arg Val Thr Asn Phe Tyr Ala Val Ser
    450                 455                 460

Ser Arg Tyr Gly Thr Pro Asp Asp Phe Lys Arg Leu Val Asp Glu Ala
465                 470                 475                 480

His Gly Leu Gly Leu Leu Val Phe Leu Glu Ile Val His Ser Tyr Ala
                485                 490                 495

Ala Ala Asp Glu Met Val Gly Leu Ser Leu Phe Asp Gly Ala Asn Asp
            500                 505                 510

Cys Tyr Phe His Thr Gly Lys Arg Gly His His Lys Phe Trp Gly Thr
        515                 520                 525

Arg Met Phe Lys Tyr Gly Asp Leu Asp Val Leu His Phe Leu Leu Ser
    530                 535                 540

Asn Leu Asn Trp Trp Val Glu Glu Tyr His Val Asp Gly Phe His Phe
545                 550                 555                 560

His Ser Leu Ser Ser Met Leu Tyr Thr His Asn Gly Phe Ala Ser Phe
                565                 570                 575

Thr Gly Asp Met Asp Glu Tyr Cys Asn Gln Tyr Val Asp Lys Glu Ala
            580                 585                 590

Leu Leu Tyr Leu Ile Leu Ala Asn Glu Val Leu His Ala Leu His Pro
        595                 600                 605

Asn Val Ile Thr Ile Ala Glu Asp Ala Thr Leu Tyr Pro Gly Leu Cys
    610                 615                 620

Asp Pro Thr Ser Gln Gly Gly Leu Gly Phe Asp Tyr Phe Ala Asn Leu
625                 630                 635                 640

Ser Ala Ser Glu Met Trp Leu Ala Leu Leu Glu Asn Thr Pro Asp His
                645                 650                 655

Glu Trp Cys Met Ser Lys Ile Val Ser Thr Leu Val Gly Asp Arg Gln
            660                 665                 670

Asn Thr Asp Lys Met Leu Leu Tyr Ala Glu Asn His Asn Gln Ser Ile
        675                 680                 685

Ser Gly Gly Arg Ser Phe Ala Glu Ile Leu Ile Gly Asn Ser Leu Gly
    690                 695                 700

Lys Ser Ser Ile Ser Gln Glu Ser Leu Leu Arg Gly Cys Ser Leu His
705                 710                 715                 720

Lys Met Ile Arg Leu Ile Thr Ser Thr Ile Gly Gly His Ala Tyr Leu
                725                 730                 735

Asn Phe Met Gly Asn Glu Phe Gly His Pro Lys Arg Val Glu Phe Pro
            740                 745                 750

Met Ser Ser Asn Asn Phe Ser Phe Ser Leu Ala Asn Arg Arg Trp Asp
        755                 760                 765

Leu Leu Glu Asp Val Val His Tyr Gln Leu Phe Ser Phe Asp Lys Gly
    770                 775                 780

Met Met Asp Leu Asp Lys Asn Gly Arg Ile Leu Ser Arg Gly Leu Ala
785                 790                 795                 800

Asn Ile His His Val Asn Asp Thr Thr Met Val Ile Ser Tyr Leu Arg
                805                 810                 815

Gly Pro Asn Leu Phe Val Phe Asn Phe His Pro Val Asn Ser Tyr Glu
            820                 825                 830

Arg Tyr Ile Ile Gly Val Glu Glu Ala Gly Glu Tyr Gln Val Thr Leu
        835                 840                 845

Asn Thr Asp Glu Asn Lys Tyr Gly Gly Arg Gly Leu Leu Gly His Asp
    850                 855                 860

Gln Asn Ile Gln Arg Thr Ile Ser Arg Arg Ala Asp Gly Met Arg Phe
865                 870                 875                 880

Cys Leu Glu Val Pro Leu Pro Ser Arg Ser Ala Gln Val Tyr Lys Leu
                885                 890                 895

Thr Arg Ile Leu Arg Ala
            900

## Beschreibung

Die Erfindung betrifft modifizierte Stärken mit einem erhöhten Gehalt an Phosphat und einem erhöhten Gehalt an Amylose.

Im Hinblick auf die zunehmende Bedeutung, die pflanzlichen Inhaltsstoffen als erneuerbaren Rohstoffquellen zur Zeit beigemessen wird, ist es eine der Aufgaben der biotechnologischen Forschung, sich um eine Anpassung dieser pflanzlichen Rohstoffe an die Anforderungen der verarbeitenden Industrie zu bemühen. Um eine Anwendung von nachwachsenden Rohstoffen in möglichst vielen Einsatzgebieten zu ermöglichen, ist es darüber hinaus erforderlich, eine große Stoffvielfalt zu erreichen.

Das Polysaccharid Stärke ist aus chemisch einheitlichen Grundbausteinen, den Glucosemolekülen, aufgebaut, stellt jedoch ein komplexes Gemisch unterschiedlicher Molekülformen dar, die Unterschiede hinsichtlich des Polymerisations- und des Verzweigungsgrades aufweisen und sich somit in ihren physikalisch-chemischen Eigenschaften stark voneinander unterscheiden. Man differenziert zwischen Amylosestärke, einem im Wesentlichen unverzweigten Polymer aus alpha-1,4-glycosidisch verknüpften Glucoseeinheiten, und der Amylopektinstärke, einem verzweigten Polymer, bei dem die Verzweigungen durch das Auftreten zusätzlicher alpha-1,6-glycosidischer Verknüpfungen zustande kommen. Ein weiterer wesentlicher Unterschied zwischen Amylose und Amylopektin liegt im Molekulargewicht. Während Amylose, je nach Herkunft der Stärke, ein Molekulargewicht von 5x10⁵ - 10⁶ Da besitzt, liegt das Molekulargewicht des Amylopektins zwischen 10⁷ und 10⁸ Da. Die beiden Makromoleküle können durch ihr Molekulargewicht und ihre unterschiedlichen physiko-chemischen Eigenschaften differenziert werden, was am einfachsten durch ihre unterschiedlichen Jodbindungseigenschaften sichtbar gemacht werden kann.

Amylose wurde lange als lineares Polymer, bestehend aus alpha-1,4-glycosidisch verknüpften alpha-D-Glucose-Monomeren, angesehen. In neueren Studien wurde jedoch die Anwesenheit von alpha-1,6-glycosidischen Verzweigungspunkten (ca. 0,1%) nachgewiesen (Hizukuri und Takagi, Carbohydr. Res. 134, (1984), 1-10; Takeda et al., Carbohydr. Res. 132, (1984), 83-92).

Es stehen unterschiedliche Methoden zur Bestimmung des Amylosegehaltes zur Verfügung. Einige dieser Methoden basieren auf dem lodbindevermögen der Amylose, das potentiometrisch (Banks & Greenwood, in W. Banks & C.T. Greenwood, Starch and its components (pp. 51-66), Edinburgh, Edinburgh University Press), amperometrisch (Larson et al., Analytical Chemistry 25(5), (1953), 802-804) oder spektrophotometrisch (Morrison & Laignelet, J. Cereal Sc. 1, (1983), 9-20) bestimmt werden kann. Die Bestimmung des Amylosegehaltes kann auch kalorimetrisch mittels DSC-(Differential Scanning Calorimetry)-Messungen erfolgen (Kugimiya & Donovan, Journal of Food Science 46, (1981), 765-770; Sievert & Holm, Starch/Stärke 45 (4), (1993), 136-139). Ferner besteht die Möglichkeit, den Amylosegehalt über den Einsatz von SEC-(size exclusion chromatography)-Chromatographie von nativer oder entzweigter Stärke zu bestimmen. Diese Methode wurde insbesondere zur Bestimmung des Amylosegehaltes gentechnisch modifizierter Stärken empfohlen (Gérard et al., Carbohydrate Polymers 44, (2001), 19-27).

Die funktionellen Eigenschaften, wie z.B. die Löslichkeit, das Retrogradationsverhalten, das Wasserbindevermögen, die Filmbildungseigenschaften, die Viskosität, die Verkleisterungseigenschaften, die Gefrier-Tau-Stabilität, die Säurestabilität, die Gelfestigkeit, die Stärkekorngröße von Stärken werden u.a. durch das Amylose/Amylopektin-Verhältnis, das Molekulargewicht, das Muster der Seitenkettenverteilung, den Gehalt an lonen, den Lipid- und Proteingehalt, die mittlere Stärkekorngröße die Stärkekornmorphologie etc. beeinflusst. Die funktionellen Eigenschaften von Stärke werden auch vom Phosphatgehalt, einer nicht-Kohlenstoffkomponente von Stärke, beeinflusst. Dabei ist zwischen Phosphat, welches in Form von Monoestern kovalent an die Glucosemoleküle der Stärke gebundenen ist (im Folgenden als Stärkephosphat bezeichnet) und Phosphat in Form von mit der Stärke assoziierten Phospholipiden zu unterscheiden.

Der Gehalt an Stärkephosphat variiert je nach Pflanzensorte. So synthetisieren z.B. bestimmte Maismutanten eine Stärke mit erhöhtem Gehalt an Stärkephosphat (waxy-Mais 0,002% und Hoch-Amylose-Mais 0,013%), während herkömmliche Mais Sorten nur Spuren von Stärkephosphat aufweisen. Ebenfalls geringe Mengen an Stärkephosphat findet man in Weizen (0,001%) während in Hafer und Sorghum kein Stärkephosphat nachgewiesen werden konnte. In Reis-Mutanten wurde ebenfalls weniger Stärkephosphat gefunden (waxy-Reis 0,003%), als in herkömmlichen Reissorten (0,013%). Signifikante Mengen von Stärkephosphat wurden in Knollen- oder Wurzelspeichestärke synthetisierenden Pflanzen wie z.B. Tapioca (0,008%), Süßkartoffel (0,011%), Pfeilwurz (0,021%) oder Kartoffel (0,089%) nachgewiesen. Die im Vorangegangenen zitierten prozentualen Werte für den Stärkephosphatgehalt beziehen sich jeweils auf das Trockengewicht der Stärke und sind von Jane et al. (1996, Cereal Foods World 41 (11), 827-832) ermittelt worden. Die Phosphatverteilung des Phosphates in von Pflanzen synthetisierter (nativer) Stärke zeichnet sich im Allgemeinen dadurch aus, dass etwa 30% bis 40% der Phosphatreste in C-3-Position und etwa 60% bis 70% der Phosphatreste in C-6-Position der Glucosemoleküle kovalent gebunden sind (Blennow et al., 2000, Int. J. of Biological Macromolecules 27, 211-218). Im Unterschied dazu weisen chemisch phosphorylierte Stärken zusätzlich Phosphatreste in C-2-Position der Glucosemoleküle kovalent gebunden auf, da die chemische Reaktion ungerichtet verläuft.

Eine Übersicht zu nativen Stärken, isoliert aus verschiedenen Pflanzenspezies, die eine Reduktion von an der Stärkebiosynthese beteilligten Enzymen aufweisen, findet sich bei Kossmann und Lloyd (2000, Critical Reviews in Plant Sciences 19(3), 171-126).
Bisher sind Pflanzen beschrieben, bei welchen die Aktivität eines SSIII Proteins (Abel et al., 1996, The Plant Journal 10(6), 9891-991; Lloyd et al., 1999, Biochemical Journal 338, 515-521) bzw. die Aktivität eines BEI Proteins (Kossmann et al. 1991,Mol Gen Genet 230, 39-44); Safford et al., 1998, Carbohydrate Polymers 35, 155-168, bzw. die Aktivität eines BEII Proteins (Jobling et al., 1999, The Plant Journal 18, bzw. die Aktivität eines BEI und BEII Proteins (Schwall et al., 2000, Nature Biotechnology 18, 551- 554; WO 96/34968, Hofvander et al., 2004, Plant Biotechnology 2, 311-321), bzw. die Aktivität eines BEI und eines SSIII (WO 00/08184) Proteins reduziert sind .

Stärken, isoliert aus Pflanzen, bei welchen die Aktivität eines SSIII Proteins reduziert ist, zeigen, verglichen mit entsprechenden Wildtyp-Pflanzen, eine relative Verschiebung der Seitenketten des Amylopektins von längeren Ketten zu kurzen Ketten (Lloyd et al., 1999, Biochemical Journal 338, 515-521), einen um 70% erhöhten Phosphatgehalt, keine Veränderung des Amylosegehaltes (Abel et al., 1996, The Plant Journal 10(6), 9891-991) und eine erniedrigte Endviskosität in der RVA Analyse (Abel, 1995, Dissertation der Freien Universität Berlin). Solche Stärken, die auch in WO 00/08184 beschrieben sind, weisen gegenüber Stärken, isoliert aus nicht transformierten Wildtyp-Pflanzen einen um 197% gesteigerten Phosphatgehalt, einen um 123% gesteigerten Amylosegehalt und eine Endviskosität in der RVA Analyse, die auf 76% des Wildtyps sinkt, auf. Außerdem sinkt die Gelfestigkeit der betreffenden Stärke auf 84% des Wildtyps.

Stärken, isoliert aus Pflanzen, die eine verringerte Aktivität sowohl eines BEI -, als auch eines BE II Proteins aufweisen, zeigen in der spektrophotometrischen Analyse nach Morrison & Laignelet (1983, J. Cereal Sc. 1, 9-20) einen Amylosegehalt von 77% bis 89,1% (entspricht maximal 348% der aus Wildtyp-Pflanzen isolierten Stärke) und einen Phosphorgehalt von 2400 µg / g Stärke (entspricht 77,4 µmol Phosphat / g Stärke) bis 3000 µg / g Stärke (entspricht 96,8 µmol Phosphat / g Stärke). Daraus ergibt sich eine maximale Steigerung um 613% im Vergleich zu Stärke, isoliert aus entsprechenden Wildtyp-Pflanzen. Stärken mit mehr als 55% Amylose zeigen keinerlei Verkleisterung mehr (Schwall et al., 2000, Nature Biotechnology 18, 551-554). Stärken mit geringeren Amylosewerten (40,9%) zeigen nach Verkleisterung in der RVA Analyse einen um 256% gesteigerten Wert der Endviskosität und weisen einen Phosphorgehalt von 206 mg / 100 g Stärke (entspricht 66,4 µmol Phosphat / g Stärke) auf. Höhere Phosphorgehalte werden erst erreicht, wenn auch höhere Amylosewerte in den betreffenden Stärken vorliegen, z.B. 240 mg Phosphor /100 g Stärke (entspricht 77,4 µmol Phosphat / g Stärke; WO 9634968). Hofvander et al. (2004, Plant Biotechnology 2, 311-321) beschreiben Stärken, isoliert aus genetisch modifizierten Kartoffelpflanzen mit einem Phosphorgrhalt von 2400 bis 3300 µg / g Stärke (entspricht 77,4 bis 106,4 µmol Phosphat / g Stärke), wobei die Stärken einen Amylosegehalt (spektrophotometrische Bestimmung des Jodbindevermögems) von 47% bis 86% aufwiesen.

Der vorliegenden Erfindung liegt die Aufgabe zu Grunde, Kartoffelstärken mit neuen Eigenschaften, neue Pflanzenzellen und/oder Pflanzen, die solche Stärken herstellen, sowie Mittel und Verfahren zur Erzeugung besagter Pflanzenzellen und/oder Pflanzen zur Verfügung zu stellen.

Diese Aufgabe wird durch die Bereitstellung der in den Patentansprüchen bezeichneten Ausführungsformen gelöst.

Somit bertrifft die vorliegende Erfindung modifizierte Stärke, isoliert aus Kartoffelpflanzen dadurch gekennzeichnet, dass sie
a) einen Amylosegehalt, gemessen nach der Methode von Hovenkamp-Hermelink et al. (1987, Theoretical and Applied Genetics 75, 217-221), zwischen 40% bis 50% enthält und
b) einen Phosphorgehalt von 80 bis 95 µmol Phosphat pro Gramm Stärke (Trockengewicht) aufweist.

Die erhöhten Mengen an Stärkephosphat erfindungsgemäßer Stärken verleihen den Stärken überraschende und vorteilhafte Eigenschaften. Erfindungsgemäße Stärken tragen durch den erhöhten Anteil an Stärkephosphat einen erhöhten Anteil an geladenen Gruppen, die die funktionellen Eigenschaften der Stärke wesentlich beeinflussen. Stärke, die geladene funktionelle Gruppen trägt, ist insbesondere in der Papierindustrie einsetzbar, wo sie für die Oberflächenbeschichtung (Coating) von Papier verwendet werden. Papier, welches mit geladenen Molekülen, die außerdem gute Klebeeigenschaften (Verkleisterungseigenschaften) aufweisen, beschichtet ist, eignet sich besonders für die Aufnahme von Farbstoffen, wie z.B. Tinte, Druckfarben etc..

Bei den erfindungsgemäßen Stärken handelt es sich um native Stärken. Der Begriff "native Stärke" bedeutet im Zusammenhang mit der vorliegenden Erfindung, dass die Stärke nach dem Fachmann bekannten Methoden aus Pflanzen oder Stärke speichernden Teilen von Pflanzen isoliert wird.

Der Amylosegehalt wird im Zusammenhang mit der vorliegenden Erfindung nach der für Kartoffelstärke beschriebenen Methode von Hovenkamp-Hermelink et al. (Potato Research 31, (1988), 241-246) bestimmt (siehe Allgemeine Methoden Punkt 1)

Der Begriff "Phosphatgehalt" der Stärke bezeichnet im Sinne der vorliegenden Erfindung den Gehalt an kovalent in Form von Stärkephosphatmonoestern gebundenem Phosphat.

Methoden zur Bestimmung des Phosphatgehaltes sind dem Fachmann bekannt und in der Literatur ausreichend beschrieben (z.B. Gericke und Kurmies, 1952, Z. Düngg. Pflanzenernähr. Bodenk. 59, 235-247. Bevorzugt wird im Zusammenhang mit der vorliegneden Erfindung die unter Allgemeine Methoden, Punkt 2 beschriebene Methode verwendet.

Verfahren zur Isolierung von Stärke aus Pflanzen oder von Stärke speichernden Teilen von Pflanzen sind dem Fachmann bekannt. Weiterhin sind Verfahren zur Extraktion der Stärke aus verschiedenen Stärke speichernde Pflanzen beschrieben, z. B. in Starch: Chemistry and Technology (Hrsg.: Whistler, BeMiller und Paschall (1994), 2. Ausgabe, Academic Press Inc. London Ltd; ISBN 0-12-746270-8; siehe z. B. Kapitel XII, Seite 412-468: Mais und Sorghum Stärken: Herstellung; von Watson; Kapitel XIII, Seite 469-479: Tapioca, Arrowroot und Sago Stärken: Herstellung; von Corbishley und Miller; Kapitel XIV, Seite 479-490: Kartoffelstärke: Herstellung und Verwendungen; von Mitch; Kapitel XV, Seite 491 bis 506: Weizenstärke: Herstellung, Modifizierung und Verwendungen; von Knight und Oson; und Kapitel XVI, Seite 507 bis 528: Reisstärke: Herstellung und Verwendungen; von Rohmer und Klem; Maisstärke: Eckhoff et al., Cereal Chem. 73 (1996), 54-57, die Extraktion von Maisstärke im industriellen Maßstab wird in der Regel durch das so genannte "wet milling" erreicht.

Stärkephosphat kann in Form von Monoestern an der C-3 oder C-6 Position der polymerisierten Glucosemonomere vorliegen (Blennow et al., 2000, Int. J. of Biological Macromolecules 27, 211-218). Die Phosphatverteilung des Phosphates in von Pflanzen synthetisierter Stärke zeichnet sich im Allgemeinen dadurch aus, dass etwa 30% bis 40% der Phosphatreste in C-3-Position und etwa 60% bis 70% der Phosphatreste in C-6-Position der Glucosemoleküle kovalent gebunden sind (Blennow et al., 2000, Int. J. of Biological Macromolecules 27, 211-218). Erfindungsgemäße Stärken zeichnen sich dadurch aus, dass sie eine Phosphatverteilung mit einem erhöhten C-6-Phosphatgehalt bezogen auf den Gesamtphosphatgehalt aufweisen.

Ein bevorzugte Ausführungsform der vorliegenden Erfindung betrifft daher erfindungsgemäße modifizierte Stärke, dadurch gekennzeichnet, dass sie einen C-6-Phosphorgehalt von 45 bis 60 µmol Phosphat pro Gramm Stärke (Trockengewicht) aufweist.

Unter dem Begriff "C-6-Phosphatgehalt" soll im Zusammenhang mit der vorliegenden Erfindung die Menge an Stärkephosphat verstanden werden, die in C-6-Position der Glucosemoleküle der Stärke kovalent gebunden ist.

Unter dem Begriff "Gesamtphosphatgehalt" soll im Zusammenhang mit der vorliegenden Erfindung die Menge an Stärkephosphat verstanden werden, die insgesamt an Glucosemoleküle der Stärke kovalent gebunden ist.

Zur Bestimmung der Menge an C-6-Phosphat sind verschiedene Methoden beschrieben (z.B. Ritte et al., 2000, Starch/Stärke 52, 179-185). Die Bestimmung der Menge an C-6-Phosphat mittels ³¹P-NMR ist bei Kasemusuwan und Jane (1996, Cereal Chemistry 73, 702-707) beschrieben. Bevorzugt wird die Methode von Ames (Methods in Enzymology VIII, (1966), 115-118) verwendet, besonders bevorzugt wird die unter Allgemeine Methoden Punkt 2 beschrieben Methode verwendet.

Erfindungsgemäße Stärken zeichnen sich auch dadurch aus, dass sie eine veränderte Seitenkettenverteilung des Amylopektins aufweisen im Vergleich zu Stärke (Amylopektin), isoliert aus entsprechenden Wildtyp-Pflanzen.

Ein bevorzugter Gegenstand der Erfindung betrifft daher erfindungsgemäße modifizierte Stärke, die eine veränderte Seitenkettenverteilung des Amylopektins aufweist im Vergleich zu Stärke, isoliert aus entsprechenden Wildtyp-Pflanzen.

Der Begriff "Wildtyp-Pflanze" bedeutet im Zusammenhang mit der vorliegenden Erfindung, dass es sich um Pflanzen handelt, deren genetische Information, abgesehen von genetischen Modifikationen, die die Synthese erfindungsgemäßer Stärke verursachen, der Pflanze entsprechen, die eine erfindungsgemäße Stärke synthetisieren.

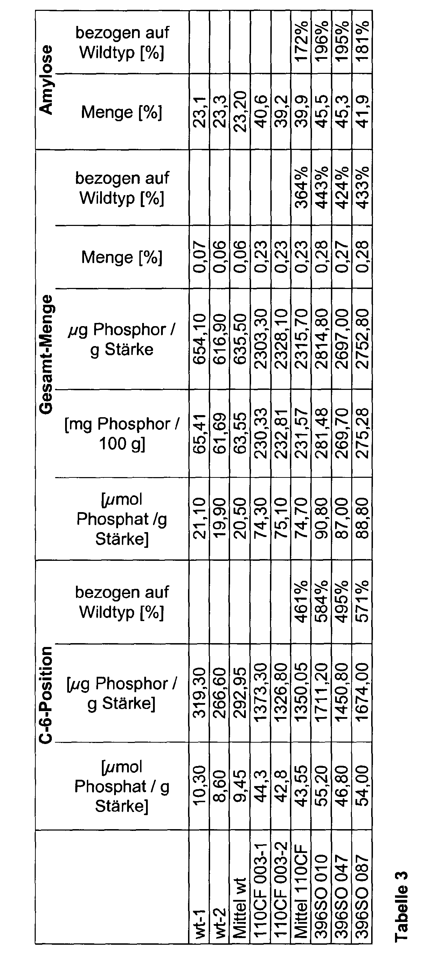

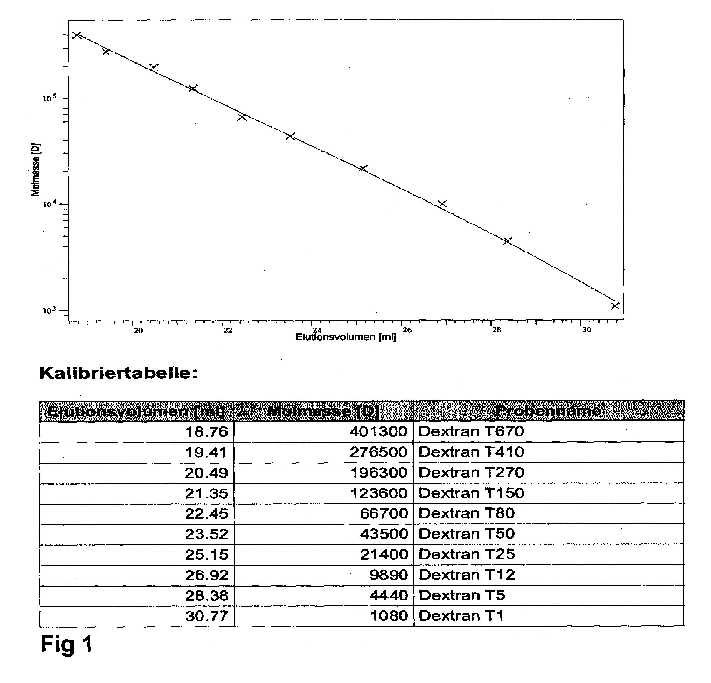

Die Bestimmung der Seitenkettenverteilung erfolgt über die Bestimmung des prozentualen Anteils einer bestimmten Gruppe von Seitenketten am Gesamtanteil aller Seitenketten im GPC-Chromatogramm. Die Gesamtfläche unterhalb der Linie des GPC-Chromatogramms wird dazu in einzelne Abschnitte unterteilt, die jeweils Gruppen von Seitenketten unterschiedlicher Länge repräsentieren. Die gewählten Abschnitte enthalten Seitenketten mit folgendem Polymerisierungsgrad ("Degree of Polymerisation"; DP = Anzahl der Glukosemonomere innerhalb einer Seitenkette): DP kleiner 11, DP 11 bis 18, DP 19 bis 24, DP 25 bis 30, DP 31 bis 36, DP 37 bis 42, DP 43 bis 48, DP 49 bis 55, DP 56 bis 61, DP 62 bis 123 und DP größer 123. Um das Elutionsvolumen mit der Molmasse zu korrelieren wird die verwendete GPC-Säule mit Dextranstandards ((Fluka, Produkt Nr. 31430) kalibriert. Die verwendeten Dextrane, ihre zugehörige Molmasse und die Elutionsvolumina sind in Fig 1 dargestellt. Mit der daraus resultierenden Kalibrierungsgeraden wird das Elutionsdiagramm als Molekulargewichtsverteilung dargestellt. Zur Festlegung des Molekulargewichts der einzelnen Seitenketten wurde für Glukose ein Molekulargewicht von 162 festgelegt. Die gesamte Fläche unterhalb der Linie im GPC Chromatogramm, wird als 100% festgesetzt und der Anteil der Flächen der einzelnen Abschnitte bezogen auf den Anteil der Gesamtfläche berechnet.

Unter dem Begriff veränderte "Seitenkettenverteilung" soll im Zusammenhang mit der vorliegenden Erfindung eine Veränderung des Anteils von Seitenketten des Amylopektins mit einem DP kleiner 11, einem DP von 11 bis 18, einem DP von 19 bis 24, einem DP von 25 bis 30, einem DP von 31 bis 36, einem DP von 37 bis 42, einem DP von 43 bis 48, einem DP von 49 bis 55, einem DP von 56 bis 61, einem DP von 62 bis 123 und/oder einem DP größer 123 bezogen auf die Menge an Seitenketten desselben Polymerisierungsgrades des Amylopektins von Stärke, isoliert aus entsprechenden Wildtyp-Pflanzen verstanden werden.

Vorzugsweise zeichnet sich erfindungsgemäße Stärke dadurch aus, dass der Anteil von Seitenketten des Amylopektins mit einem DP kleiner 11, einem DP von 11 bis 18 und/oder einem DP von 19 bis 24 verringert ist und dass der Anteil von Seitenketten des Amylopektins mit einem DP von 56 bis 61, einem DP von 62 bis 123 und/oder einem DP größer 123 erhöht ist, im Vergleich zu Seitenketten desselben Polymerisierungsgrades des Amylopektins von Stärke, isoliert aus entsprechenden Wildtyp-Pflanzen.
Der Anteil von Seitenketten mit einem DP kleiner 11 des Amylopektins erfindungsgemäßer Stärke ist vorzugsweise um mindestens 65%, bevorzugt mindestens 70%, besonders bevorzugt mindestens 75% und insbesondere bevorzugt mindestens 80% verringert, bezogen auf die Menge an Seitenketten mit einem DP kleiner 11 des Amylopektins von Stärke, isoliert aus entsprechenden Widtyp-Pflanzen.
Der Anteil von Seitenketten mit einem DP von 11 bis 18 des Amylopektins erfindungsgemäßer Stärke ist vorzugsweise um mindestens 40%, bevorzugt mindestens 45%, besonders bevorzugt mindestens 50% und insbesondere bevorzugt mindestens 53% verringert, bezogen auf die Menge an Seitenketten mit einem DP von 11 bis 18 des Amylopektins von Stärke, isoliert aus entsprechenden Widtyp-Pflanzen.
Der Anteil von Seitenketten mit einem DP von 19 bis 24 des Amylopektins erfindungsgemäßer Stärke ist vorzugsweise um mindestens 5%, bevorzugt mindestens 10% und besonders bevorzugt mindestens 15% verringert, bezogen auf die Menge an Seitenketten mit einem DP von 19 bis 24 des Amylopektins von Stärke, isoliert aus entsprechenden Widtyp-Pflanzen.

Der Anteil von Seitenketten mit einem DP von 56 bis 61 des Amylopektins erfindungsgemäßer Stärken ist vorzugsweise um mindestens 20%, bevorzugt mindestens 30%, besonders bevorzugt mindestens 35% und insbesondere bevorzugt mindestens 40% erhöht, bezogen auf die Menge an Seitenketten mit einem DP von (56 bis 61 des Amylopektins von Stärke, isoliert aus entsprechenden Widtyp-Pflanzen.
Der Anteil von Seitenketten mit einem DP von 62 bis 123 des Amylopektins erfindungsgemäßer Stärken ist vorzugsweise um mindestens 100%, bevorzugt mindestens 150%, besonders bevorzugt mindestens 200% und insbesondere bevorzugt mindestens 230% erhöht, bezogen auf die Menge an Seitenketten mit einem DP von 62 bis 123 des Amylopektins von Stärke, isoliert aus entsprechenden Widtyp-Pflanzen.
Der Anteil von Seitenketten mit einem DP größer 123 des Amylopektins erfindungsgemäßer Stärken ist vorzugsweise um mindestens 700%, bevorzugt mindestens 800%, besonders bevorzugt mindestens 900% und insbesondere bevorzugt mindestens 1000% erhöht, bezogen auf die Menge an Seitenketten mit einem DP größer 123 des Amylopektins von Stärke, isoliert aus entsprechenden Widtyp-Pflanzen.

Erfindungsgemäße Stärke besitzt die Eigenschaft, dass sie in eine erhöhte Endviskosität in der RVA Analyse aufweist, wenn die RVA Analyse in einer CaCl₂ enthaltenden wässrigen Lösung durchgeführt wird. Die Zugabe von CaCl₂ bewirkt dabei, dass die Stärke vollständig verkleistert wird.

Ein bevorzugter Gegenstand der vorliegenden Erfindung betrifft daher erfindungsgemäße Stärke, die nach Verkleisterung in der RVA Analyse unter Zusatz von CaCl₂ eine erhöhte Endviskosität aufweist, in Vergleich zu unter gleichen Bedingungen verkleisterter Stärke, isoliert aus Wildtyp-Pflanzen. Bevorzugt wird die RVA Analyse unter Zusatz von mindestens 1,5 M, besonders bevorzugt von mindestens 2 M und insbesondere bevorzugt von mindestens 2,5 M CaCl₂ durchgeführt..

Protokolle zur Durchführung der RVA (Papid Visco Analyser) Analyse ist weiter unten unter Allgemeine Methoden, Punkt 4 beschrieben. Insbesondere ist darauf hinzuweisen, dass in der RVA Analyse von Kartoffelstärken häufig eine 8%ige Stärkesuspension (w/w) eingesetzt wird. In den zum Gerät "RVAsuper3" beigefügten Unterlagen (Gebrauchsanweisung, Newport Scientific Pty Ltd., Investment Support Group, Warried NSW 2102, Australien) wird eine Suspension enthaltend ca. 10% Stärke zur Analyse von Kartoffelstärke empfohlen.
Überraschenderweise wurde im Falle der Stärke aus Kartoffelpflanzen, betreffend die vorliegende Erfindung, gefunden, dass die Verwendung einer 8%igen Stärkesuspension (2g Stärke in 25 ml Wasser) für die Analyse nicht möglich war, weil die Endviskosität Werte erreichte, die von dem Gerät nicht mehr erfasst werden konnten. Darum wurden für die RVA Analyse anstelle von 8%igen Stärkesuspensionen nur 6%ige Stärkesuspensionen (1,5g Stärke in 25 ml Wasser) eingesetzt. Im Zusammenhang mit der vorliegenden Erfindung soll daher unter erhöhter Endviskosität in der RVA Analyse eine Erhöhung um mindestens 100%, besonders um mindestens 120%, insbesondere um mindestens 140% gegenüber nicht genetisch modifizierten Wildtyp Pflanzen verstanden werden. Die Erhöhung der Endviskositäten ist dabei auf 6%ige Stärkesuspensionen zu beziehen, die in einer wässrigen CaCl₂ Lösung durchgeführt wurden. Bevorzugt wird zur Ermittlung der Endviskosität in der RVA Analyse im Zusammenhang mit der vorliegenden Erfindung die unter Allgemeine Methoden, Punkt 4 beschriebene RVA Anylysemethode 1 verwendet.

Weiterhin wurde festgestellt, dass erfindungsgemäße Stärken in der RVA Analyse eine erhöhte Verkleisterungstemperatur aufweisen.

Eine bevorzugte Ausführungsform der vorliegenden Erfindung betrifft daher erfindungsgemäße Stärke, die in der RVA Analyse eine erhöhte Verkleisterungstemperatur aufweist. Die Verkleisterungstemperaturn erfindungsgemäßer Stärke beträgt vorzugsweise mindestens 80°C, bevorzugt mindestens 85°C besonders bevorzugt mindestens 90°C und insbesondere bevorzugt mindestens 92°C. Bevorzugt wird die Verkleisterungstemperatur im Zusammenhang mit der vorliegenden Erfindung mit der unter Allgemeine Methoden Punkt 4 beschriebenen RVA Analysemethode 3 durchgeführt.

Stärken mit erhöhter Verkleisterungstemperatur bieten den Vorteil, dass sie in erwärmten Flüssigkeiten besser dispergierbar sind. Daher eignet sich erfindungsgemäße Stärke besonders für die Herstellung von Nahrungsmitteln, wobei Stärke z.B. als Dickungsmittel zu erwärmten Nahrungsmittelzubereitungen zugegeben wird.

Bevorzugt bildet erfindungsgemäße Stärke nach Verkleisterung Gele, die eine erhöhte Festigkeit aufweisen.

In einer weiteren bevorzugten Ausführungsform betrifft die vorliegende Erfindung erfindungsgemäße Stärke, die nach Verkleisterung in Wasser ein Gel bildet, das im Vergleich zu einem Gel aus Stärke, isoliert von Wildtyp-Pflanzen eine erhöhte Gelfestigkeit aufweist. Besonders bevorzugt bildet erfindungsgemäße Stärke nach Verkleisterung in einer wässrigen CaCl₂ Lösung ein Gel, das eine erhöhte Endviskosität ausbildet, im Vergleich zu Gel aus Stärke, isoliert von entsprechenden Wildtyp-Pflanzen.

Der Vorteil erfindungsgemäßer Stärke gegenüber herkömmlicher Stärke besteht darin, dass sie nach Verkleisterung in salzhaltigen Lösungen festere Gele ausbildet, als herkömmliche Stärke. Daher ist erfindungsgemäße Stärke besonders geeignet für Anwendungen im Nahrungsmittelbereich, wo Stäke häufig als Dickungsmittel eingesetzt wird. Nahrungsmittel enthalten in der Regel Salze. Zum Andicken von Nahrungsmitteln muss daher im Vergleich zu herkömmlicher Stärke weniger erfindungsgemäße Stärke verwendet werden. Dieses spart Kosten und verringert den Kaloriengehalt von Nahrungsmitteln, die erfindungsgemäße Stärke enthalten im Vergleich zu Nahrungsmitteln, die herkömmliche Stärke enthalten.

Unter dem Begriff "erhöhte Gelfestigkeit" soll im Zusammenhang mit der vorliegenden Erfindung eine Erhöhung der Gelfestigkeit, vorzugsweise um mindestens 20%, insbesondere um mindestens 40%, weiter bevorzugt um mindestens 60% und besonders bevorzugt um mindestens 90%, im Vergleich zur Gelfestigkeit von Stärke, isoliert aus Wildtyp-Pflanzen verstanden werden.

Die Bestimmung der Gelfestigkeit soll im Zusammenhang mit der vorliegenden Erfindung mit Hilfe eines Texture Analyzers unter der unter Allgemeine Methoden, Punkt 3 beschriebenen Methode beschriebenen Methode erfolgen.
Um Stärkegele herzustellen muß die kristalline Struktur von nativer Stärke zunächst durch Erhitzen in wässriger Suspension unter ständigem Rühren zerstört werden. Dieses kann mit Hilfe eines Rapid Visco Analysers (Newport Scientific Pty Ltd., Investmet Support Group, Warriewod NSW 2102, Australien) durchgeführt werden. Wie weiter oben bereits ausgeführt, wurde dabei für erfindungsgemäße Stärke aus Kartoffelpflanzen eine 6%ige Stärkesuspension anstatt einer standardmäßig verwendeten 8%igen Suspension eingesetzt,.Zur Bestimmung der Gelfestigkeit werden die im Rapid Visco Analyser verkleisterten Stärkesuspensionen für eine gewisse Zeit gelagert und dann einer Analyse mit einem Texture Analyser unterzogen. Folglich sind auch zur Bestimmung der Gelfestigkeit 6%ige anstelle von 8%igen verkleisterten Stärkesuspensionen eingesetzt worden.

Die erfindungsgemäße Stärke, vorzugsweise native Kartoffelstärke, kann mit Hilfe von Standardverfahren, die dem Fachmann bekannt sind, nach der Extraktion aus Pflanzen oder stärkehaltigen Pflanzenteilen chemisch und/oder physikalisch modifiziert werden.

Erfindungsgemäße Stärke bietet dabei den Vorteil, dass sie bei höheren Temperaturen derivatisiert werden kann, da sie wie oben bereits beschrieben eine erhöhte Verkleisterungstemperatur aufweist, als herkömmliche Stärke. Dadurch können z.B. bei der chemischen Derivatisierung die Reaktionen bei höheren Temperaturen ablaufen, was dazu führt, dass die Reaktionen effizienter abläuft, ohne dass die Struktur des Stärkekorns zerstört wird.
Weiterhin bietet erfindungsgemäße Stärke den Vorteil, dass sie als Ausgangssubstanz für die Derivatisierung besser geeignet ist, als herkömmliche Stärken (z.B. isoliert aus Kartoffel-Wildtyp-Pflanzen), weil sie durch den höheren Gehalt an kovalent gebundenem Stärkephosphat einen höheren Anteil an reaktiven funktionalen Gruppen aufweist, stärker hydrophil ist und chemischen Agenzien besser zugänglich ist.

Die vorliegende Erfindung betrifft daher auch Verfahren zur Herstellung einer derivatisierten Stärke, worin erfindungsgemäße Stärke, nachträglich derivatisiert wird.

Unter dem Begiff "derivatisierte Stärke" soll im Zusammenhang mit der vorliegenden Erfindung eine erfindungsgemäße Stärke verstanden werden, deren Eigenschaften nach der Isolierung aus pflanzlichen Zellen mit Hilfe von chemischen, enzymatischen, thermischen oder mechanischen Verfahren verändert wurde.

In einer bevorzugten Ausführungsform der vorliegenden Erfindung handelt es sich bei der erfindungsgemäßen derivatisierten Stärke um Hitze und/oder Säurebehandelte Stärke.

In einer weiteren bevorzugten Ausführungsform handelt es sich bei den derivatisierten Stärken um Stärkeether, insbeondere um Stärke-Alkylether, O-Allylether, Hydroxylalkylether, O-Carboxylmethylether, stickstoffhaltige Stärkeether, phosphathaltige Stärkeether oder schwefelhaltige Stärkeether.

In einer weiteren bevorzugten Ausführungsform handelt es sich bei den derivatisierten Stärken um vernetzte Stärken.

In einer weiteren bevorzugten Ausführungsform handelt es sich bei den derivatisierten Stärken um Stärke-Pfropf-Polymerisate.

In einer weiteren bevorzugten Ausführungsform handelt es sich bei den derivatisierten Stärken um oxidierte Stärken.

In einer weiteren bevorzugten Ausführungsform handelt es sich bei den derivatisierten Stärken um Stärkeester, insbesondere um Stärkeester, die unter Verwendung von organischen Säuren in die Stärke eingefürhrt wurden. Besonders bevorzugt handelt es sich um Phosphat-, Nitrat-, Sulfat-, Xanthat-, Acetat- oder Citratstärken.

Methoden zur Herstellung von erfindungsgemäßen derivatisierten Stärken sind dem Fachmann bekannt und in der allgemeinen Literatur ausreichend beschrieben. Eine Übersicht zur Herstellung von derivatisierten Stärken findet sich z.B. bei Orthoefer (in Corn, Chemistry and Technology, 1987, eds. Watson und Ramstad, Chapter 16, 479-499).

Derivatisierte Stärke, erhältlich nach dem erfindungsgemäßen Verfahren zur Herstellung einer derivatisierten Stärke ist ebenfalls Gegenstand der vorliegenden Erfindung.

Ferner ist die Verwendung erfindungsgemäßer Stärke zur Herstellung von derivatisierter Stärke Gegenstand der vorliegenden Erfindung.

Erfindungsgemäße Stärke kann durch Isolierung aus genetisch modifizierten Pflanzen hergestellt werden, die im Vergleich zu entsprechenden nicht genetisch modifizierten Wildtyp-Pflanzen eine verringerte Aktivität eines oder mehrerer endogen in der Pflanze vorkommenden SSIII-Proteine und eines oder mehrerer endogen in der Pflanze vorkommenden BEI-Proteine und eines oder mehrerer endogen in der Pflanze vorkommenden BEII-Proteine und eines oder mehrerer endogen in der Pflanze vorkommenden Proteine, die die unter SEQ ID NO 12 oder SEQ ID NO 14 angegebene Aminosäuresequenz aufweisen, hergestellt werden.

Ein weiterer Gegenstand der Erfindung betrifft daher genetisch modifizierte Pflanzen, die eine erfindungsgemäße Stäke synthetisieren und eine verringerte Aktivität
a) eines oder mehrerer endogen in der Pflanze vorkommenden SSIII-Proteine und
b) eines oder mehrerer endogen in der Pflanze vorkommenden BEI-Proteine und
c) eines oder mehrerer endogen in der Pflanze vorkommenden BEII-Proteine und
d) eines oder mehrerer endogen in der Pflanze vorkommenden Proteine, die mindestens 80% Identität mit der unter SEQ ID NO 12 oder SEQ ID NO 14 angegebenen Aminosäuresequenz aufweisen,
aufweist im Vergleich zu entsprechenden nicht genetisch modifizierten Wildtyp-Pflanzen.

Der Begriff "genetisch modifiziert" bedeutet im Zusammenhang mit der vorliegenden Erfindung, dass die Pflanzenzelle in ihrer genetischen Information verändert ist.

Die genetische Modifikation kann dabei jede genetische Modifikation sein, die zu einer Verringerung der Aktivität eines oder mehrerer endogen in der Pflanze vorkommenden SSIII-Proteine und zur Verringerung der Aktivität eines oder mehrerer endogen in der Pflanzen vorkommenden BEI-Proteine und zur Verringerung der Aktivität eines oder mehrerer endogen in der Pflanze vorkommenden BEII-Proteine und zur Verringerung der Aktivität eines oder mehrerer endogen in der Pflanze vorkommenden Proteinen, die die unter SEQ ID NO 12 oder SEQ ID NO 14 angegebene Aminosäuresequenz aufweisen, führt im Vergleich zu entsprechenden nicht genetisch modifizierten Pflanzenzellen von Wildtyp-Pflanzen.

Der Begriff "entsprechend" bedeutet im Zusammenhang mit der vorliegenden Erfindung, dass beim Vergleich von mehreren Gegenständen die betreffenden Gegenstände, die miteinander verglichen werden, unter gleichen Bedingungen gehalten wurden. Im Zusammenhang mit der vorliegenden Erfindung bedeutet der Begriff "entsprechend" im Zusammenhang mit Wildtyp-Pflanze, dass die Pflanzen, die miteinander verglichen werden, unter gleichen Kulturbedingungen aufgezogen wurden und dass sie ein gleiches (Kultur-) Alter aufweisen.

Der Begriff "Verringerung der Aktivität" bedeutet dabei im Rahmen der vorliegenden Erfindung eine Verringerung der Expression endogener Gene, die SSIII-Proteine und/oder BEI-Proteine und/oder BEII-Proteine und/oder Proteine, die die unter SEQ ID NO 12 oder SEQ ID NO 14 angegebene Aminosäuresequenz aufweisen, codieren und/oder eine Verringerung der Menge an SSIII-Protein, BEI-Protein, BEII-Protein und/oder Protein, das die unter SEQ ID NO 12 oder SEQ ID NO 14 angegebene Aminosäuresequenz aufweist, in den Pflanzen und/oder eine Verringerung der enzymatischen Aktivität der SSIII-Proteine, BEI-Proteine, BEII-Proteine und/oder von Proteinen, die die unter SEQ ID NO 12 oder SEQ ID NO 14 angegebene Aminosäuresequenz aufweisen, in den Pflanzen im Vergleich zu entsprechenden nicht genetisch modifizierten Wildtyp-Pflanzen..

Die Verringerung der Expression kann beispielsweise bestimmt werden durch Bestimmung der Menge an codierenden Transkripten von SSIII-Proteinen, BEI-Proteinen, BEII-Proteinen oder Proteinen, die die unter SEQ ID NO 12 oder SEQ ID NO 14 angegebene Aminosäuresequenz aufweisen. Dies kann z.B. durch Northern-Blot-Analyse oder RT-PCR erfolgen. Eine Verringerung bedeutet dabei vorzugsweise eine Verringerung der Menge an Transkripten im Vergleich zu entsprechenden nicht genetisch modifizierten Zellen um mindestens 50%, insbesondere um mindestens 70%, bevorzugt um mindestens 85% und besonders bevorzugt um mindestens 95%. Die Verringerung der Menge an SSIII-Proteinen, BEI-Proteinen, BEII-Proteinen und/oder Proteinen, die die unter SEQ ID NO 12 oder SEQ ID NO 14 angegebene Aminosäuresequenz aufweisen, die eine verringerte Aktivität dieser Proteine in den betreffenden Pflanzenzellen zur Folge hat, kann beispielsweise bestimmt werden durch immunologische Methoden wie Western-Blot-Analyse, ELISA (Enzyme Linked Immuno Sorbent Assay) oder RIA (Radio Immune Assay). Eine Verringerung bedeutet dabei vorzugsweise eine Verringerung der Menge an SSIII-Protein, BEI-Protein, BEII-Protein und/oder Protein, das die unter SEQ ID NO 12 oder SEQ ID NO 14 angegebene Aminosäuresequenz aufweist, im Vergleich zu entsprechenden nicht genetisch modifizierten Pflanzen um mindestens 50%, insbesondere um mindestens 70%, bevorzugt um mindestens 85% und besonders bevorzugt um mindestens 95%.

Unter "SSIII-Protein" ist im Zusammenhang mit der vorliegenden Erfindung eine Klasse von löslichen Stärkesynthasen (ADPGlukose 1,4-alpha-D-glucan 4-alpha-D-glucosyltransferase; EC 2.4.1.21) zu verstehen. Lösliche Stärkesynthasen katalysieren eine Glycosylierungsreaktion, bei der Glukosereste des Substrates ADP-Glukose unter Bildung einer alpha 1,4-Verknüpfung auf alpha-1,4-verknüpfte Glukanketten übertragen werden. (ADP-Glukose + {(1,4)-alpha-D-glucosyl}(N) <=> ADP + {(1,4)-alpha-D-glucosyl}(N+1)).

Beschrieben sind SSIII Proteine zum Beispiel bei Marshall et al. (The Plant Cell 8; (1996); 1121-1135), Li et al. (2000, Plant Physiology 123, 613-624), Abel et al. (The Plant Journal 10(6); (1996); 981-991) und in WO 0066745. SSIII Proteine weisen häufig in ihrem Aufbau eine Abfolge von Domänen auf. SSIII Proteine haben am N-Terminus ein Signalpeptid für den Transport in Plastiden. In Richtung C-Terminus folgen eine N-terminale Region, eine SSIII spezifische Region und eine katalytische Domäne (Li et al., 2000, Plant Physiology 123, 613-624). Weitere Analysen, basierend auf Primärsequenzvergleichen (http://hits.isb-sib.ch/cgi-bin/PFSCAN), ergaben, dass das SSIII Protein aus Kartoffel eine so genannte Kohlenhydrat Bindedomäne (CBM von Carbohydrate Binding Domain) aufweist.. Diese Domäne (Pfam Motiv cbm 25) umfasst die Aminosären 377 bis 437 der in Seq ID Nr. 2 dargestellten Sequenz des SSIII-Proteins aus Kartoffel. Unter einem SSIII Protein sollen daher im Zusammenhang mit der vorliegenden Erfindung Stärkesynthasen verstanden werden, die zu der in Seq ID Nr. 3 dargestellten Sequenz eine Identität von mindestens 50% aufweisen, bevorzugt von mindestens 60%, besonders bevorzugt von mindestens 70%, weiter bevorzugt von mindestens 80%, und insbesondere von mindestens 90%.

Unter dem Begriff Homologie bzw. Identität soll die Anzahl der übereinstimmenden Aminosäuren (Identität) mit anderen Proteinen, ausgedrückt in Prozent verstanden werden. Bevorzugt wird die Identität durch Vergleiche der Seq. ID Nr. 3 zu anderen Proteinen mit Hilfe von Computerprogrammen ermittelt. Weisen Sequenzen, die miteinander verglichen werden, unterschiedliche Längen auf, ist die Identität so zu ermitteln, dass die Anzahl an Aminosäuren, welche die kürzere Sequenz mit der längeren Sequenz gemeinsam hat, den prozentualen Anteil der Identität bestimmt. Die Identität kann standardmäßig mittels bekannten und der Öffentlichkeit zur Verfügung stehenden Computerprogrammen wie z.B. ClustalW (Thompson et al., Nucleic Acids Research 22 (1994), 4673-4680) ermittelt werden. ClustalW wird öffentich zur Verfügung gestellt von Julie Thompson (Thompson@EMBL-Heidelberg.DE) und Toby Gibson (Gibson@EMBL-Heidelberg.DE), European Molecular Biology Laboratory, Meyerhofstrasse 1, D 69117 Heidelberg, Germany. ClustalW kann ebenfalls von verschiedenen Internetseiten, u.a. beim IGBMC (Institut de Genetique et de Biologie Moléculaire et Cellulaire, B.P.163, 67404 Illkirch Cedex, France; ftp://ftp-igbmc.u-strasbg.fr/pub/) und beim EBI (ftp://ftp.ebi.ac.uk/pub/software/) sowie bei allen gespiegelten Internetseiten des EBI (European Bioinformatics Institute, Wellcome Trust Genome Campus, Hinxton, Cambridge CB10 1 SD, UK), heruntergeladen werden.
Wenn das ClustalW Computerprogramm der Version 1.8 benutzt wird, um die Identität zwischen z.B. dem Referenzprotein der vorliegenden Anmeldung und anderen Proteinen zu bestimmen, sind folgende Parameter einzustellen: KTUPLE=1, TOPDIAG=5, WINDOW=5, PAIRGAP=3, GAPOPEN=10, GAPEXTEND=0.05, GAPDIST=8, MAXDIV=40, MATRIX=GONNET, ENDGAPS(OFF), NOPGAP, NOHGAP.
Eine Möglichkeit zum Auffinden von ähnlichen Sequenzen, ist die Durchführung von Sequenzdatenbankrecherchen. Hierbei wird eine oder werden mehrere Sequenzen als sogenannte Abfrage (= query) vorgegeben. Diese Abfragesequenz wird dann mittels statistischen Computerprogrammen mit Sequenzen, dieei in den ausgewählten Datenbanken enthalten sind, verglichen. Solche Datenbankabfragen (blast searches) sind dem Fachmann bekannt und können bei verschiedenen Anbietern durchgeführt werden. Wird eine solche Datenbankabfrage z.B. beim NCBI (National Center for Biotechnology Information, http://www.ncbi.nlm.nih.gov/) durchgeführt, so sollen die Standardeinstellungen, die für die jeweilige Vergleichsanfrage vorgegeben sind, benutzt werden. Für Proteinsequenzvergleiche (blastp) sind dieses folgende Einstellungen: Limit entrez = nicht aktiviert; Filter = low compexity aktiviert; Expect value = 10; word size = 3; Matrix = BLOSUM62; Gap costs: Existence = 11, Extension = 1. Als Ergebnis einer solchen Abfrage werden neben anderen Parametern auch der Anteil an Identität zwischen der Abfragesequenz und den in den Datenbanken aufgefundenen ähnlichen Sequenzen dargestellt.
Unter einem SSIII Protein sollen daher im Zusammenhang mit der vorliegenden Erfindung Stärkesynthasen verstanden werden, die bei der Verwendung mindestens einer der vorstehend beschriebenen Methoden zur Identitätsbestimmung zu der in Seq ID Nr. 3 dargestellten Sequenz eine Identität von mindestens 50%, bevorzugt von mindestens 60%, besonders bevorzugt von mindestens 70%, weiter bevorzugt von mindestens 80%, und insbesondere von mindestens 90% aufweisen, wobei die Identität mit Hilfe mindestens eines der oben beschriebenen Methoden bestimmt wurde.

Im Rahmen der vorliegenden Erfindung wird unter dem Begriff "Branching Enzyme" oder "BE-Protein" (α-1,4-Glucan: α-1,4-Glucan 6-Glycosyltransferase, E.C. 2.4.1.18) ein Protein verstanden, das eine Transglycosylierungsreaktion katalysiert, in der α-1,4-Verknüpfungen eines α-1,4-Glucandonors hydrolysiert und die dabei freigesetzten α-1,4-Glucanketten auf eine α-1,4-Glucanakzeptorkette transferiert und dabei in α-1,6-Verknüpfungen überführt werden.

Unter dem Begriff "BEI-Protein" soll im Zusammenhang mit der vorliegenden Erfindung ein Verzweigungsenzym (branching enzyme = BE) der Isoform I verstanden werden., vorzugsweise stammt das BEI-Protein aus Kartoffelpflanzen. Die Bezeichnung der Isoformen lehnt dabei an der von Smith-White und Preiss vorgeschlagenen Nomenklatur an (Smith-White und Preiss, Plant Mol Biol. Rep. 12, (1994), 67-71, Larsson et al., Plant Mol Biol. 37, (1998), 505-511). Diese Nomenklatur geht davon aus, dass alle Enzyme, die zum BEI-Protein aus Mais (GenBank Acc. No. D11081; Baba et al., Biochem. Biophys. Res. Commun. 181 (1), (1991), 87-94; Kim et al. Gene 216, (1998), 233-243) eine höhere Homologie (Identität) auf Aminosäureebene aufweisen als zum BEII-Protein aus Mais (Genbank Acc. No AF072725 , U65948), als Verzweigungsenzym der lsoform I oder kurz als BEI-Proteine bezeichnet werden.

Unter dem Begriff "BEII-Protein" soll im Zusammenhang mit der vorliegenden Erfindung ein Verzweigungsenzym (branching enzyme = BE) der Isoform II verstanden werden., vorzugsweise stammt dieses aus Kartoffelpflanzen. Im Zusammenhang mit der vorliegenden Erfindung sollen alle Enzyme, die auf Aminosäureebene zum BEII-Protein aus Mais (Genbank Acc. No AF072725, U65948) eine höhere Homologie (Identität) aufweisen als zum BEI-Protein aus Mais (Genbank Acc. No. D 11081, AF 072724), als als Verzweigungsenzym der Isoform II oder kurz BEII-Protein bezeichnet werden.

"Proteine, die die unter SEQ ID NO 12 oder SEQ ID NO 14 angegebene Aminosäuresequenz aufweisen", sind an der Stärkebiosynthese in Pflanzen beteiligt. Aminosäresequenzen, die solche Proteine codieren weisen eine Homologie zu Aminosäuren auf, die Verzweigungsenzym ähnliche Proteine aus *Arabidopsis thaliana* (EMBL acc No: BAB02827) codieren. Es wurde überraschenderweise gefunden, dass Pflanzen, die eine verringerte Aktivität eines Proteins, das die unter SEQ ID NO 12 oder SEQ ID NO 14 angegebene Aminosäuresequenz aufweist, und eine Verringerte Aktivität eines SSIII-Proteins, eines BEI-Proteins und eines BEII-Proteins aufweisen, eine Stärke synthetiseren, die einen erhöhten Phosphatgehalt, eine veränderte Seitenkettenverteilung des Amylopektins und einen erhöhten Amylosegehalt aufweist im Vergleich zu Stärke, isoliert aus Kartoffelpflanzen, die eine verringerte Aktivität eines SSIII-Proteins, eines BEI-Proteins und eines BEII-Proteins aufweisen. Bezüglich der Seitenkettenverteilung bewirkt das Enzym eine Verringerung der Seitenketten mit einem Polymerisierungsgrad von DP kleiner 11 und einem DP von 11 bis 18 und eine Erhöhung des Anteils der Seitenketten mit einem Polymerisierungsgrad von DP 62 bis 123 und einem DP größer 123 im Vergleich zu Stärke, isoliert aus Kartoffelpflanzen, die eine verringerte Aktivität eines SSIII-Proteins, eines BEI-Proteins und eines BEII-Proteins aufweisen. Daraus kann geschlossen werden, dass Proteine, die die unter SEQ ID NO 12 oder SEQ ID NO 14 angegebene Aminosäuresequenz aufweisen, an der Synthese von Seitenketten des Amylopektins von Stärke beteiligt sind.

Die Verringerung der Aktivität eines oder mehrerer endogen in der Pflanze vorkommenden SSIII-Proteine und eines oder mehrerer endogen in der Pflanzen vorkommenden BEI-Proteine und eines oder mehrerer endogen in der Pflanze vorkommenden BEII-Proteine und eines oder mehrerer endogen in der Pflanze vorkommenden Proteine, die die unter SEQ ID NO 12 oder SEQ ID NO 14 angegebene Aminosäuresequenz aufweisen, kann durch Einführung eines oder mehrerer fremder Nukleinsäuremoleküle in die Pflanze erfolgen.

Unter dem Begriff "fremdes Nukleinsäuremolekül" bzw. "fremder Nukleinsäuremoleküle" versteht man im Zusammenhang mit der vorliegenden Erfindung ein solches Molekül, das entweder natürlicherweise in entsprechenden Pflanzen nicht vorkommt, oder das in der konkreten räumlichen Anordnung nicht natürlicherweise in den Pflanzen vorkommt oder das an einem Ort im Genom der Pflanzen lokalisiert ist, an dem es natürlicherweise nicht vorkommt. Bevorzugt ist das fremde Nukleinsäuremolekül ein rekombinantes Molekül, das aus verschiedenen Elementen besteht, deren Kombination oder spezifische räumliche Anordnung natürlicherweise in pflanzlichen Zellen nicht auftritt.

Bei dem bzw. den zur genetischen Modifikation verwendeten fremden Nukleinsäuremolekül(en) kann es sich um ein zusammengefügtes oder mehrere getrennte Nukleinsäuremolekülen handeln, insbesondere so genannte Einfach-, Zweifach-, Dreifach- oder Vierfachkonstrukte. So kann das fremde Nukleinsäuremolekül beispielsweise ein so genanntes "Vierfachkonstrukt" sein, worunter man einen einzigen Vektor zur Pflanzentransformation versteht, der sowohl die genetische Information zur Inhibierung der Expression eines oder mehrerer endogener SSIII-Proteine und zur Inhibierung der Expression eines oder mehrerer BEI-Proteine und zur Inhibierung der Expression eines oder mehrerer BEII-Proteine und zur Inhibierung der Expression eines oder mehrerer Proteine, die die unter SEQ ID NO 12 oder SEQ ID NO 14 angegebene Aminosäuresequenz aufweisen, enthält oder dessen Vorhandensein zur Verringerung der Aktivität eines oder mehrerer SSIII-Proteine, BEI-Proteine, BEII-Proteine und von Proteinen, die die unter SEQ ID NO 12 oder SEQ ID NO 14 angegebene Aminosäuresequenz aufweisen, führt.

In einer weiteren Ausführungsform der Erfindung wird in das Genom der Pflanze nicht ein Vierfachkonstrukt, sondern es werden mehrere unterschiedliche fremde Nukleinsäuremoleküle eingeführt, wobei eines dieser fremden Nukleinsäuremoleküle beispielsweise ein DNA-Molekül ist, das z.B. ein Cosuppressions-Konstrukt darstellt, das eine Verringerung der Expression von einem oder mehreren endogenen SSIII-Proteinen bewirkt, und ein weiteres fremdes Nukleinsäuremolekül ein DNA-Molekül ist, das z.B. eine antisense-RNA codiert, die eine Verringerung der Expression von einem oder mehreren endogenen BEI- und/oder BEII-Proteinen bewirkt. Grundsätzlich ist bei der Konstruktion der fremden Nukleinsäuremoleküle aber auch die Verwendung jeder Kombination aus antisense-, cosuppressions-, Ribozym- und doppelsträngigen RNA Konstrukten oder in-vivo-Mutagenese geeignet, die zu einer gleichzeitigen Verringerung der Expression eines oder mehrerer SSIII-Proteine, BEI-Proteine, BEII-Proteine und Proteinen, die die unter SEQ ID NO 12 oder SEQ ID NO 14 angegebene Aminosäuresequenz aufweisen, führt.
Die fremden Nukleinsäuremoleküle können hierbei zeitgleich ("Cotransformation") oder auch nacheinander, d.h. zeitlich aufeinanderfolgend ("Supertransformation") in das Genom der Pflanzenzelle eingeführt werden.

Die fremden Nukleinsäuremoleküle können auch in verschiedene individuelle Pflanzen einer Spezies eingeführt werden. Es können dabei Pflanzen erzeugt werden, bei weichen die Aktivität von einem Zielprotein, zwei oder drei Zielproteinen reduziert ist. Durch anschließendes Kreuzen können dann Pflanzen erzeugt werden, bei welchen die Aktivität aller vier Zielproteine reduziert ist.

Weiterhin kann zur Einführung eines fremden Nukleinsäuremoleküls bzw. zur Erzeugung der erfindungsgemäßen Pflanzenzellen oder Pflanzen anstelle einer Wildtyp-Pflanzenzelle bzw. Wildtyp-Pflanze eine Mutante, die sich dadurch auszeichnet, dass sie bereits eine verminderte Aktivität für eines oder mehrerer Zielproteine aufweist, herangezogen werden. Bei den Mutanten kann es sich sowohl um spontan auftretende Mutanten, als auch um solche handeln, die durch den gezielten Einsatz von Mutagenen erzeugt wurden.

Die Herstellung von Pflanzen, die eine erfindungsgemäße Stärke synthetisieren, kann durch verschiedene, dem Fachmann bekannte Verfahren erzielt werden, z.B. durch solche, die zu einer Inhibierung der Expression endogener Gene führen, die ein SSIII-Protein, BEI-Protein,. BEII-Protein und/oder ein Protein, das die unter SEQ ID NO 12 oder SEQ ID NO 14 angegebene Aminosäuresequenz aufweist, codieren. Hierzu zählen beispielsweise die Expression einer entsprechenden antisense-RNA, oder eines doppelsträngigen RNA Konstruktes, die Bereitstellung von Molekülen oder Vektoren, die einen Cosuppressionseffekt vermitteln, die Expression eines entsprechend konstruierten Ribozyms, das spezifisch Transkripte spaltet, die ein SSIII-Protein, BEI-Protein, BEII-Protein bzw. ein Protein, das die unter SEQ ID NO 12 oder SEQ ID NO 14 angegebene Aminosäuresequenz aufweist, codieren, oder die so genannte "in-vivo-Mutagenese". Ferner kann die Verringerung der Aktivität von SSIII-Proteinen und/oder der BEI-Proteinen und/oder der BEII-Proteinen und/oder Proteinen, die die unter SEQ ID NO 12 oder SEQ ID NO 14 angegebene Aminosäuresequenz aufweisen, in den Pflanzen auch durch die simultane Expression von sense und antisense RNA Molekülen des jeweiligen zu reprimierenden Zielgens hervorgerufen werden. Diese Methoden sind dem Fachmann geläufig.
Darüber hinaus ist bekannt, dass *in planta* die Bildung von doppelsträngigen RNA-Molekülen von Promotorsequenzen *in trans* zu einer Methylierung und einer transkriptionellen Inaktivierung homologer Kopien dieses Promotors führen kann (Mette et al., EMBO J. 19, (2000), 5194-5201).
Alle diese Verfahren basieren auf der Einführung eines fremden oder mehrerer fremder Nukleinsäuremoleküle in das Genom von Pflanzenzellen.

Zur Inhibierung der Genexpression mittels antisense- oder cosuppressions-Technologie kann beispielsweise ein DNA-Molekül verwendet werden, das die gesamte für ein SSIII-Protein und/oder BEI-Protein, BEII-Protein und/oder ein Protein, das die unter SEQ ID NO 12 oder SEQ ID NO 14 angegebene Aminosäuresequenz aufweist, codierende Sequenz einschließlich eventuell vorhandener flankierender Sequenzen umfaßt, als auch DNA-Moleküle, die nur Teile der codierenden Sequenz umfassen, wobei diese Teile lang genug sein müssen, um in den Zellen einen antisense-Effekt bzw. cosuppressions-Effekt zu bewirken. Geeignet sind im allgemeinen Sequenzen mit einer Mindestlänge von 23 bp, vorzugsweise einer Länge von 100-500 bp, für eine effiziente antisense- bzw. cosuppressions-Inhibition insbesondere Sequenzen mit einer Länge über 500 bp.

Für antisense- oder cosuppressions-Ansätze geeignet ist auch die Verwendung von DNA-Sequenzen, die einen hohen Grad an Homologie zu den endogen in der Pflanzenzelle vorkommenden Sequenzen haben, die SSIII-Proteine, BEI-Proteine, BEII-Proteine bzw. Proteine, die die unter SEQ ID NO 12 oder SEQ ID NO 14 angegebene Aminosäuresequenz aufweisen, codieren. Die minimale Identität sollte größer als ca. 65 % sein. Die Verwendung von Sequenzen mit Homologien von mindestens 90%, insbesondere zwischen 95% und 100% ist zu bevorzugen.

Ferner ist zur Erzielung eines antisense- oder eines cosuppressions-Effektes auch die Verwendung von Introns, d.h. von nicht-codierenden Bereichen von Genen, die für SSIII-Proteine, BEI-Proteine, BEII-Proteine und/oder Proteine, die die unter SEQ ID NO 12 oder SEQ ID NO 14 angegebene Aminosäuresequenz aufweisen, codieren, denkbar.
Die Verwendung von Intron-Sequenzen zur Inhibierung der Genexpression von Genen, die für Proteine der Stärkebiosynthese codieren, wurde beschrieben in den internationalen Patentanmeldungen WO97/04112, WO97/04113, WO98/37213, WO98/37214.
Dem Fachmann ist bekannt, wie er einen antisense- und einen cosuppressions-Effekt erzielen kann. Das Verfahren der cosuppressions-Inhibierung wurde beispielsweise beschrieben in Jorgensen (Trends Biotechnol. 8 (1990), 340-344), Niebel et al., (Curr. Top. Microbiol. Immunol. 197 (1995), 91-103), Flavell et al. (Curr. Top. Microbiol. Immunol. 197 (1995), 43-46), Palaqui und Vaucheret (Plant. Mol. Biol. 29 (1995), 149-159), Vaucheret et al., (Mol. Gen. Genet. 248 (1995), 311-317), de Borne et al. (Mol. Gen. Genet. 243 (1994), 613-621).

Auch die Expression von Ribozymen zur Verringerung der Aktivität von bestimmten Enzymen in Zellen ist dem Fachmann bekannt und ist beispielsweise beschrieben in EP-B1 0321201. Die Expression von Ribozymen in pflanzlichen Zellen wurde z.B. beschrieben in Feyter et al. (Mol. Gen. Genet. 250, (1996), 329-338).

Ferner kann die Verringerung der Aktivität von SSIII-Proteinen und/oder der BEI-AkProteinen tivität und/oder der BEII-Proteinen und/oder von Proteinen, die die unter SEQ ID NO 12 oder SEQ ID NO 14 angegebene Aminosäuresequenz aufweisen, in den Pflanzen auch durch die so genannte "in vivo-Mutagenese" erreicht werden, bei der durch Transformation von Zellen ein hybrides RNA-DNA-Oligonucleotid ("Chimeroplast") in Zellen eingeführt wird (Kipp, P.B. et al., Poster Session beim " 5th International Congress of Plant Molecular Biology, 21.-27. September 1997, Singapore; R. A. Dixon und C.J. Arntzen, Meeting report zu "Metabolic Engineering in Transgenic Plants", Keystone Symposia, Copper Mountain, CO, USA, TIBTECH 15, (1997), 441-447; internationale Patentanmeldung WO 9515972; Kren et al., Hepatology 25, (1997), 1462-1468; Cole-Strauss et al., Science 273, (1996), 1386-1389; Beetham et al., 1999, PNAS 96, 8774-8778).
Ein Teil der DNA-Komponente des RNA-DNA-Oligonucleotids ist homolog zu einer Nukleinsäuresequenz codierend ein endogenes SSIII-Protein, BEI-Protein, ein BEII-Protein und/oder ein Protein, das die unter SEQ ID NO 12 oder SEQ ID NO 14 angegebene Aminosäuresequenz aufweist, weist jedoch im Vergleich zur Nukleinsäuresequenz codierend endogene SSIII-Proteine, BEI-Protein, BEII-Proiteine und/oder Proteine, die die unter SEQ ID NO 12 oder SEQ ID NO 14 angegebene Aminosäuresequenz aufweisen, eine Mutation auf oder enthält eine heterologe Region, die von den homologen Regionen umschlossen ist.
Durch Basenpaarung der homologen Regionen des RNA-DNA-Oligonucleotids und des endogenen Nukleinsäuremoleküls, gefolgt von homologer Rekombination, kann die in der DNA-Komponente des RNA-DNA-Oligonucleotids enthaltene Mutation oder heterologe Region in das Genom einer Pflanzenzelle übertragen werden. Dies führt zu einer Verringerung der Aktivität eines oder mehrerer SSIII-Proteine, BEI-Proteine, BEII-Proteine und/oder von Proteinen, die die unter SEQ ID NO 12 oder SEQ ID NO 14 angegebene Aminosäuresequenz aufweisen.
Ferner kann die Verringerung der Aktivität eines SSIII-Proteins und/oder der BEI-Proteins, der BEII-Proteins und/oder der Aktivität eines Proteins, das die unter SEQ ID NO 12 oder SEQ ID NO 14 angegebene Aminosäuresequenz aufweist, in den Pflanzen auch durch die simultane Expression von sense und antisense RNA Molekülen des jeweiligen zu reprimierenden Zielgens hervorgerufen werden.
Dies kann beispielsweise durch die Verwendung von chimären Konstrukten erreicht werden, die "inverted repeats" des jeweiligen Zielgens oder Teilen des Zielgens enthalten (RNAi Technologie). Hierbei codieren die chimären Konstrukte für sense und antisense RNA Moleküle des jeweiligen Zielgens. Sense und antisense RNA werden *in planta* gleichzeitig als ein RNA-Molekül synthetisiert, wobei sense und antisense RNA durch einen Spacer voneinander getrennt sein und ein doppelsträngiges RNA-Molekül bilden können.
Es konnte gezeigt werden, dass die Einführung von inverted-repeat-DNA-Konstrukten in das Genom von Pflanzen eine sehr effiziente Methode ist, um die zu den inverted-repeat-DNA-Konstrukten korrespondierenden Gene zu reprimieren (Waterhouse et al., Proc. Natl. Acad. Sci. USA 95, (1998), 13959-13964; Wang and Waterhouse, Plant Mol. Biol. 43, (2000), 67-82;Singh et al., Biochemical Society Transactions Vol. 28 part 6 (2000), 925- 927; Liu et al., Biochemical Society Transactions Vol. 28 part 6 (2000), 927-929); Smith et al., (Nature 407, (2000), 319-320; internationale Patentanmeldung WO99/53050 A1). Sense und antisense Sequenzen des Zielgens bzw. der Zielgene können auch getrennt voneinander mittels gleicher oder unterschiedlicher Promotoren exprimiert werden (Nap, J-P et al, 6th International Congress of Plant Molecular Biology, Quebec, 18.-24. Juni, 2000; Poster S7-27, Vortrag Session S7).

Die Verringerung der Aktivität einesSSIII-Proteins und/oder BEI-Proteins und/oder BEII-Proteins und/oder der Aktivität eines Proteins, das die unter SEQ ID NO 12 oder SEQ ID NO 14 angegebene Aminosäuresequenz aufweist, in den Pflanzen kann somit auch durch die Erzeugung doppelsträngiger RNA-Moleküle, die "inverted repeats" von Nucleinsäuresequenzen aufweisen, die SSIII-Proteine und/oder BEI-Proteine und/oder BEII-Proteine codieren und/oder Proteine, die die unter SEQ ID NO 12 oder SEQ ID NO 14 angegebene Aminosäuresequenz aufweisen, erreicht werden. Vorzugsweise werden hierzu "inverted repeats" von DNA-Molekülen, die SSIII-Proteine und/oder BEI-Proteine, BEII-Proteine codieren und/oder von Proteinen, die die unter SEQ ID NO 12 oder SEQ ID NO 14 angegebene Aminosäuresequenz aufweisen, in das Genom von Pflanzen eingeführt, wobei die zu transkribierenden DNA-Moleküle unter Kontrolle eines Promotors stehen, der die Initiation der Transkription in pflanzlichen Zellen besagter DNA-Moleküle bewirkt.

Darüberhinaus ist bekannt, dass die Bildung von doppelsträngigen RNA-Molekülen von Promotor-DNA-Molekülen in Pflanzen *in trans* zu einer Methylierung und einer transkriptionellen Inaktivierung homologer Kopien dieser Promotoren führen kann, die im folgenden als Zielpromotoren bezeichnet werden sollen (Mette et al., EMBO J. 19, (2000), 5194-5201).
Über die Inaktivierung des Zielpromotors ist es somit möglich, die Expression eines bestimmten Proteins (z.B. SSIII-Protein, BEI-Protein, BEII-Protein und/oder Protein, das die unter SEQ ID NO 12 oder SEQ ID NO 14 angegebene Aminosäuresequenz aufweist), das natürlicherweise unter der Kontrolle dieses Zielpromotors steht, zu verringern.

D.h., die DNA-Moleküle, die die Zielpromotoren der zu reprimierenden Gene (Zielgene) umfassen, werden in diesem Fall, im Gegensatz zur ursprünglichen Funktion von Promotoren in Pflanzen, nicht als Steuerelemente zur Expression von Genen oder cDNAs, sondern selbst als transkribierbare DNA-Moleküle verwendet.
Zur Erzeugung der doppelsträngigen Zielpromotor-RNA-Moleküle *in planta,* die dort als RNA-Haarnadel-Moleküle (RNA hairpin) vorliegen können, werden vorzugsweise Konstrukte verwendet, die "inverted repeats" der Zielpromotor-DNA-Moleküle enthalten, wobei die Zielpromotor-DNA-Moleküle unter Kontrolle eines Promotors stehen, der die Genexpression besagter Zielpromotor-DNA-Moleküle steuert. Anschließend werden diese Konstrukte in das Genom von Pflanzen eingeführt. Die Expression der "inverted repeats" besagter Zielpromotor-DNA-Moleküle führt *in planta* zur Bildung doppelsträngiger Zielpromotor-RNA-Moleküle (Mette et al., EMBO J. 19, (2000), 5194-5201). Hierdurch kann der Zielpromotor inaktiviert werden.

Ferner ist dem Fachmann bekannt, dass er die Aktivität eines oder mehrerer SSIII-Proteine, BEI-Proteine, BEII-Proteine und/oder eines oder mehrere Proteine, die die unter SEQ ID NO 12 oder SEQ ID NO 14 angegebene Aminosäuresequenz aufweisen, durch die Expression von nicht-funktionellen Derivaten insbesondere trans-dominanten Mutanten solcher Proteine und/oder durch die Expression von Antagonisten/Inhibitoren solcher Proteine erreichen kann.
Antagonisten/Inhibitoren solcher Proteine umfassen beispielsweise Antikörper, Antikörperfragmente oder Moleküle mit ähnlichen Bindungseigenschaften. Beispielsweise wurde ein cytoplasmatischer scFv Antikörper eingesetzt, um die Aktivität des Phytochrom A Proteins in gentechnisch veränderten Tabakpflanzen zu modulieren (Owen, Bio/Technology 10 (1992), 790-4; Review: Franken, E, Teuschel, U. und Hain, R., Current Opinion in Biotechnology 8, (1997), 411-416; Whitelam, Trends Plant Sci. 1 (1996), 268-272).

Sinnvolle Promotoren für die Expression von Nukleinsäuren, die die Aktivität eines Zielgens verringern, sind z.B. der Promotor der 35S RNA des Cauliflower Mosaic Virus und der Ubiquitin-Promotor aus Mais für eine konstitutive Expression, der Patatingen-Promotor B33 (Rocha-Sosa et al., EMBO J. 8 (1989), 23-29), der MCPI-Promotor des Metallocarboypeptidase-Inhibitor-Gens aus Kartoffel (ungarische Patentanmeldung HU9801674) oder der GBSSI-Promotor aus Kartoffel (internationale Patentanmeldung WO 92/11376) für eine knollenspezifische Expression in Kartoffeln.
Die Expression des fremden Nukleinsäuremoleküls (der fremden Nukleinsäuremoleküle) ist insbesondere in solchen Organen der Pflanze von Vorteil, die Stärke speichern. Solche Organe sind insbesondere Knollen der Kartoffelpflanzen.
Es können jedoch auch Promotoren verwendet werden, die nur zu einem durch äußere Einflüsse determinierten Zeitpunkt aktiviert werden (siehe beispielsweise WO 93/07279). Von besonderem Interesse können hierbei Promotoren von heat-shock Proteinen sein, die eine einfache Induktion erlauben.

Weiterhin kann eine Terminationssequenz vorhanden sein, die der korrekten Beendigung der Transkription dient sowie der Addition eines Poly-A-Schwanzes an das Transkript, dem eine Funktion bei der Stabilisierung der Transkripte beigemessen wird. Derartige Elemente sind in der Literatur beschrieben (vgl. z.B. Gielen et al., EMBO J. 8 (1989), 23-29) und sind beliebig austauschbar.

Für die Einführung von DNA in eine pflanzliche Wirtszelle steht eine Vielzahl von Techniken zur Verfügung. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, die Injektion, die Elektroporation von DNA, die Einbringung der DNA mittels des biolistischen Ansatzes sowie weitere Möglichkeiten.
Die Verwendung der Agrobakterien-vermittelten Transformation von Pflanzenzellen ist intensiv untersucht und ausreichend in EP 120516; Hoekema, IN: The Binary Plant Vector System Offsetdrukkerij Kanters B.V. Alblasserdam (1985), Chapter V; Fraley et al., Crit. Rev. Plant Sci. 4, 1-46 und bei An et al. EMBO J. 4, (1985), 277-287 beschrieben worden. Für die Transformation von Kartoffel, siehe z.B. Rocha-Sosa et al., EMBO J. 8, (1989), 29-33).

Pflanzenzellen und Pflanzen, die durch Einführung eines fremden Nucleinsäuremoleküls genetisch modifiziert sind, lassen sich von Wildtyp-Pflanzenzellen bzw. Wildtyp-Pflanzen unter anderem dadurch unterscheiden, dass sie ein fremdes Nucleinsäuremolekül enthalten, das natürlicherweise in Wildtyp-Planzenzellen bzw. Wildtyp-Pflanzen nicht vorkommt oder dadurch, dass ein solches Molekül an einem Ort im Genom der erfindungsgemäßen Pflanzenzelle oder im Genom der erfindungsgemäßen Pflanze integriert vorliegt, an dem es bei Wildtyp-Pflanzenzellen bzw. Wildtyp-Pflanzen nicht vorkommt, d.h. in einer anderen genomischen Umgebung. Ferner lassen sich derartige erfindungsgemäße Pflanzenzellen und erfindungsgemäße Pflanzen von Wildtyp-Pflanzenzellen bzw. Wildtyp-Pflanzen dadurch unterscheiden, dass sie mindestens eine Kopie des fremden Nucleinsäuremoleküls stabil integriert in ihr Genom enthalten, gegebenenfalls zusätzlich zu natürlicherweise in den Wildtyp-Pflanzenzellen bzw. Wildtyp-Pflanzen vorkommenden Kopien eines solchen Moleküls. Handelt es sich bei dem (den) in die erfindungsgemäßen Pflanzenzellen oder erfindungsgemäßen Pflanzen eingeführten fremden Nucleinsäuremolekül(en) um zusätzliche Kopien zu bereits natürlicherweise in den Wildtyp-Pflanzenzellen bzw. Wildtyp-Pflanzen vorkommenden Molekülen, so lassen sich die erfindungsgemäßen Pflanzenzellen und die erfindungsgemäßen Pflanzen von Wildtyp-Pflanzenzellen bzw. Wildtyp-Pflanzen insbesondere dadurch unterscheiden, dass diese zusätzliche(n) Kopie(n) an Orten im Genom lokalisiert ist (sind), an denen sie bei Wildtyp-Pflanzenzellen bzw. Wildtyp-Pflanzen nicht vorkommt (vorkommen). Dies lässt sich beispielsweise mit Hilfe einer Southern Blot-Analyse nachprüfen.
Weiterhin lassen sich die erfindungsgemäßen Pflanzenzellen und die erfindungsgemäßen Pflanzen von Wildtyp-Pflanzenzellen bzw. Wildtyp-Pflanzen vorzugsweise durch mindestens eines der folgenden Merkmale unterscheiden: Ist das eingeführte fremde Nucleinsäuremolekül heterolog in Bezug auf die Pflanzenzelle oder Pflanze, so weisen die erfindungsgemäßen Pflanzenzellen oder erfindungsgemäßen Pflanzen Transkripte der eingeführten Nucleinsäuremoleküle auf. Diese lassen sich z. B. durch Northern-Blot-Analyse oder durch RT-PCR (Reverse Transcription Polymerase Chain Reaction) nachweisen. Erfindungsgemäße Pflanzenzellen und erfindungsgemäße Pflanzen, die ein Antisense- und/oder ein RNAi-Transkript exprimieren, können z.B. mit Hilfe von spezifischen Nucleinsäure-Sonden, die komplementär zur der für das Protein codierenden (natürlich in der Pflanzenzelle vorkommenden) RNA sind, nachgewiesen werden.

Der Begriff "Kartoffelpflanze" oder "Kartoffel" meint im Zusammenhang mit der vorliegenden Erfindung Pflanzenspezies der Gattung *Solanum,* besonders Knollen produzierende Spezies der Gattung *Solanum* und insbesondere *Solanum tuberosum*.

Ein weiterer Gegenstan der vorliegendenb Erfindung betrifft ein Verfahren zur Herstellung einer erfindungsgemäßen genetisch modifizierten, worin
a) eine Pflanzenzelle genetisch modifiziert wird, wobei die genetische Modifikation zur Verringerung der Aktivität eines oder mehrerer endogen in der Pflanze vorkommenden SSIII-Proteine und eines oder mehrerer endogen in der Pflanze vorkommenden BEI-Proteine und eines oder mehrerer endogen in der Pflanze vorkommenden BEII-Proteine und eines oder mehrerer endogen in der Pflanze vorkommenden Proteine, die mindestens 80% Identität mit der unter SEQ ID No 12 oder SEQ ID NO 14 angegebenen Aminosäuresequenz aufweisen, im Vergleich zu entsprechenden nicht genetisch modifizierten Wildtyp-Pflanzenzellen führt;
b) aus Pflanzenzellen von Schritt a) eine Pflanze regeneriert wird; und
c) gegebenenfalls weitere Pflanzen mit Hilfe der Pflanzen nach Schritt b) erzeugt werden.

Für die laut Schritt a) in die Pflanzenzelle eingeführte genetische Modifikation gilt, dass es sich grundsätzlich um jede Art von Modifikation handeln kann, die zur Verringerung der Aktivität eines oder mehrerer endogen in der Pflanze vorkommenden SSIII-Proteine und eines oder mehrerer endogen in der Pflanze vorkommenden BEI-Proteine und eines oder mehrerer endogen in der Pflanze vorkommenden BEII-Proteine und eines oder mehrerer endogen in der Pflanze vorkommenden Proteine, die mindestens 80% Identität mit der unter SEQ ID NO 12 oder SEQ ID NO 14 aufweisen, führt.
Die Regeneration der Pflanzen gemäß Schritt (b) kann nach dem Fachmann bekannten Methoden erfolgen (z.B. beschrieben in "Plant Cell Culture Protocols", 1999, edt. by R.D. Hall, Humana Press, ISBN 0-89603-549-2).

Die Erzeugung weiterer Pflanzen gemäß Schritt (c) des erfindungsgemäßen Verfahrens kann z.B. erfolgen durch vegetative Vermehrung (beispielsweise über Stecklinge, Knollen oder über Calluskultur und Regeneration ganzer Pflanzen) oder durch sexuelle Vermehrung. Die sexuelle Vermehrung findet dabei vorzugsweise kontrolliert statt, d.h. es werden ausgewählte Pflanzen mit bestimmten Eigenschaften miteinander gekreuzt und vermehrt. Die Auswahl erfolgt dabei bevorzugt in der Weise, dass die weiteren Pflanzen, die nach Schritt c) erhalten werden, die genetische Modifikation, die in Schritt a) eingeführte wurde, aufweisen.

### Beschreibung der Sequenzen

Seq ID 1: Nukleinsäuresequnz der Stärkesynthase SSIII aus Kartoffel (*Solanum tuberosum*) mit Angabe der Sequenzen, die für das entsprechende SSIII Protein kodieren.
Seq ID 2: Aminosäuresequenz eines SSIII Proteins aus Kartoffel.
Seq ID 3: Aminosäuresequenz der Pfam cbm25 Bindedömäne des SSIII-Proteins aus Kartoffel *(Solanum tuberosum)*.
Seq ID 4: Kodierende Nukleinsäuresequenz des Verzweigungsenzyms BEI aus Kartoffel (*Solanum tuberosum).*
Seq ID 5: Aminosäuresequenz des Verzweigungsenzyms BEI aus Kartoffel *(Solanum tuberosum)*
Seq ID 6: Kodierende Nukleinsäuresequenz des Verzweigungsenzyms BEII aus Kartoffel (*Solanum tuberosum).*
Seq ID 7: Aminosäuresequenz des Verzweigungsenzyms BEII aus Kartoffel (*Solanum tuberosum)*
Seq ID 8: Mittels PCR amplifizierte Nukleinsäuresequenz des Verzweigungsenzyms BEII aus Kartoffel (*Solanum tuberosum*)
SEQ ID NO 9: Nucleinsäuresequenz enthaltend die codierende Region des 3'-Bereichs eines an der Stärkebiosynthese beteiligten Proteins aus *Solanum tuberosum* (cv Désirée). Diese Sequenz ist in Plasmid AN 46-196 inseriert.
SEQ ID NO 10: Nucleinsäuresequenz enthaltend die codierende Region des 5'-Bereichs eines an der Stärkebiosynthese beteiligten Proteins aus *Solanum tuberosum* (cv Désirée). Diese Sequenz ist in Plasmid AN 47-196 inseriert.
SEQ ID NO 11: Nucleinsäuresequenz enthaltend die vollständige codierende Region eines an der Stärkebiosynthese beteiligten Proteins aus *Solanum tuberosum* (cv Désirée). Diese Sequenz ist in Plasmid AN 49 inseriert und wurde nach dem Budapester Vertrag hinterlegt am 15. September 2003 unter der Nummer DSM 15926 bei der Deutschen Sammlung von Mikroorganismen und Zellkulturen GmbH, Mascheroder Weg 1 b, 38124 Braunschweig, Deutschland.
SEQ ID NO 12: Aminosäuresequenz codierend eine an der Stärkebiosynthese beteiligtes Protein aus *Solanum tuberosum* (cv Desiree). Diese Sequenz ist von der in Plasmid AN 49 inserierten Nucleinsäuresequenz bzw. von der unter SEQ ID NO 11 beschriebenen Nucleinsäuresequenz ableitbar.
SEQ ID NO 13: Nucleinsäuresequenz enthaltend die vollständige codierende Region eines an der Stärkebiosynthese beteiligten Proteins aus *Solanum tuberosum* (cv Désirée). Diese Sequenz wurde erhalten durch Zusammenfügen der unter SEQ ID NO 9 und SEQ ID NO 10 beschriebenen Nucleinsäuresequenzen. Diese Nucleinsäuresequenz stellt eine allelische Variante der unter SEQ ID NO 11 beschriebenen Nucleinsäuresequenz, codierend ein an der Stärkebiosynthese beteiligtes Protein dar.
SEQ ID NO 14: Aminosäuresequenz codierend ein an der Stärkebiosynthese beteiligtes Protein aus *Solanum tuberosum* (cv Désirée). Diese Sequenz ist. von der unter SEQ ID NO 13 beschriebenen Nucleinsäuresequenz ableitbar und stellt die Aminosäuresequenz einer allelischen Variante zu der unter SEQ ID NO 12 beschriebenen Aminosäuresequenz, codierend ein an der Stärkebiosynthese beteiligtes Protein dar.

### Beschreibung der Abbildungen

- Fig 1:: Kalibrierkurve und Tabelle mit zugehörigen Dextran-Standards

### Allgemeine Methoden

### Stärkeanalytik

1. Bestimmung des Amylosegehaltes bzw. des Amylose/Amylopektinverhältnisses Stärke wurde nach Standardmethoden aus Kartoffelpflanzen isoliert, und der Amylosegehalt sowie das Verhältnis Amylose zu Amylopektin wurde nach der von Hovenkamp-Hermelink et al. beschriebenen Methode (Potato Research 31, (1988), 241-246) bestimmt.
2. Bestimmung des Phosphatgehaltes
In der Stärke können die Positionen C2, C3 und C6 der Glukoseeinheiten phosphoryliert sein. Zur Bestimmung des C6-P-Gehaltes der Stärke werden 50 mg Stärke in 500 µl 0,7 M HCI 4 h bei 95°C hydrolysiert. Anschließend werden die Ansätze für 10 min bei 15500xg zentrifugiert und die Überstände abgenommen. Von den Überständen werden 7µl mit 193 µl Imidazol-Puffer (100 mM Imidazol, pH 7,4; 5 mM MgCl₂, 1 mM EDTA und 0,4 mM NAD) gemischt. Die Messung wurde im Photometer bei 340 nm durchgeführt. Nach der Etablierung einer Basisabsorption wurde die Enzymreaktion durch die Zugabe von 2 Einheiten (units) Glukose-6-Phosphat Dehydrogenase (von Leuconostoc mesenteroides, Boehringer Mannheim) gestartet. Die Absorptionsänderung ist direkt proportional zur Konzentration des G-6-P Gehaltes der Stärke.
Die Bestimmung des Gesamtphosphatgehaltes erfolgte nach der Methode von Ames (Methods in Enzymology VIII, (1966), 115-118).
Es werden ca. 50 mg Stärke mit 30 µl ethanolischer Magnesiumnitrat-Lösung versetzt und drei Stunden bei 500°C im Muffelofen verascht. Der Rückstand wird mit 300 µl 0,5 M Salzsäure versetzt und 30 min bei 60°C inkubiert. Anschließend wird ein Aliquot auf 300 µl 0,5 M Salzsäure aufgefüllt, zu einer Mischung aus 100 µl 10%iger Ascorbinsäure und 600 µl 0,42% Ammoniummolybdat in 2 M Schwefelsäure gegeben und 20 min bei 45°C inkubiert.
Es folgt eine photometrische Bestimmung bei 820nm unter Berücksichtigung einer Phosphat-Eichreihe als Standard.
3. Bestimmung der Gelfestigkeit (Texture Analyser)
1,5 g Stärke (TS) werden in 25 ml einer wäßrigen Suspension im RVA-Gerät verkleistert (für die Bedingungen siehe Allgemeine Methoden, Punkt 4: RVA Analysemethode 1) und anschließend für 24 h bei Raumtemperatur in einem geschlossenen Gefäß gelagert. Die Proben werden unter der Sonde (zylindrischer Stempel mit planer Oberfläche) eines Texture Analysers TA-XT2 der Firma Stable Micro Systems (Surrey, UK) fixiert und die Gelfestigkeit mit folgenden Parametern bestimmt:

| | |
|---|---|
| - Test-Geschwindigkeit | 0,5 mm/s |
| - Eindringtiefe | 7 mm |
| - Kontaktfläche | 113 mm² |
| - Druck | 2 g |

4. Bestimmung der Viskositätseigenschaften mittels eines Rapid Visco Analyzers (RVA)

### Standardmethode

2 g Stärke (TS) werden in 25 ml H₂O (VE Wasser, Leitfähigkeit von mindestens 15 mega Ohm) aufgenommen und für die Analyse in einem Rapid Visco Analyser (Newport Scientific Pty Ltd., Investmet Support Group, Warriewod NSW 2102, Australien) verwendet. Der Betrieb des Gerätes erfolgt nach den Angaben des Herstellers. Dabei erfolgt die Angabe der Viskositätswerte in RVUs gemäß der Betriebsanleitung des Herstellers, die hiermit insoweit durch Bezugnahme in die Beschreibung aufgenommen wird. Zur Bestimmung der Viskosität der wäßrigen Lösung der Stärke wird die Stärkesuspension zunächst für eine Minute auf 50°C erhitzt (Schritt 1), danach von 50°C auf 95°C mit einer Geschwindigkeit von 12°C pro Minute (Schritt 2) erhitzt. Anschließend wird die Temperatur für 2.5 Min bei 95°C gehalten (Schritt 3). Danach wird die Lösung von 95°C auf 50°C abgekühlt, mit einer Geschwindigkeit von 12°C pro Minute (Schritt 4). Während der gesamten Dauer wird die Viskosität bestimmt.

Insbesondere in den Fällen, wo bei der Einwage von 2,0 g (TS) Stärke in 25 ml H₂O (VE Wasser, Leitfähigkeit von mindestens 15 mega Ohm) die Grenzen des Meßbereichs des RVA nicht ausreichten, wurden nur 1,5 g Stärke (TS) in 25 ml H₂O (VE Wasser, Leitfähigkeit von mindestens 15 mega Ohm) aufgenommen.

### RVA- Analysemethode 1:

Zur Bestimmung der Viskosität einer 6%igen wässrigen Lösung der Stärke wird die Stärkesuspension zunächst für 10 Sekunden mit 960 rpm gerührt, um anschließend bei einer Rührgeschwindigkeit von 160 rpm für zunächst eine Minute auf 50 °C erhitzt zu werden (Schritt 1). Danach erfolgt eine Erhöhung der Temperatur von 50 °C auf 95° C mit einer Heizgeschwindigkeit von 12°C pro Minute (Schritt 2). Die Temperatur wird für 2,5 Minuten bei 95 °C gehalten (Schritt 3) und danach mit 12° C pro Minute von 95 °C auf 50 °C gekühlt (Schritt 4). Der letzte Schritt (Schritt 5) hält die Temperatur von 50° C für 2 Minuten.
Nach Beendigung des Programms wird der Rührer entfernt und der Becher abgedeckt. Die verkleisterte Stärke steht nun für die Texturanalyse nach 24 h zur Verfügung.

### RVA Analysemethode 2:

Zur Bestimmung der Viskosität einer 6%igen wässrigen Lösung der Stärke mit 2,7 M Calciumchlorid wird die Stärkesuspension zunächst bei 30 °C für 10 Sekunden mit 960 rpm gerührt (Schritt 1). Danach erfolgt bei einer Rührgeschwindigkeit von 160 rpm eine Erhöhung der Temperatur von 30 °C auf 95 °C mit einer Heizgeschwindigkeit von 12 °C pro Minute (Schritt 2). Die Temperatur wird für 2 Minuten und 30 Sekunden bei 95 °C gehalten (Schritt 3) und danach mit 12 °C pro Minute von 95 °C auf 50 °C gekühlt (Schritt 4). Der letzte Schritt (Schritt 5) hält die Temperatur von 50°C für 2 Minuten.
Nach Beendigung des Programms wird der Rührer entfernt und der Becher abgedeckt. Die verkleisterte Stärke steht nun für die Texturanalyse nach 24 h zur Verfügung.

Um eine eine Erhöhung der Verkleisterungstemperatur deutlich darzustellen, wurde zudem in einigen Fällen ein geändertes Temperaturprofil benutzt.
Folgendes Temperaturprofil wurden verwendet:

### RVA Analysemethode 3

Zur Bestimmung der Viskosität einer 6%igen wässrigen Lösung der Stärke wird die Stärkesuspension zunächst für 10 Sekunden mit 960 rpm gerührt, um anschließend bei einer Rührgeschwindigkeit von 160 rpm für zunächst zwei Minuten auf 50 °C erhitzt zu werden (Schritt 1). Danach erfolgt eine Erhöhung der Temperatur von 50 °C auf 95 °C mit einer Heizgeschwindigkeit von 1,5 °C pro Minute (Schritt 2). Die Temperatur wird für 15 Minuten bei 95 °C gehalten (Schritt 3) und danach mit 1.5 °C pro Minute von 95 °C auf 50 °C gekühlt (Schritt 4). Der letzte Schritt (Schritt 5) hält die Temperatur von 50°C für 30 Minuten.
Nach Beendigung des Programms wird der Rührer entfernt und der Becher abgedeckt. Die verkleisterte Stärke steht nun für die Texturanalyse nach 24 h zur Verfügung.

Im Profil der RVA Analyse gibt es charakteristische Werte, die zum Vergleich unterschiedlicher Messungen und Substanzen dargestellt werden. Die folgenden Begriffe sollen im Zusammenhang mit der vorliegenden Erfindung wie folgt verstanden werden:
Maximale Viskosität (RVA Max)
Unter der maximalen Viskosität versteht man den höchsten Viskositätswert, gemessen in RVUs, der in Schritt 2 oder 3 des Temperaturprofils erreicht wird.
Minimale Viskosität (RVA Min)
Unter der minimalen Viskosität versteht man den geringsten Viskositätswert, gemessen in RVUs, der nach der Maximalen Viskosität im Temeraturprofil auftritt. Normalerweise erfolgt dieses in Schritt 3 des Temperaturprofils.
Finale Viskosität (RVA Fin)
Unter der Finalen Viskosität versteht man den Viskositätswert, gemessen in RVUs, der am Ende der Messung auftritt.
Setback (RVA Set)
Der so genannte "Setback" wird berechnet, indem man den Wert der Finalen Viskososität von der desjenigen Minimums, welches im Kurvenverlauf nach erreichen der Maximalen Viskosität auftritt, subtrahiert.
Verkleisterungstemperatur
Unter der Verkleisterungstemperatur versteht man die Temperatur im RVA Profil, bei welcher die Viskosität zum erstem mal stark innerhalb einer kurzen Periode ansteigt.
Peak Time (RVA T) ist die "peak time"
Unter der Peak Time versteht man die Zeit im Temperaturprofil, zu welcher die Viskosität den maximalen Wert erreicht hat.

### 5. Extraktionsprozess der Stärke aus Kartoffelknollen

Alle Knollen einer Linie (4 bis 5 kg) werden gemeinsam in einem handelsüblichen Entsafter (Multipress automatic MP80, Braun) aufgearbeitet. Das stärkehaltige Fruchtwasser wird in einem 10 L Eimer (Verhältnis Höhe des Eimers/Durchmesser des Eimers = ca. 1,1), in dem 200 ml Leitungswasser mit einer Löffelspitze (ca. 3-4 g) Natrium-Disulfit vorgelegt wurden, aufgefangen. Im Anschluss wird der Eimer mit Leitungswasser vollständig aufgefüllt. Nach einem 2 stündigen Absetzen der Stärke wird der Überstand abdekantiert, die Stärke erneut in 10 I Leitungswasser aufgeschwemmt und über ein Sieb mit 125 µm Maschenweite gegeben. Nach 2 Stunden (Stärke hat sich erneut am Boden des Eimers abgesetzt) wird erneut der wässrige Überstand dekantiert. Dieser Waschvorgang wird noch 3 mal wiederholt, so dass die Stärke insgesamt fünf mal in frischem Leitungswasser resuspendiert wird.

Im Anschluss werden die Stärken bei 37 °C auf einen Wassergehalt von 12 - 17% getrocknet und im Mörser homogenisiert. Die Stärken stehen nun für Analysen zu Verfügung.

### 6. Analyse der Seitenkettenverteilung des Amylopektins mittels Gelpermeationschromatographie

Zur Trennung von Amylose und Amylopektin werden 100 mg Stärke in 6 ml 90 % (v/v) DMSO unter ständigem Rühren gelöst. Nach Zugabe von 3 Volumen Ethanol wird das Präzipitat durch 10 minütige Zentrifugation bei 1800 g bei Raumtemperatur abgetrennt. Das Pellet wird anschließend mit 30 ml Ethanol gewaschen, getrocknet und in 10 ml 1 %iger (w/v) NaCI-Lösung bei 60 °C gelöst. Nach Abkühlung der Lösung auf 30 °C werden langsam etwa 50 mg Thymol zugegeben und diese Lösung für 2 bis 3 Tage bei 30°C inkubiert. Anschließend wird die Lösung für 30 min bei 2000 g bei Raumtemperatur zentrifugiert. Der Überstand wird mit 3 Volumen Ethanol versetzt und das ausfallende Amylopektin mittels 5 minütiger Zentrifugation bei 2000 x g bei Raumtemperatur abgetrennt. Das Pellet (Amylopektin) wird mit 10 ml 70 % (v/v) Ethanol gewaschen, 10 min bei 2000 x g bei Raumtemperatur zentrifugiert und mittels Aceton getrocknet.
Anschließend werden 10 mg Amylopektin in 250 µl 90 % (v/v) DMSO für 10 Minuten bei 70 °C gerührt. Der Lösung werden 375 µl Wasser bei 80 °C zugegeben bis zum vollständiges Lösen.
Von dieser Lösung werden 200 µl mit 300 µl einer 16,6 mM Natriumacetat-Lösung pH 3,5 und 2 µl Isoamylase (0,24 u/µl, Megazyme, Sydney, Australien) versetzt und für 15 Stunden bei 37 °C inkubiert.
Eine 1:4 Verdünnung dieses wässrigen Isoamylase Reaktionsgemisches mit DMSO, enthaltend 90 mM Na-Nitrat, wird anschließend mit einem 0.2 µm Filter filtriert und davon 24 µl des Filtrats chromatografisch analysiert. Die Separation erfolgte mit zwei Säulen, welche in Serie verbunden sind. Zuerst eine Gram PSS3000 (Polymer Standards Service, mit entsprechender Vorsäule) gefolgt von einer Gram PSS100. Die Detektion erfolgte mittels Refractionsindex Detektor (RI 71, Shodex). Die Säule wurde äquilibriert mit DMSO enthaltend 90 mM Natriumnitrat. Eluiert wurde mit DMSO, enthaltend 90 mM Natriumnitrat mit einer Flußrate von 0,7 ml/min über einen Zeitraum von 1 Stunde.
Um das Elutionsvolumen mit der Molmasse und damit mit der Kettenlänge der Seitenketten zu korrelieren, wurde die verwendete Säule mit Dextranstandards kalibriert. Die verwendeten Dextrane, ihre zugehörige Molmasse und die Elutionsvolumina sind in Fig 1 dargestellt.
Zur weiteren Auswertung der erhaltenen Chromatogramme wurde das Programm Wingpc Version 6 der Firma Polymer Standards Service GmbH, Mainz, Germany verwendet.
Die Gesamtfläche unterhalb der Linie des GPC Chromatogramms wurde in einzelne Abschnitte unterteilt, die jeweils Gruppen von Seitenketten unterschiedlicher Länge repräsentieren. Die gewählten Abschnitte enthielten dabei Glukanketten mit folgendem Polymerisierungsgrad (DP = Anzahl der Glukosemonomere innerhalb einer Seitenkette): DP kleiner 11, DP12-18, DP19-24, DP25-30, DP31-36, DP37-42, DP43-48, DP49-55, DP56-61, DP62-123 und DP größer 123). Zur Festlegung des Molekulargewichts der einzelnen Seitenketten wurde für Glukose ein Molekulargewicht von 162 angenommen. Die gesamte Fläche unterhalb der Linie im GPC Chromatogramm, wurde als 100% festgesetzt und der Anteil der Flächen der einzelnen Abschnitte bezogen auf den Anteil der Gesamtfläche berechnet.

### Beispiele

### 1. Herstellung des Expressionsvektors ME5/6

pGSV71 ist ein Derivat des Plasmides pGSV7, welches sich vom intermediären Vektor pGSV1 ableitet. pGSV1 stellt ein Derivat von pGSC1700 dar, dessen Konstruktion von Cornelissen und Vanderwiele (Nucleic Acid Research 17, (1989), 19-25) beschrieben wurde. pGSV1 wurde aus pGSC1700 erhalten, durch Deletion des Carbenicillin Resistenzgen, sowie Deletion der T-DNA-Sequenzen der TL-DNA-Region des Plasmides pTiB6S3.
pGSV7 enthält den Replikationsursprung des Plasmides pBR322 (Bolivar et al., Gene 2, (1977), 95-113) sowie den Replikationsursprung des *Pseudomonas-*Plasmides pVS1 (Itoh et al., Plasmid 11, (1984), 206). pGSV7 enthält außerdem das selektierbare Markergen *aadA,* aus dem Transposon Tn1331 aus *Klebsiella pneumoniae,* welches Resistenz gegenüber den Antibiotika Spectinomycin und Streptomycin verleiht (Tolmasky, Plasmid 24 (3), (1990), 218-226; Tolmasky and Crosa, Plasmid 29(1), (1993), 31-40)
Das Plasmid pGSV71 wurde erhalten durch Klonierung eines chimären *bar*-Gens zwischen die Borderregionen von pGSV7. Das chimäre *bar*-Gen enthält die Promotorsequenz des Blumenkohlmosaikvirus zur Initiation der Transkription (Odell et al., Nature 313, (1985), 180), das *bar*-Gen aus *Streptomyces hygroscopicus* (Thompson et al., Embo J. 6, (1987), 2519-2523) und den 3'-untranslatierten Bereich des Nopalinsynthasegens der T-DNA von pTiT37, zur Termination der Transkription und Polyadenylierung. Das bar-Gen vermittelt Toleranz gegenüber dem Herbizid Glufosinat-Ammonium.
Die T-DNA enthält an Position 198-222 die rechte Randsequenz der TL-DNA aus dem Plasmid pTiB6S3 (Gielen et al., EMBO J. 3, (1984), 835-846). Zwischen Nukleotid 223-249 befindet sich eine Polylinker-Sequenz. Die Nukleotide 250-1634 enthalten die P35S3 Promotor-Region des Blumenkohl-Mosaik-Virus (Odell et al., siehe oben). Die codierende Sequenz des Phosphinothricin-Resistenzgen (*bar*) aus *Streptomyces hygroscopicus* (Thompson et al. 1987, siehe oben) ist zwischen den Nukleotiden 1635-2186 enthalten. Dabei wurden die zwei endständigen Codons am 5'-Ende des *bar*-Wildtyp-Gens ersetzt durch die Codons ATG und GAC. Zwischen den Nukleotiden 2187-2205 befindet sich eine Polylinker-Sequenz. Das 260 bp lange Taql-Fragment des nicht-translatierten 3'-Endes des Nopalinsynthase-Gens (3'nos) aus der T-DNA des Plasmides pTiT37 (Depicker et al., J. Mol. Appl. Genet. 1, (1982), 561-573) befindet sich zwischen den Nukleotiden 2206 und 2465. Die Nukleotide 2466-2519 enthalten eine Polylinker-Sequenz. Die linke Randsequenz der TL-DNA aus pTiB6S3 (Gielen et al., EMBO J. 3, (1984), 835-846) befindet sich zwischen den Nukleotiden 2520-2544.
Der Vektor pGSV71 wurde dann mit dem Enzym Pstl aufgeschnitten und geglättet. Aus dem Vektor pB33-Kan wurde der B33 Promotor sowie die *ocs*-Kassette als *Eco*RI-*Hind*III-Fragment ausgeschnitten und geglättet und in den mit *Pst*l aufgeschnittenen und geglätteten Vektor pGSV71 eingefügt. Der erhaltene Vektor diente als Ausgangsvektor zur Konstruktion von ME5/6: In die zwischen B33-Promotor und *ocs*-Element gelegene *Pst*l-Schnittstelle des Vektors ME4/6 wurde ein Oligonukleotid, enthaltend die Schnittstellen *Eco*RI, *Pac*l, *Spe*I, *Srf*l, *Spe*l, *Not*l, *Pac*l und *Eco*RI, unter Verdopplung der *Pst*l-Schnittstelle eingeführt. Der erhaltene Expressionsvektor wurde als ME5/6 bezeichnet.

### Beschreibung des Vektors pSK-Pac:

pSK-Pac ist ein Derivat des pSK-Bluescript (Stratagene, USA) bei dem flankierend zur multiplen Klonierungsstelle (MCS) je eine *Pac*l Schnittstelle eingeführt wurde.

### 2. Herstellung transgener Kartoffelpflanzen, die eine verringerte Aktivität eines BEI-Proteins , SSIII-Proteins, und eines BEII-Proteins aufweisen

Zur Erzeugung transgener Pflanzen, die eine verringerte Aktivität eines BEI-Proteins, eines SSIII-Proteins und eines BEII-Proteins aufweisen, wurden zunächst transgene Pflanzen erzeugt, die eine verringerte Aktivität eines BEI-Proteins und eines SSIII Proteins aufweisen. Zu diesem Zwecke wurde die T-DNA des Plasmids pB33-alpha-BEI-alpha-SSIII-Kan mit Hilfe von Agrobakterien, wie bei Rocha-Sosa et al. (EMBO J. 8, (1989), 23-29) beschrieben, in Kartoffelpflanzen transferiert.
Zur Konstruktion des Plasmids pB33-alpha-BEI-alpha-SSIII-Kan wurde zunächst der Expressionsvektor pBin33-Kan konstruiert. Dazu wurde der Promotor des Patatin Gens B33 aus *Solanum tuberosum* (Rocha-Sosa et al., 1989, siehe oben) als Dral-Fragment (Nukleotide -1512 - +14) in den mit *Sst*l geschnittenen Vektor pUC19 (Genbank Acc. No. M77789), dessen Enden mit Hilfe der T4 DNA-Polymerase geglättet worden waren, ligiert. Daraus entstand das Plasmid pUC19-B33. Aus diesem Plasmid wurde der B33-Promotor mit *Eco*RI und *Sma*l herausgeschnitten und in den entsprechend geschnittenen Vektor pBinAR ligiert. Hieraus entstand der pflanzliche Expressionsvektor pBin33-Kan. Das Plasmid pBinAR ist ein Derivat des Vektorplasmids pBin19 (Bevan, Nucl. Acid Research 12, (1984), 8711-8721) und wurde von Höfgen and Willmitzer (Plant Sci. 66, (1990), 221-230) konstruiert. Anschließend wurde ein 1631 bp langes Hindll-Fragment, welches eine partielle cDNA codierend für das BEI-Enzym aus Kartoffel enthält (Kossmann et al., 1991, Mol. & Gen. Genetics 230(1-2):39-44), geglättet und in "antisense"-Orientierung bezüglich des B33 Promotors (Promotor des Patatin Gens B33 aus Solanum tuberosum; Rocha-Sosa et al., 1989) in den mit *Smal* vorgeschnittenen Vektor pBinB33 eingeführt. Das erhaltene Plasmid wurde mit *BamHl* aufgeschnitten. In die Schnittstelle wurde ein 1363 bp langes BamHl-Fragment, enthaltend eine partielle cDNA codierend für das SS III-Protein aus Kartoffel (Abel et al., 1996, loc.cit.), ebenfalls in "antisense"-Orientierung bezüglich des B33-Promotors eingeführt.

Nach der Transformation konnten verschiedene Linien transgener Kartoffelpflanzen identifiziert werden, deren Knollen eine deutlich verringerte Aktivität eines BEI-Proteins und eines SSIII-Proteins aufwiesen. Die aus dieser Transformation resultierenden Pflanzen wurden mit 038VL bezeichnet.
Zum Nachweis der Aktivität löslicher Stärkesynthasen durch nicht-denaturierende Gelelektrophorese wurden Gewebeproben von Kartoffelknollen in 50 mM Tris-HCI pH 7,6, 2 mM DTT, 2,5 mM EDTA, 10 % Glycerin und 0,4 mM PMSF aufgeschlossen. Die Elektrophorese wurde in einer MiniProtean 11 Kammer (BioRAD) durchgeführt. Die Monomerkonzentration der 1,5 mm dicken Gele war 7,5 % (w/v), als Gel- und Laufpuffer diente 25 mM Tris-Glycin pH 8,4. Gleiche Mengen an Proteinextrakt wurden aufgetragen und für 2 h bei 10 mA je Gel aufgetrennt.
Anschließend erfolgte die Inkubation der Aktivitäts-Gele in 50 mM Tricine-NaOH pH 8,5, 25 mM Kaliumacetat, 2 mM EDTA, 2 mM DTT, 1 mM ADP-Glukose, 0,1 % (w/v) Amylopektin und 0,5 M Natriumcitrat. Gebildete Glukane wurden mit Lugolscher Lösung angefärbt.
Der Nachweis der BEI Aktivität erfolgte ebenfalls mit Hilfe der nicht denaturierenden Gelelektrophorese:
Zur Isolierung von Proteinen aus Pflanzen wurde das Probenmaterial in flüssigem Stickstoff gemörsert, in Extraktionspuffer (50 mM Na-Citrat, pH 6.5; 1 mM EDTA, 4 mM DTT) aufgenommen und nach Zentrifugation (10 min, 14.000 g, 4 °C) direkt zur Messung der Proteinkonzentration nach Bradford eingesetzt. Anschließend wurde je nach Bedarf 5 bis 20 µg Gesamt-Proteinextrakt mit 4-fach Loading-Buffer (20% Glycerin, 125 mM Tris HCI, pH 6,8) versetzt und auf ein "BE-Aktivitätsgel" geladen. Der Laufpuffer (RB) setzte sich wie folgt zusammen: RB = 30,2 g Tris-Base, pH 8.0, 144 g Glycine auf 1 L H₂O.
Nach Beendigung des Gellaufes wurden die Gele in je 25 ml "Phosphorylase-Puffer" (25 ml 1M Na-Citrat pH 7,0, 0,47 g Glucose-1-Phosphat, 12,5 mg AMP, 2,5mg Phosphorylase a/b aus "rabbit") über Nacht bei 37 °C inkubiert. Die Färbung der Gele wurde mit Lugol'scher Lösung durchgeführt.

Weitergehende Analysen zeigten, dass isolierte Stärken der Linie 038VL008 und 038VL107, welche eine Reduzierung sowohl des BEI-, als auch des SSIII-Proteins aufweisen, den höchsten Phosphatgehalt aller untersuchten unabhängigen Transformanten aufwiesen.
Pflanzen dieser Linien wurden anschließend wie beschrieben bei Rocha-Sosa et al. (EMBO J. 8, (1989), 23-29) mit dem Plasmid pGSV71-alpha-BEII-basta transformiert. Plasmid pGSV71-alpha-BEII-basta wurde konstruiert, indem eine knollenspezifische Kartoffel cDNA Bank mit einem durch RT-PCR (Primer: 5'- gggggtgttggctttgacta und 5'- cccttctcctcctaatccca; Stratagene ProSTAR™ HF Single-Tube RT-PCR System) mit Gesamt-RNA aus Knollen als Vorlage amplifizierten DNA Fragment nach Standardmethoden durchmustert wurde. Auf diese Weise wurde ein etwa 1250 bp großes DNA Fragment isoliert (SEQ ID No. 8), welches dann als *EcoRV-Smal* Fragment in die *EcoRV* Schnittstelle des Klonierungsvektor pSK-Pac (siehe oben) subkloniert und abschließend als *Pacl* Fragment in antisense Orientierung, bezogen auf den Promotor, in den Expressionsvektor ME5/6 ligiert wurde. Somit entstand Plasmid pGSV71-alpha-BEII-basta (siehe Fig 6).

Von den durch Transformation mit dem Plasmid pGSV71-alpha-BEII-basta erhaltenen Pflanzen, die als 108CF bzw. 110CF bezeichnet wurden, wurden Gewebeproben von Knollen der unabhängigen Transformanten genommen und deren Amylosegehalt ermittelt (siehe Methoden). Die Stärken der unabhängigen Linien, deren Knollen den höchsten Amylosegehalt aufwiesen, wurden für eine weitere Analyse der Stärkeeigenschaften herangezogen. Zum Nachweis, dass diese Pflanzen zusätzlich zu einer reduzierte Aktivität eines BEI-Proteins und SSIII-Proteins auch eine reduzierte Aktivität eines BEII-Proteins aufweisen, wurde ebenfalls eine Analyse mit Hilfe der nicht denaturierenden Gelelektrophorese durchgeführt. Die Analyse wurde mit der gleichen Methode wie oben bereits für die Analyse der reduzierten BEI-Aktivität durchgeführt, außer, dass das nicht denaturierende Polyacrylamidgel zusätzlich zur oben beschriebenen Zusammensetzung 0,5% Maltodextrin (Beba, Maltodextrin-Lösung 15%ig für Neugeborene, Nestle) enthielt. Durch Zusatz des Dextrins konnten die unterschiedlichen Aktivitäten der BEI-Proteine und BEII-Proteine nach Inkubation der Gele in "Phosphorylase-Puffer" (25 ml 1M Na-Citrat pH 7,0, 0,47 g Glucose-1-Phosphat, 12,5 mg AMP, 2,5mg Phosphorylase a/b aus "rabbit") über Nacht bei 37 °C und anschließender Färbung mit Lugol'scher Lösung in einem Gel dargestellt werden.

### 3. Klonierung einer Vollängensequenz eines Proteins mit der unter SEQ ID NO 12 oder SEQ ID NO 14 angegebenen Sequenz aus Solanum tuberosum

Die Gensequenz codierend für ein Protein mit der unter SEQ ID NO 12 oder SEQ ID NO 14 angegebenen Sequenz aus *Solanum tuberosum* ist bisher noch nicht beschrieben worden.
Über Sequenzvergleiche mit verschiedenen Verzweigungsenzymen konnte eine Domäne identifiziert werden, mit deren Hilfe EST-Datenbanken durchmustert wurden. Hierbei konnte der EST TC73137 (TIGR Datenbank; http://www.tigr.org/tigr-scripts/tgi/tc_report.pl?tc=TC73137&species=potato) aus Kartoffel identifiziert werden.
Mit Hilfe der Primer B1_Asp (GAT GGG TAC CAG CAC TTC TAC TTG GCA GAG G) und B2_Sal (TCA AGT CGA CCA CAA CCA GTC CAT TTC TGG) konnte eine zu dieser EST-Sequenz korrespondierende Sequenz aus einer knollespezifischen cDNA-Bank von *Solanum tuberosum* (cv. Désirée) amplifiziert werden. Versuche, blattspezifische, "sink"- oder "source"-Gewebe spezifische cDNA-Banken als Template für die PCR Reaktion zu verwenden, führten zu keinem Amplifikat.
Um die gesamte codierende Sequenz des betreffenden Proteins, die auch bisher unbekannte Sequenzen umfasst, zu amplifizieren, wurden Primer hergestellt, die zu den Enden der bisher bekannten Sequenz und Vektorsequenzen der betreffenden cDNA Banken komplememtär sind. Bei allen mittels dieses Ansatzes verwendeten Primerkombinationen zur Amplifikation einer vollänge Sequenz konnte kein weiterer Bereich amplifiziert werden. Daraufhin wurden EST-Datenbanken von Tomate erneut durchmustert.
Hierbei konnten zwei ESTs aus Tomate identifiziert werden (TIGR Datenbank; BG127920 und TC130382), .die entweder eine hohe Homologie zu dem oben beschriebenen Amplifikat des Proteins aus Kartoffel (TC130382) bzw. (BG127920). oder zu einem putativen Verzweigungsenzym aus *Arabidopsis* (Genbank: GP|9294564|dbj|BAB02827.1) aufweisen.

Es wurden nun erneut Primer hergestellt, um auch bisher unbekannte Sequenzen des Proteins mit der unter SEQ ID NO 12 oder SEQ ID NO 14 dargestellten Aminosäuresequenz zu amplifizieren. Mittels PCR wurde aus einer cDNA Bank, hergestellt aus Knollen von *Solanum tuberosum* (cv. Désirée), mit den Primern KM2_Spe (5'-TCAAACTAGTCACAACCAGTCCATTTCTGG-3') und SoputE (5'-CACTTTAGAAGGTATCAGAGC-3') der 3'-Bereich des betreffenden Proteins amplifiziert. Das erhaltende ca. 1 kb große Fragment wurde ungerichtet in den pCR4-TOPO Vektor von Invitrogen (Produktnummer: 45-0030) kloniert. Das entstandene Plasmid wurde als AN 46-196 bezeichnet. Die Sequenz des inserierten Fragments im Plasmid AN 46-196 ist unter SEQ ID NO 9 dargestellt.

Der 5'-Bereich wurde ebenfalls mittels PCR-Technik und unter Verwendung der Primer So_put5' (5'-GTATTTCTGCGAAGGAACGACC-3') und So_putA (5'-AACAATGCTCTCTCTGTCGG-3') aus der selben cDNA-Bank amplifiziert. Das erhaltende ca. 2 kb große Fragment wurde ungerichtet in den pCR4-TOPO Vektor von Invitrogen (Produktnummer: 45-0030) kloniert. Das entstandene Plasmid wurde als AN 47-196 bezeichnet. Die Sequenz des inserierten Fragments im Plasmid AN 47-196 ist unter SEQ ID NO 10 dargestellt.

Es wurden nun erneut Primer hergestellt, um eine Vollängensequenz zu amplifizieren.
Folgende Primer wurden verwendet: SOputA (AACAATGCTCTCTCTGTCGG) und SO_putE (CACTTTAGAAGGTATCAGAGC). Ein ungefähr 3,2 kb großes PCR-Produkt wurde erhalten und in den pCR2.1-Vektor der Firma Invitrogen (Produktnummer: 45-0030) kloniert. Das erhaltene Plasmid (hinterlegt unter DSM 15926) wurde mit AN 49 bezeichnet. Die Sequenz des inserierten Fragments im Plasmid AN 49 ist unter SEQ ID NO 11 dargestellt.

### 4. Herstellung transgener Kartoffelpflanzen, die eine verringerte Aktivität eines BEI-Proteins , SSIII-Proteins, BEII-Proteins und eines Proteins mit der unter SEQ ID NO 12 oder SEQ ID NO 14 dargestellten Aminosäuresequenz aufweisen

### a) Angaben zum Vektor pBinB33-Hyg

Ausgehend vom Plasmid pBinB33 wurde das *Eco*RI-*Hind*III-Fragment umfassend den B33-Promotor, einen Teil des Polylinkers sowie den *ocs*-Terminator herausgeschnitten und in den entsprechend geschnittenen Vektor pBIB-Hyg ligiert (Becker, 1990).
Das Plasmid pBinB33 wurde erhalten, indem der Promotor des Patatin Gens B33 aus *Solanum tuberosum* (Rocha-Sosa et al., 1989) als *Dra*I-Fragment (Nukleotide - 1512 - +14) in den mit *Sst*l geschnittenen Vektor pUC19, dessen Enden mit Hilfe der T4 DNA-Polymerase geglättet worden waren, ligiert wurde. Daraus entstand das Plasmid pUC19-B33. Aus diesem Plasmid wurde der B33-Promotor mit *Eco*RI und *Sma*l herausgeschnitten und in den entsprechend geschnittenen Vektor pBinAR ligiert. Hieraus entstand der pflanzliche Expressionsvektor pBinB33.
Das Plasmid pBinAR ist ein Derivat des Vektorplasmids pBin19 (Bevan, 1984) und wurde folgendermaßen konstruiert:
Ein 529 bp langes Fragment, das die Nukleotide 6909-7437 des 35S RNA-Promotors des Blumenkohl-Mosaik-Virus (Pietrzak et al., 1986, Nucleic Acids Research 14, 5857-5868) umfasst, wurde als *Eco*RI/*Kpn*I-Fragment aus dem Plasmid pDH51 (Pietrzak et al., 1986) isoliert und zwischen die *Eco*RI*-* und *Kpn*I-Schnittstellen des Polylinkers von pUC18 ligiert. Dabei entstand das Plasmid pUC18-35S.
Aus dem Plasmid pAGV40 (Herrera-Estrella et al., 1983) wurde mit Hilfe der Restriktionsendonukleasen *Hind*III und *Pvu*II ein 192 bp langes Fragment isoliert, welches das Polyadenylierungssignal (3'-Ende) des *Octopin Synthase*-Gens (Gen 3) der T-DNA des Ti-Plasmids pTiACH5 (Gielen et al., 1984) umfasst (Nukleotide 11749-11939). Nach Addition von Sspl-Linkern an die Pvull-Schnittstelle wurde das Fragment zwischen die *Sph*I- und *Hind*lll-Schnittstelle von pUC18-35S ligiert. Daraus resultierte das Plasmid pA7.
Aus pA7 wurde der gesamte Polylinker enthaltend den 35S-Promotor und den ocs-Terminator mit EcoRI und HindIII herausgeschnitten und in den entsprechend geschnittenen pBin19 ligiert. Dabei entstand der pflanzliche Expressionsvektor pBinAR (Höfgen and Willmitzer, 1990).

### b) Angaben zum Vektor AN 54-196

AN 54-196 ist ein Derivat des Plasmides pBinB33-Hyg, welchem eine Teilsequenz der für das Protein mit der unter SEQ ID NO 12 oder SEQ ID NO 14 angegebenen Aminosäuresequenz codierenden Nucleinsäuresequenz als "inverted repeat" ,(RNAi Technologie) unter der Kontrolle des Promotors des Patatin Gens B33 aus *Solanum tuberosum* (Rocha-Sosa et al., 1989) eingefügt wurde. Hierzu wurde zuerst ein PCR-Produkt mit den Primern B1_Asp (GAT GGG TAC CAG CAC TTC TAC TTG GCA GAG G) und B2_Sal (TCA AGT CGA CCA CAA CCA GTC CAT TTC TGG) aus einer knollenspezifischen cDNA Bank von *Solanum tuberosum* (cv. Désirée) amplifiziert, wodurch die Schnittstellen *Asp718* und *Sall* zugefügt wurden. Das erhaltene PCR-Produkt (625 bp) wurde über diese beiden Schnittstellen in "antisense" Orientierung zum B33-Promotor kloniert. Ein zweites PCR-Fragment, welches mit den Primern B3_Sal (GCT TGT CGA CGG GAG AAT TTT GTC CAG AGG) und B4_Sal (GAT CGT CGA CAG CAC TTC TAC TTG GCA GAG G) aus einer knollenspezifischen cDNA Bank von *Solanum tuberosum* (cv. Désirée) amplifiziert wurde und mit dem 301 bp des ersten Fragments identisch ist, wurde über die Sall-Schnittstelle hinter das erste Fragment kloniert, jedoch in "sense"-Orientierung zum B33-Promotor. Diese Anordnung wird als "inverted repeat" (RNAi Technologie) bezeichnet.

### c) Herstellung transgener Kartoffelpflanzen

Zur Erzeugung transgener Kartoffelpflanzen, die eine verringerte Aktivität eines BEI-Proteins , SSIII-Proteins, BEII-Proteins und eines Proteins mit der unter SEQ ID NO 12 oder SEQ ID NO 14 dargestellten Aminosäuresequenz aufweisen, wurde die T-DNA des Plasmids AN 54-196 mit Hilfe von Agrobakterien, wie bei Rocha-Sosa et al. (EMBO J. 8, (1989), 23-29) beschrieben, in transgene Kartoffelpflanzen der Linie 110CF-003 transferiert. Die durch Transformation mit dem Plasmid AN 53-196 erhaltenen Pflanzen 376SO bezeichnet.

### 5. Analyse der Stärke von Pflanzen mit verringerter Aktivität eines BEI-Proteins, SSIII-Proteins, BEII-Proteins und eines Proteins, das die SEQ ID NO 12 oder SEQ ID NO 14 dargestellte Aminosäuresequenz aufweist.

Stärke aus Knollen verschiedener unabhängiger Linien der in den oben genannten Beispielen beschriebenen Transformationen 110CF und 376SO und aus Knollen von Wildtyp-Pflanzen wurden (cv Désirée) isoliert. Anschließend wurden die physiko-chemischen Eigenschaften dieser Stärke analysiert.

### a) RVA Analyse

Das Viskositätsprofil von Stärken, isoliert aus Knollen von den Linien der in den oben genannten Beispielen beschriebenen Transformationen 110CF und 376SO und aus Knollen von Wildtyp-Pflanzen wurden (cv Désirée) wurde nach der unter allgemeine Methoden, Punkt 4 beschriebenen RVA Analysemethode 2 bestimmt. Anschließend wurde die Gelfestigkeit nach der unter allgemeine Methoden, Punkt 3 beschriebenen Methode bestimmt.

Die folgende Tabelle 1 fasst die Ergebnisse der RVA Analyse und der Analyse der Gelfestigkeit zusammen. Die angegeben Werte stellen den prozentualen Anteil der jeweiligen ermittelten Messwerte bezogen auf den entsprechenden Messwert von Stärke, isoliert aus Knollen von Wildtyp-Pflanzen dar, der mit jeweils100% festgesetzt wurde.

**Tabelle 1 RVA nach Methode 2, TA-Kraft von mit CaCl₂ verkleisterten Stärken**

| | RVA Max (%) | RVA Min (%) | RVA Fin (%) | RVA Set (%) | RVA T (%) | RVA Pasting Temperature | TA "Kraft 1" |
|---|---|---|---|---|---|---|---|
| Wildtyp (Désirée) | 100,0% | 100,0% | 100,0% | 100,0% | 100,0% | 100,0% | 100 % |
| 110CF-003 | 97,1% | 314,5% | 165,9% | 83,6% | 167,2% | 115,6% | 159,4% |
| 376SO-010 | 103,9% | 284,4% | 153,7% | 81,7% | 143,7% | 115,8% | 195,3 % |
| 376SO-047 | 94,1% | 292,4% | 163,3% | 92,9% | 164,3% | 134,2% | 175,5% |
| 376SO-087 | 82,5% | 252,9% | 147,1% | 89,4% | 157,6% | 125,7% | 173,4% |

Das Viskositätsprofil von Stärken, isoliert aus Knollen von den Linien 110CF-003 und 376SO-010 wurde nach der unter allgemeine Methoden, Punkt 4 beschriebenen RVA Analysemethode 3 bestimmt.
Die folgende Tabelle 2 stellt die erhaltenen Werte für die Verkleisterungstemperatur der gemessenen Linien dar:

**Tabelle 2**

| | Pasting Temperature |
|---|---|
| 110CF-003 | 78,8° C |
| 376SO-010 | 93,6°C |

### b) Analyse der Phosphat- und Amylosegehalte

Der C-6-Phosphatgehalt von Stärken, isoliert aus Knollen von den Linien der in den oben genannten Beispielen beschriebenen Transformationen 110CF und 376S0 und aus Knollen von Wildtyp-Pflanzen (cv Desiree) wurde nach der unter allgemeine Methoden, Punkt 2 beschriebenen Methode bestimmt.

Der Gesamtphosphatgehalt von Stärken, isoliert aus Knollen von den Linien der in den oben genannten Beispielen beschriebenen Transformationen 110CF und 376S0 und aus Knollen von Wildtyp-Pflanzen wurden (cv Désirée) wurde nach der unter allgemeine Methoden, Punkt 2 beschriebenen Methode bestimmt.

Der Amylosegehalt von Stärken, isoliert aus Knollen von einzelnen Linien der in den oben genannten Beispielen beschriebenen Transformationen 110CF und 376SO und aus Knollen von Wildtyp-Pflanzenwurden (cv Désirée) wurde nach der unter allgemeine Methoden, Punkt 1 beschriebenen Methode bestimmt.

Die Ergebnisse aus repräsentativen Linien sind in Tabelle 3 dargestellt.
Die Menge an C-6-Phosphat bzw. Gesamtphosphat wurde zunächst in µmol / g Stärke ermittelt. Alle anderen in Tabelle 3 angegebnen Werte betreffend den C-6-Phosphatgehalt bzw. den Gesamtphosphatgehalt können aus dem zunächst bestimmten Wert (µmol / g Stärke) errechnet werden. Für diese Berechnungen wird 31 als Wert für das Molekulargewicht von Phosphor eingesetzt.

Die Angaben "Menge [%] geben jeweils den prozentualen Anteil der betreffenden Substanz an der Gesamtmenge der Stärke an.
Die Angaben "bezogen auf Wildtyp [%]" geben jeweils die Menge der betreffenden Substanz an, die diese Menge, bezogen auf die entsprechende Menge der gleichen Substanz in Stärke, isoliert aus Knollen von Wildtyp-Pflanzen in Prozent darstellt.

### 6. Analyse der Seitenkettenverteilung des Amylopektins

Der Seitenkettenverteilung von Stärken, isoliert aus Knollen von einzelnen Linien der in den oben genannten Beispielen beschriebenen Transformationen 110CF und 376SO und aus Knollen von Wildtyp-Pflanzen wurden (cv Desiree) wurde nach der unter allgemeine Methoden, Punkt 6 beschriebenen Methode bestimmt. Die Ergebnisse sind in Tabelle 4 zusammengefasst dargestellt.

**Tabelle 4**

| | MW Desi | 110-CF | 376-SO-10 | 376-SO-47 | MW 376-SO-87 |
|---|---|---|---|---|---|
| <dp11 | 100,0% | 24,2% | 14,3% | 15,8% | 13,5% |
| dp11-dp18 | 100,0% | 45,7% | 36,3% | 40,1% | 37,5% |
| dp19-dp24 | 100,0% | 77,6% | 68,8% | 72,2% | 71,3% |
| dp25-dp30 | 100,0% | 100,2% | 93,6% | 96,2% | 94,8% |
| dp31-dp36 | 100,0% | 101,9% | 98,5% | 100,0% | 98,3% |
| dp37-dp42 | 100,0% | 98,4% | 96,8% | 97,4% | 96,6% |
| dp43-dp48 | 100,0% | 103,6% | 103,1% | 103,2% | 103,1% |
| dp49-dp55 | 100,0% | 118,7% | 119,8% | 119,3% | 119,9% |
| dp56-dp61 | 100,0% | 139,6% | 143,4% | 142,1% | 143,2% |
| dp62-dp123 | 100,0% | 231,1% | 256,3% | 246,5% | 252,0% |
| >123dp | 100,0% | 929,4% | 1236,3% | 1135,4% | 1209,4% |

## Patentansprüche

1. Modifizierte Stärke isoliert aus Kartoffelpflanzen **dadurch gekennzeichnet, dass** sie
a) einen Amylosegehalt, gemessen nach der Methode von Hovenkamp-Hermelink et al. (1987, Theoretical and Applied Genetics 75, 217-221), zwischen 40% bis 50% enthält und
b) einen Phosphorgehalt von 80 bis 95 µmol Phosphat pro Gramm Stärke (Trockengewicht) aufweist.

2. Modifizierte Stärke nach Anspruch 1, **dadurch gekennzeichnet, dass** sie einen C-6-Phosphorgehalt von 45 bis 60 µmol Phosphat pro Gramm Stärke (Trockengewicht) aufweist.

3. Modifizierte Stärke nach einem der Ansprüche 1 oder 2, die eine veränderte Seitenkettenverteilung des Amylopektins aufweist im Vergleich zu Stärke, isoliert aus entsprechenden Kartoffel Wildtyp-Pflanzen.

4. Modifizierte Stärke nach einem der Ansprüche 1, 2 oder 3, wobei der Anteil der Seitenketten mit einem DP kleiner 11 und/oder einem DP von 11 bis 18 reduziert ist und der Anteil der Seitenketten mit einem DP von 56 bis 62 und/oder einem DP von 62 bis 123 erhöht ist im Vergleich zu Stärke, isoliert aus entsprechenden Kartoffel Wildtyp-Pflanzen.

5. Verfahren zur Herstellung einer derivatisierten Stärke, worin modifizierte Stärke nach einem der Ansprüche 1 bis 4 nachträglich derivatisiert wird.

6. Verwendung von modifizierter Stärke nach einem der Ansprüche 1 bis 4 zur Herstellung von derivatisierter Stärke.
